# FASCICULE DE BREVET EUROPEEN

(11) **EP 2 094 706 B1**
(45) Date de publication et mention de la délivrance du brevet: **05.01.2011**
(21) Numéro de dépôt: 07870295.8
(22) Date de dépôt: 19.11.2007
(51) Int. Cl.: C07D 487/04, A61K 31/5025, A61P 25/00, A61P 3/00, C07D 495/04

(54) **DERIVES DE 2,5-DFFLYDRO-3H-PYRAZOLO[4,3-C]PYRIDAZIN-3-ONE SUBSTITUES, LEUR PREPARATION ET LEUR UTILISATION COMME LIGANDS DES RECEPTEURS CB1 DES CANNABINOIDES**
SUBSTITUIERTE 2,5-DIHYDRO-3H-PYRAZOL-[4,3-C]-PYRIDAZIN-3-ON-DERIVATE, IHRE HERSTELLUNG UND IHRE VERWENDUNG ALS CANNIBINOID-CB1-REZEPTORLIGANDEN
SUBSTITUTED 2,5-DFFLYDRO-3H-PYRAZOLO[4,3-C]PYRIDAZIN-3-ONE DERIVATIVES, PREPARATION THEREOF AND USE OF SAME AS CANNABINOID CB1 RECEPTOR LIGANDS

(30) Priorité: 23.11.2006 FR 0610371
(43) Date de publication de la demande: 02.09.2009
(73) Titulaire: Sanofi-Aventis, 75013 Paris (FR)
(72) Inventeur: CONGY, Christian, 75013 Paris (FR); DOS SANTOS, Victor, 75013 Paris (FR); RINALDI-CARMONA, Murielle, 75013 Paris (FR); ROUQUETTE, Arnaud, 75013 Paris (FR); VAN BROECK, Didier, 75013 Paris (FR)
(74) Mandataire: Werner, Alain Henri
(86) Numéro de dépôt international: PCT/FR2007/001889
(87) Numéro de publication internationale: WO 2008/068424

(56) Documents cités:
- WO-A-2004/108728
- WO-A-2005/061504

## Description

La présente invention a pour objet des dérivés de 2,5-dihydro-3*H*-pyrazolo[4,3-c]pyridazin-3-one substitués, leur préparation et leur application en thérapeutique.

Des dérivés de diphénylpyrazole présentant une affinité pour les récepteurs CB₁ des cannabinoïdes ont été décrits notamment dans les brevets US 5 624 941, EP 0 576 357, EP 0 656 354, EP 1 150 961.

Des dérivés de 2,5-dihydro-3*H*-pyrazolo[4,3-c]pyridazin-3-one présentant une activité comme agents chimiques hybridant sont décrits dans Youji Huaxue, 2004, 24(6), 645-649.

On a maintenant trouvé de nouveaux dérivés de 2,5-dihydro-3*H-*pyrazolo[4,3-c]pyridazin-3-one substitués qui possèdent des propriétés antagonistes des récepteurs CB1 des cannabinoïdes localisés au niveau central et/ou périphérique.

La présente invention a pour objet des composés répondant à la formule : 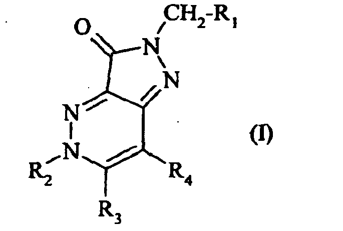 dans laquelle :
- R₁ représente :
   . un (C₁-C₁₂)alkyle non substitué ou substitué une ou plusieurs fois par un atome de fluor ;
   . un radical carbocyclique non aromatique en (C₃-C₁₂) non substitué ou substitué une ou plusieurs fois par des substituants choisis indépendamment parmi un (C₁-C₄)alkyle, un (C₁-C₄)alcoxy, un atome de fluor, un hydroxyle, un radical trifluorométhyle, un radical trifluorométhoxy, un (C₁-C₄)alkylthio ;
   . un phényle non substitué ou substitué une ou plusieurs fois par des substituants choisis indépendamment parmi un atome d'halogène, un hydroxyle, un groupe Alk, un groupe OAlk, un méthylènedioxy, un groupe CH₂-NHAlk, un groupe -CH₂N(Alk)₂, un cyano, un nitro, un groupe S(O)ₙAlk, un groupe OS(O)ₙAlk, un groupe (C₁-C₄)alkylcarbonyle, un groupe (C₁-C₄)alcoxycarbonyle ; ou parmi un radical phényle, phénoxy, pyrrolyle, imidazolyle, pyridyle, pyrazolyle, oxazolyle, thiazolyle, triazolyle ou thiadiazolyle, ledit radical étant non substitué ou substitué une ou plusieurs fois par un (C₁-C₄)alkyle ;
   . un benzyle non substitué ou substitué une ou plusieurs fois sur le phényle par des substituants choisis indépendamment parmi un atome d'halogène, un groupe Alk, un hydroxyle, un groupe OAlk, un méthylènedioxy, un groupe S(O)ₙAlk, un groupe OS(O)ₙAlk ;
   . un phénéthyle non substitué ou substitué une ou plusieurs fois sur le phényle par des substituants choisis indépendamment parmi un atome d'halogène, un (C₁-C₄)alkyle, un (C₁-C₄)alcoxy, un radical trifluorométhyle, un radical trifluorométhoxy ;
   . un benzhydryle ; un benzhydrylméthyle ;
   . un radical hétérocyclique aromatique choisi parmi un pyrrolyle, un imidazolyle, un furyle, un thiényle, un pyrazolyle, un oxazolyle, un pyridyle, un indolyle, un benzothiényle, un thiéno[3,2-b]thiényle, ledit radical étant non substitué ou substitué une ou plusieurs fois par des substituants choisis indépendamment parmi un atome d'halogène, un groupe Alk, un groupe OAlk, un cyano, un nitro, un groupe S(O)ₙAlk ;
      - R₂ représente un phényle non substitué ou substitué une ou plusieurs fois par des substituants choisis indépendamment parmi un atome d'halogène, un hydroxy, un groupe Alk, un groupe OAlk, un groupe S(O)ₙAlk, un groupe OS(O)ₙAlk, un groupe -O(CH₂)ₘR₅ ou un groupe -S(CH₂)ₘR₆ ;
      - R₃ représente un phényle non substitué ou substitué une ou plusieurs fois par des substituants choisis indépendamment parmi un atome d'halogène, un hydroxy, un groupe Alk, un groupe OAlk, un groupe S(O)ₙAlk, un groupe OS(O)ₙAlk, un groupe -O(CH₂)ₘR₅ ou un groupe -S(CH₂)ₘR₆ ;
      - R₄ représente un atome d'hydrogène, un (C₁-C₄)alkyle, un (C₁-C₄)alcoxy, un hydroxy ;
      - R₅ représente un groupe -NR₇R₈ ou un groupe -SAlk;
      - R₆ représente un hydroxy, un groupe -NR₇R₈, un groupe NR₇COR₈ ou un groupe -NR₇SO₂R₉ ;
      - R₇ représente un atome d'hydrogène ou un (C₁-C₄)alkyle ;
      - R₈ représente un atome d'hydrogène, un groupe Alk, un (C₃-C₇)cycloalkyle ;
      - R₉ représente un (C₁-C₄)alkyle ;
      - m représente 2 ou 3 ;
      - n représente 0, 1 ou 2 ;
      - Alk représente un (C₁-C₄)alkyle non substitué ou substitué une ou plusieurs fois par un atome de fluor.

Les composés de formule (I) peuvent comporter un ou plusieurs atomes de carbone asymétriques. Ils peuvent donc exister sous forme d'énantiomères ou de diastéréoisomères. Ces énantiomères, diastéréoisomères ainsi que leurs mélanges, y compris les mélanges racémiques, font partie de l'invention.

Les composés de formule (I) peuvent exister à l'état de bases ou de sels d'addition à des acides. De tels sels d'addition font partie de l'invention.

Ces sels sont avantageusement préparés avec des acides pharmaceutiquement acceptables mais les sels d'autres acides utiles pour la purification ou l'isolement des composés de formule (I) font également partie de l'invention.

Les composés de formule (I) peuvent exister sous forme d'hydrates ou de solvats, à savoir sous forme d'associations ou de combinaisons avec une ou plusieurs molécules d'eau ou avec un solvant. De tels hydrates et solvats font également partie de l'invention.

Par atome d'halogène on entend un atome de brome, de chlore, de fluor ou d'iode.

Par (C₁-C₄)alkyle ou respectivement (C₁-C₁₂)alkyle, on entend un radical alkyle linéaire ou ramifié de un à quatre atomes de carbone ou respectivement de un à douze atomes de carbone, tel que le radical méthyle, éthyle, propyle, isopropyle, butyle, isobutyle, *sec*-butyle, *tert*-butyle, pentyle, isopentyle, hexyle, isohexyle, heptyle, octyle, nonyle, décyle, undécycle, dodécyle.

Par (C₁-C₄)alcoxy on entend un radical alcoxy linéaire ou ramifié de un à quatre atomes de carbone tel que le radical méthoxy, éthoxy, propoxy, isopropoxy, butoxy, *sec*-butoxy, *tert*-butoxy.

Par (C₃-C₇)cycloalkyle on entend un groupe alkyle cyclique de 3 à 7 atomes de carbone, tel que le groupe cyclopropyle, cyclobutyle, cyclopentyle, cyclohexyle, cycloheptyle.

Les radicaux carbocycliques non aromatiques en C₃-C₁₂ comprennent les radicaux mono ou polycycliques, condensés, pontés ou spiraniques. Les radicaux monocycliques incluent les (C₃-C₇)cycloalkyles. Les radicaux di- ou tricycliques condensés, pontés ou spiraniques, incluent par exemple les radicaux norbornyle, bornyle, isobornyle, noradamantyle, adamantyle, spiro[5.5]undécyle, bicyclo[2.2.1] heptyle, bicyclo[3.2.1]octyle ; bicyclo[3.1.1]heptyle.

Parmi les composés de formule (I), objets de l'invention, on distingue :
- les composés de formule (IA) dans laquelle R₁ représente:
   - un (C₁-C₁₂)alkyle non substitué ou substitué une ou plusieurs fois par un atome de fluor ;
      les substituants R₂, R₃ et R₄ sont tels que définis pour les composés de formule (I).
- Les composés de formule (IB) dans laquelle R₁ représente :
   - un radical carbocyclique non aromatique en (C₃-C₁₂) non substitué ou substitué une ou plusieurs fois par des substituants choisis indépendamment parmi un (C₁-C₄)alkyle, un (C₁-C₄)alcoxy, un atome de fluor, un hydroxyle, un radical trifluorométhyle, un radical trifluorométhoxy, un (C₁-C₄)alkylthio ;
      les substituants R₂, R₃ et R₄ sont tels que définis pour les composés de formule (I).
- Les composés de formule (IC) dans laquelle R₁ représente :
   - un phényle non substitué ou substitué une ou plusieurs fois par des substituants choisis indépendamment parmi un atome d'halogène, un hydroxyle, un groupe Alk, un groupe OAlk, un méthylènedioxy, un groupe CH₂-NHAlk, un groupe -CH₂N(Alk)₂, un cyano, un nitro, un groupe S(O)ₙAlk, un groupe OS(O)ₙAlk, un groupe (C₁-C₄)alkylcarbonyle, un groupe (C₁-C₄)alcoxycarbonyle ; ou parmi un radical phényle, phénoxy, pyrrolyle, imidazolyle, pyridyle, pyrazolyle, oxazolyle, thiazolyle, triazolyle ou thiadiazolyle, ledit radical étant non substitué ou substitué une ou plusieurs fois par un (C₁-C₄)alkyle;
      les substituants R₂, R₃ et R₄ sont tels que définis pour les composés de formule (I).
- Les composés de formule (ID) dans laquelle R₁ représente :
   - un benzyle non substitué ou substitué une ou plusieurs fois sur le phényle par des substituants choisis indépendamment parmi un atome d'halogène, un groupe Alk, un hydroxyle, un groupe OAlk, un méthylènedioxy, un groupe S(O)ₙAlk, un groupe OS(O)ₙAlk ;
      les substituants R₂, R₃ et R₄ sont tels que définis pour les composés de formule (I).
- Les composés de formule (IE) dans laquelle R₁ représente :
   - un radical hétérocyclique aromatique choisi parmi un pyrrolyle, un imidazolyle, un furyle, un thiényle, un pyrazolyle, un oxazolyle, un pyridyle, un indolyle, un benzothiényle, un thiéno[3,2-b]thiényle, ledit radical étant non substitué ou substitué une ou plusieurs fois par des substituants choisis indépendamment parmi un atome d'halogène, un groupe Alk, un groupe OAlk, un cyano, un nitro, un groupe S(O)ₙAlk ;
      les substituants R₂, R₃ et R₄ sont tels que définis pour les composés de formule (I).

Parmi les composés de formule (I), objets de l'invention, un premier groupe de composés est constitué par les composés pour lesquels :
- R₁ représente :
   - un (C₁-C₁₂)alkyle ;
   - un (C₃-C₇)cycloalkyle non substitué ou substitué par un (C₁-C₄)alkyle, un radical trifluorométhyle ; un adamantyle ;
   - un phényle non substitué ou substitué une ou plusieurs fois par des substituants choisis indépendamment parmi un atome d'halogène, un groupe Alk, un groupe OAlk, un groupe CH₂N(Alk)₂, un groupe -S(O)ₙAlk, un groupe (C₁-C₄)alcoxycarbonyle ; ou parmi un radical phényle, triazolyle, thiadiazolyle ;
   - un benzyle non substitué ou substitué une ou plusieurs fois sur le phényle par des substituants choisi indépendamment parmi un atome d'halogène, un groupe Alk ;
   - un radical hétérocyclique aromatique choisi parmi un pyridyle, un thiéno[3,2-b]thiényle, un benzothiényle, ledit radical étant non substitué ou substitué par un atome d'halogène, un radical trifluorométhyle ;
- R₂ représente un phényle mono- ou disubstitué par un atome d'halogène, un hydroxy, un groupe OAlk, un groupe S(O)ₙAlk, un groupe OS(O)ₙAlk, un groupe -O(CH₂)MR₅, un groupe -S(CH₂)ₘR₆ ;
- R₃ représente un phényle mono- ou disubstitué par un atome d'halogène, un groupe OAlk, un groupe S(O)ₙ Alk, un groupe -O(CH₂)ₘR₅, un groupe -S(CH₂)ₘR₆ ;
- R₄ représente un atome d'hydrogène, un (C₁-C₄)alkyle, un (C₁-C₄)alcoxy, un hydroxy ;
- n représente 0, 1 ou 2 ;
- Alk représente un (C₁-C₄)alkyle non substitué ou substitué une ou plusieurs fois par un atome de fluor ;

Les substituants m, R₅ et R₆ sont tels que définis pour les composés de formule (I) ; ainsi que leurs sels d'addition, leurs hydrates ou leurs solvats.

Parmi les composés de formule (I), objets de l'invention, on distingue les composés de formule (IC) dans laquelle :
- R₁ représente :
   - un phényle non substitué ou substitué une ou plusieurs fois par des substituants choisis indépendamment parmi un atome d'halogène, un groupe Alk, un groupe OAlk, un groupe -S(O)ₙAlk ;
- R₂ représente un phényle mono- ou disubstitué par un atome d'halogène, un hydroxy, un groupe OAlk, un groupe S(O)ₙAlk, un groupe OS(O)ₙAlk, un groupe -O(CH₂)ₘR₅, un groupe -S(CH₂)ₘR₆ ;
- R₃ représente un phényle mono- ou disubstitué par un atome d'halogène, un groupe OAlk, un groupe S(O)ₙ Alk, un groupe -O(CH₂)ₘR₅, un groupe -S(CH₂)ₘR₆ ;
- R₄ représente un atome d'hydrogène, un (C₁-C₄)alkyle, un (C₁-C₄)alcoxy, un hydroxy ;
- n représente 0, 1 ou 2 ;
- Alk représente un (C₁-C₄)alkyle non substitué ou substitué une ou plusieurs fois par un atome de fluor ;

Les substituants m, R₅ et R₆ sont tels que définis pour les composés de formule (I) ; ainsi que leur sels d'addition, leurs hydrates ou leurs solvats.

Parmi les composés de formule (I), objets de l'invention, on peut citer également les composés pour lesquels :
- R₁ représente :
   - un 1-propylbutyle ;
   - un cyclohexyle, un 4-*tert*-butylcyclohexyle, un 4-(trifluorométhyl)cyclohexyle ; un adamantan-1-yle ;
   - un phényle, 4-fluorophényle, un 2-méthylphényle, un 4-méthylphényle, un 4-isopropylphényle, un 4-butylphényle, un 4-*tert*-butylphényle, un 4-(trifluorométhyl)phényle, un 4-méthoxyphényle, un 4-butoxyphényle, un 4-*tert-*butoxyphényle, un 3-(trifluorométhoxy)phényle, un 4-(trifluorométhoxy)phényle, un 4-(difluorométhoxy)phényle, un 4-(1,1,2,2-tétrafluoroéthoxy)phényle, un 4-(éthylthio)phényle, un 3-[(trifluorométhyl)thio]phényle, un 4-[(trifluorométhyl)thio]phényle, un 4-[(2,2,2-trifluoroéthyl)thio]phényle, un 4-(méthylsulfonyl)phényl, un 4-[[éthyl(propyl)amino]méthyl]phényle, un 4-[[méthyl(2,2,2-trifluoroéthyl)amino]méthyl]phényle, un 3-chloro-4-(trifluorométhyl)phényle, un 2-fluoro-4-(trifluorométhyl)phényle, un 3-fluoro-4-(trifluorométhyl)phényle, un 3-fluoro-4-propoxyphényle, un 3-chloro-4-(trifluorométhoxy)phényle, un 3,5-bis(trifluorométhyl)phényle, un 4-(méthoxycarbonyl)phényle, un biphényl-4-yle, un 4-(1*H*-1,2,4-triazol-1-yl)phényle, un 4-(1,2,3-thiadiazol-4-yl)phényle ;
   - un benzyle, un [3,5-difluoro-4-(trifluorométhyl)phényl]méthyle ;
   - un pyridin-4-yle, un 6-(trifluorométhyl)pyridin-3-yle, un thiéno[3,2-b]thién-2-yle, un 5-chloro-1-benzothién-2-yle ;
- R₂ représente un 4-bromophényle, un 4-chlorophényle, un 4-fluorophényle, un 4-méthoxyphényle, un 4-(méthylthio)phényle, un 4-hydroxyphényle, un 4-[[(3,3,3-trifluoropropyl)sulfonyl]oxy]phényle, un 4-[(propylsulfonyl)oxy]phényle, un 2,4-dichlorophényle, un 4-(trifluorométhoxy)phényle, un 4-[(trifluorométhyl)thio]phényle, un 4-[2-(diméthylamino)éthoxy]phényle, un 4-[(3-hydroxypropyl)thio]phényle, un 4-[(2-acétamidoéthyl)thio]phényle, un 4-[[3-[(méthylsulfonyl)amino]propyl]thio]phényle ;
- R₃ représente un 2-chlorophényle, un 4-chlorophényle, un 2,4-dichlorophényle, un 4-bromo-2-chlorophényle, un 2-chloro-4-fluorophényle, un 2-chloro-4-méthoxyphényle, un 2-chloro-4-(méthylthio)phényle, un 2-chloro-4-(éthylthio)phényle, un 2-chloro-4-[(3,3,3-trifluoropropyl)thio]phényle, un 2-chloro-4-(2,2,2-trifluoroéthoxy)phényle, un 2-chloro-4-[2-(diméthylamino)éthoxy]phényle, un 2-chloro-4-[2-(méthylthio)éthoxy]phényle, un 2-chlore-4-[(3-hydroxypropyl)thio]phényle, un 2-chloro-4-[(2-acétamidoéthyl)thio]phényle, un 2-chloro-4-[[3-[(méthylsulfonyl)amino]propyl]thio]phényle, un 4-[(2-aminoéthyl)thio]-2-chlorophényle, un 2-chloro-4-[[2-(diméthylamino)éthyl]thio]phényle, un 2-chloro-4-[[2-(diéthylamino)éthyl]thio]phényle, un 2-chloro-4-[[2-(isopropylamino)éthyl]thio]phényle, un 2-chloro-4-[(2-formamidoéthyl)thio]phényle, un 2-chloro-4-[[2-[(méthylsulfonyl)amino]éthyl]thio]phényle, un 2-chloro-4-[[2-[(trifluoroacétyl)amino]éthyl]thio]phényl, un 2-chloro-4-[[2-[(cyclopropylcarbonyl)amino]éthyl]thio]phényle ;
- R₄ représente un atome d'hydrogène, un méthyle, un méthoxy, un hydroxy ; ainsi que leurs sels d'addition, leurs hydrates ou leurs solvats.

Parmi les composés de formule (I), objets de l'invention, on peut aussi citer également les composés pour lesquels :
- R₁ représente :
   - un 4-isopropylphényle, un 4-*tert*-butylphényle, un 4-(trifluorométhyl)phényle, un 4-(trifluorométhoxy)phényle, un 4-[(trifluorométhyl)thio]phényle, un 2-fluoro-4-(trifluorométhyl)phényle, un 3-fluoro-4-(trifluorométhyl)phényle ;
- R₂ représente un 4-bromophényle, un 4-chlorophényle, un 4-fluorophényle, un 4-méthoxyphényle, un 4-(méthylthio)phényle, un 4-[[(3,3,3-trifluoropropyl)sulfonyl]oxy]phényle, un 4-[(propylsulfonyl)oxy]phényle, un 4-[[3-[(méthylsulfonyl)amino]propyl]thio]phényle ;
- R₃ représente un 2-chlorophényle, un 2,4-dichlorophényle, un 4-bromo-2-chlorophényle, un 2-chloro-4-méthoxyphényle, un 2-chloro-4-(méthylthio)phényle, un 2-chloro-4-[2-(diméthylamino)éthoxy]phényle, un 2-chloro-4-[2-(méthylthio)éthoxy]phényle, un 2-chloro-4-[(3-hydroxypropyl)thio]phényle, un 2-chloro-4-[(2-acétamidoéthyl)thio]phényle, un 4-[(2-aminoéthyl)thio]-2-chlorophényle, un 2-chloro-4-[[2-(diméthylamino)éthyl]thio]phényle, un 2-chloro-4-[[2-(diéthylamino)éthyl]thio]phényle, un 2-chloro-4-[[2-(isopropylamino)éthyl]thio]phényle, un 2-chloro-4-[[2-[(méthylsulfonyl)amino]éthyl]thio]phényle, un 2-chloro-4-[(2-formamidoéthyl)thio]phényle, un 2-chloro-4-[[2-[(trifluoroacétyl)amino]éthyl]thio]phényl, un 2-chloro-4-[[2-[(cyclopropylcarbonyl)amino]éthyl]thio]phényle ;
- R₄ représente un atome d'hydrogène ;
   ainsi que leurs sels d'addition, leurs hydrates ou leurs solvats.

Parmi les composés de formule (I), objets de l'invention, on peut notamment citer les composés suivants :
- 5-(4-chlorophényl)-6-(2,4-dichlorophényl)-2-(4-isopropylbenzyl)-2,5-dihydro-3*H-*pyrazolo[4,3-c]pyridazin-3-one ;
- 2-(4-*tert*-butylbenzyl)-5-(4-chlorophényl)-6-(2,4-dichlorophényl)-2,5-dihydro-3*H*-pyrazolo[4,3-c]pyridazin-3-one ;
- 5-(4-chlorophényl)-6-(2,4-dichlorophényl)-2-[4-(trifluorométhyl)benzyl]-2,5-dihydro-3*H-*pyrazolo[4,3-c]pyridazin-3-one ;
- 5-(4-chlorophényl)-6-(2,4-dichlorophényl)-2-[2-fluoro-4-(trifluorométhyl)benzyl]-2,5-dihydro-3*H*-pyrazolo[4,3-c]pyridazin-3-one ;
- 5-(4-chlorophényl)-6-(2,4-dichlorophényl)-2-[4-[(trifluorométhyl)thio]benzyl]-2,5-dihydro-3*H-*pyrazolo[4,3-c]pyridazin-3-one ;
- 5-(4-bromophényl)-6-(2,4-dichlorophényl)-2-(4-isopropylbenzyl)-2,5-dihydro-3*H*-pyrazolo[4,3-c]pyridazin-3-one ;
- 5-(4-bromophényl)-2-(4-butylbenzyl)-6-(2,4-dichlorophényl)-2,5-dihydro-3*H-*pyrazolo[4,3-c]pyridazin-3-one;
- 5-(4-bromophényl)-6-(2,4-dichlorophényl)-2-[3-fluoro-4-(trifluorométhyl)benzyl]-2,5-dihydro-3*H-*pyrazolo[4,3-c]pyridazin-3-one ;
- 5-(4-bromophényl)-6-(2,4-dichlorophényl)-2-[4-(trifluorométhoxy)benzyl]-2,5-dihydro-3*H-*pyrazolo[4,3-c]pyridazin-3-one ;
- 5-(4-bromophényl)-6-(2,4-dichlorophényl)-2-[4-[(trifluorométhyl)thio]benzyl]-2,5-dihydro-3*H*-pyrazolo[4,3-c]pyridazin-3-one ;
- 6-(2,4-dichlorophényl)-5-[4-(méthylthio)phényl]-2-[4-(trifluorométhyl)benzyl]-2,5-dihydro-3*H*-pyrazolo[4,3-c]pyridazin-3-one ;
- 6-(2,4-dichlorophényl)-2-[2-fluoro-4-(trifluorométhyl)benzyl]-5-[4-(méthylthio)phényl]-2,5-dihydro-3*H-*pyrazolo[4,3-c]pyridazin-3-one ;
- 6-(2,4-dichlorophényl)-5-[4-(méthylthio)phényl]-2-[4-(trifluorométhoxy)benzyl]-2,5-dihydro-3*H-*pyrazolo[4,3-c]pyridazin-3-one ;
- 6-(2-chlorophényl)-5-(4-chlorophényl)-2-[4-[(trifluorométhyl)thio]benzyl]-2,5-dihydro-3*H-*pyrazolo[4,3-c]pyridazin-3-one ;
- 6-(2,4-dichlorophényl)-5-(4-méthoxyphényl)-2-[4-[(trifluorométhoxy)benzyl]-2,5-dihydro-3*H*-pyrazolo[4,3-c]pyridazin-3-one ;
- 6-(2,4-dichlorophényl)-5-(4-méthoxyphényl)-2-[4-[(trifluorométhyl)thio]benzyl]-2,5-dihydro-3*H-*pyrazolo[4,3-c]pyridazin-3-one ;
- 6-(2-chlorophényl)-5-(4-fluorophényl)-2-[4-[(trifluorométhyl)thio]benzyl]-2,5-dihydro-3*H-*pyrazolo[4,3-c]pyridazin-3-one ;
- 4-[6-(2,4-dichlorophényl)-3-oxo-2-[4-[(trifluorométhyl)thio] benzyl]-2,3-dihydro-5*H-*pyrazolo[4,3-c]pyridazin-5-yl]phényl-3,3,3-trifluoropropane-1-sulfonate ;
- 4-[6-(2,4-dichlorophényl)-3-oxo-2-[4-[(trifluorométhyl)thio] benzyl]-2,3-dihydro-5*H-*pyrazolo[4,3-c]pyridazin-5-yl]phénylpropane-1-sulfonate ;
- 6-(4-bromo-2-chlorophényl)-5-[4-(méthylthio)phényl]-2-[4-(trifluorométhyl)benzyl]-2,5-dihydro-3*H*-pyrazolo[4,3-*c*]pyridazin-3-one ;
- 6-{2-chloro-4-[(3-hydroxypropyl)thio]phényl}-5-[4-(méthylthio)phényl]-2-[4-(trifluorométhyl)benzyl]-2,5-dihydro-3*H-*pyrazolo[4,3-*c*]pyridazin-3-one ;
- 6-(4-bromo-2-chlorophényl)-5-[4-(méthylthio)phényl]-2-{4-[(trifluorométhyl)thio]benzyl}-2,5-dihydro-3*H-*pyrazolo[4,3-*c*]pyridazin-3-one ;
- *N*-(2-{[3-chloro-4-(5-[4-(méthylthio)phényl]-3-oxo-2-{4-[(trifluorométhyl)thio]benzyl}-3,5-dihydro-2*H*-pyrazolo[4,3-*c*]pyridazin-6-yl)phényl]thio}éthyl)acetamide ;
- 6-{2-chloro-4-[(3-hydroxypropyl)thio]phényl}-5-[4-(méthylthio)phényl]-2-{4-[(trifluorométhyl)thio]benzyl}-2,5-dihydro-3*H-*pyrazolo[4,3-*c*]pyridazin-3-one ;
- 6-(2-chloro-4-méthoxyphényl)-5-[4-(méthylthio)phényl]-2-[4-(trifluorométhyl)benzyl]-2,5-dihydro-3*H-*pyrazolo[4,3-*c*]pyridazin-3-one ;
- 6-(2-chloro-4-méthoxyphényl)-5-[4-(méthylthio)phényl]-2-[4-(trifluorométhoxy)benzyl]-2,5-dihydro-3*H-*pyrazolo[4,3-*c*]pyridazin-3-one ;
- 6-(2-chloro-4-méthoxyphényl)-5-[4-(méthylthio)phényl]-2-{4-[(trifluorométhyl)thio]benzyl}-2,5-dihydro-3*H*-pyrazolo[4,3-*c*]pyridazin-3-one ;
- 6-{2-chloro-4-[2-(diméthylamino)éthoxy]phényl}-5-[4-(méthylthio)phényl]-2-[4-(trifluorométhyl)benzyl]-2,5-dihydro-3*H-*pyrazolo[4,3-*c*]pyridazin-3-one ;
- 6-{2-chloro-4-[2-(méthylthio)éthoxy]phényl}-5-[4-(méthylthio)phényl]-2-[4-(trifluorométhyl)benzyl]-2,5-dihydro-3*H*-pyrazolo[4,3-*c*]pyridazin-3-one ;
- 6-[2-chloro-4-(méthylthio)phényl]-5-[4-(méthylthio)phényl]-2-[4-(trifluorométhyl)benzyl]-2,5-dihydro-3*H*-pyrazolo[4,3-*c*]pyridazin-3-one ;
- 6-[2-chloro-4-(méthylthio)phényl]-5-[4-(méthylthio)phényl]-2-{4-[(trifluorométhyl)thio]benzyl}-2,5-dihydro-3*H-*pyrazolo[4,3-*c*]pyridazin-3-one ;
- *N-*[3-({4-[6-(2,4-dichlorophényl)-3-oxo-2-{4-[(trifluorométhyl)thio]benzyl}-2,3-dihydro-5*H*-pyrazolo[4,3-*c*]pyridazin-5-yl]phényl}thio)propyl]méthanesulfonamide ;
- *N-*{3-[(4-{6-(2,4-dichlorophényl)-3-oxo-2-[4-(trifluorométhyl)benzyl]-2,3-dihydro-5*H-*pyrazolo[4,3-*c*]pyridazin-5-yl}phényl)thio]propyl}méthanesulfonamide ;
- 6-{4-[(2-aminoéthyl)thio]-2-chlorophényl}-5-[4-(méthylthio)phényl]-2-[4-(trifluorométhyl)benzyl]-2,5-dihydro-3*H-*pyrazolo[4,3-*c*]pyridazin-3-one ;
- 6-(2-chloro-4-{[2-(diméthylamino)éthyl]thio}phényl)-5-[4-(méthylthio)phényl]-2-[4-(trifluorométhyl)benzyl]-2,5-dihydro-3*H*-pyrazolo[4,3-*c*]pyridazin-3-one ;
- 6-(2-chloro-4-{[2-(diéthylamino)éthyl]thio}phényl)-5-[4-(méthylthio)phényl]-2-[4-(trifluorométhyl)benzyl]-2,5-dihydro-3*H*-pyrazolo[4,3-*c*]pyridazin-3-one ;
- 6-(2-chloro-4-{[2-(isopropylamino)éthyl]thio}phényl)-5-[4-(méthylthio)phényl]-2-[4-(trifluorométhyl)benzyl]-2,5-dihydro-3*H*-pyrazolo[4,3-*c*]pyridazin-3-one ;
- *N*-{2-[(3-chloro-4-{5-[4-(méthylthio)phényl]-3-oxo-2-[4-(trifluorométhyl)benzyl]-3,5-dihydro-2*H*-pyrazolo[4,3-*c*]pyridazin-6-yl}phényl)thio]éthyl}méthanesulfonamide ;
- *N*-{2-[(3-chloro-4-{5-[4-(méthylthio)phényl]-3-oxo-2-[4-(trifluorométhyl)benzyl]-3,5-dihydro-2*H*-pyrazolo[4,3-*c*]pyridazin-6-yl}phényl)thio]éthyl}formamide ;
- *N*-{2-[(3-chloro-4-{5-[4-(méthylthio)phényl]-3-oxo-2-[4-(trifluorométhyl)benzyl]-3,5-dihydro-2*H*-pyrazolo[4,3-*c*]pyridazin-6-yl}phényl)thio]éthyl}-2,2,2-trifluoroacétamide ;
- *N*-{2-[(3-chloro-4-{5-[4-(méthylthio)phényl]-3-oxo-2-[4-(trifluorométhyl)benzyl]-3,5-dihydro-2*H*-pyrazolo[4,3-*c*]pyridazin-6-yl}phényl)thio]éthyl}cyclopropanecarboxamide ;
   ainsi que leurs sels d'addition, leurs hydrates ou leurs solvats.

Dans ce qui suit, on entend par groupe protecteur Pg un groupe qui permet, d'une part, de protéger une fonction réactive telle qu'un hydroxy ou une amine pendant une synthèse et, d'autre part, de régénérer la fonction réactive intacte en fin de synthèse. Des exemples de groupes protecteurs ainsi que des méthodes de protection et de déprotection sont données dans "Protective Group in Organic Synthesis", Green et al., 2nd Edition (John Wiley & Sons, Inc., New York), 1991.

On entend par groupe partant, dans ce qui suit, un groupe pouvant être facilement clivé d'une molécule par rupture d'une liaison hétérolytique, avec départ d'une paire électronique. Ce groupe peut ainsi être remplacé facilement par un autre groupe lors d'une réaction de substitution, par exemple. De tels groupes partants sont, par exemple, les halogènes ou un groupe hydroxy activé tel qu'un méthanesulfonate, benzènesulfonate, p-toluènesulfonate, triflate, acétate, etc. Des exemples de groupes partants ainsi que des références pour leur préparation sont donnés dans "Advances in Organic Chemistry", J. March, 3rd Edition, Wiley Interscience, 1985, p. 310-316.

Conformément à l'invention, on peut préparer les composés de formule (I) selon un procédé qui est **caractérisé en ce que :**
On fait réagir, en présence d'une base, un composé de formule : 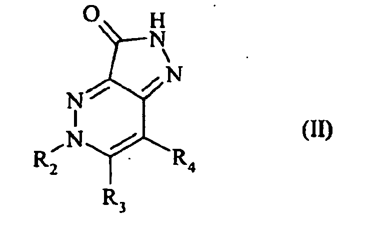 dans laquelle R₂, R₃ et R₄ sont tels que définis pour un composé de formule (I) avec un composé de formule :

   Y-CH₂-R₁ (III)

   dans laquelle R₁ est tel que défini pour un composé de formule (I) et Y représente un groupe partant tel que défini ci-dessus, de préférence un atome d'halogène ou un groupe hydroxy activé tel qu'un groupe méthanesulfonate, benzènesulfonate, p-toluènesulfonate ou triflate.

La réaction s'effectue en présence d'une base telle qu'un hydrure de métal alcalin, l'hydrure de sodium par exemple, ou un carbonate de métal alcalin, le carbonate de potassium ou le carbonate de césium par exemple, dans un solvant tel que le dioxane, le N,N-diméthylformamide, le tétrahydrofurane ou un mélange de ces solvants, et à une température comprise entre la température ambiante et la température de reflux du solvant.

Selon une variante du procédé, on peut également préparer un composé de formule (I) dans laquelle R₄ = -OH par réaction d'un composé de formule (I) dans laquelle R₄ = -OCH₃ avec un acide fort tel que l'acide bromhydrique, dans un solvant tel que l'acide acétique et à une température comprise entre la température ambiante et la température de reflux du solvant.

Selon une autre variante, on peut préparer un composé de formule (I) dans laquelle R₂ ou R₃ = hydroxyphényle par réaction d'un composé de formule (I) dans laquelle R₂ ou R₃ = méthoxyphényle avec BBr₃, dans un solvant tel que le dichlorométhane et à une température comprise entre -20°C et la température ambiante.

Selon une autre variante, on peut préparer un composé de formule (I) dans laquelle R₂ ou R₃ = AlkS(O)ₙO-phényle par réaction d'un composé de formule (I) dans laquelle R₂ ou R₃ = hydroxyphényle avec un Halogénure de formule, Hal-S(O)ₙAlk, dans laquelle Hal représente un atome d'halogène de préférence le chlore, en présence d'une base telle que la triéthylamine, dans un solvant tel que le dichlorométhane et à une température comprise entre -20°C et la température ambiante.

Selon une autre variante, on peut préparer un composé de formule (I) dans laquelle R₂ ou R₃ = phényl-O(CH₂)ₘR₅ par réaction d'un composé de formule (I) dans laquelle R₂ ou R₃ = phényl-OH avec un composé de formule Y-(CH₂)ₘR₅, Y étant tel que défini précédemment et m et R₅ étant tel que définis pour un composé de formule (I). La réaction s'effectue en présence d'une base telle qu'un hydrure de métal alcalin, l'hydrure de sodium par exemple, ou un carbonate de métal alcalin, le carbonate de potassium ou le carbonate de césium par exemple, dans un solvant tel que le dioxane, le N,N-diméthylformamide, le tétrahydrofurane, l'acétonitrile, l'éthanol ou un mélange de ces solvants, et à une température comprise entre la température ambiante et la température de reflux du solvant.

Selon une autre variante, on peut préparer un composé de formule (I) dans laquelle R₂ ou R₃ = phényl-S(CH₂)ₘR₆ par réaction d'un composé de formule (I) dans laquelle R₂ ou R₃ = phényl-Br avec un composé de formule HS(CH₂)ₘR₆, m et R₆ étant tel que définis pour un composé de formule (I). La réaction s'effectue en présence d'une base et du système catalytique Pd₂(dba)₃/xantphos selon les conditions opératoires décrites dans Tetrahedron 2005, 61, 5253-5259.

Selon une autre variante, on peut préparer un composé de formule (I) dans laquelle R₂ ou R₃ = phényl-S(CH₂)ₘR₆ dans lequel R₆ représente un groupe -NR₇R₈, NR₇COR₈ ou NR₇SO₂R₉ à partir des composés de formule (I) dans laquelle R₂ ou R₃ = phényle-S-(CH₂)ₘNH₂ en utilisant des méthodes bien connues de l'homme de l'art telles que l'alkylation ou l'acylation des amines.

Selon une autre variante on peut préparer les composés de formule (I) dans laquelle R₂ ou R₃ = phényl-S(CH₂)ₘR₆ dans lequel _{R6} représente un groupe -NR₇SO₂R₉ à partir des composés de formule (I) dans laquelle R₂ ou R₃ = phényl-S(CH₂)ₘ-oH et après activation de la fonction hydroxyle et réaction avec un composé de formule HNR₇SO₂R₉ selon les méthodes bien connues de l'homme de l'art.

Les composés de formule (I) ainsi obtenus peuvent être ultérieurement séparés du milieu réactionnel et purifiés selon les méthodes classiques, par exemple par cristallisation ou chromatographie.

Les composés de formules (II) se préparent par cyclisation d'un composé de formule : 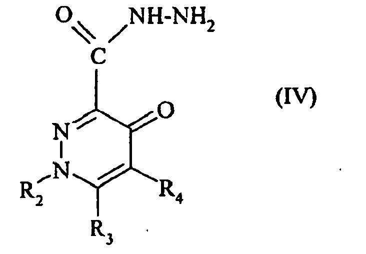 dans laquelle R₂, R₃ et R₄ sont tels que définis pour un composé de formule (I). La réaction s'effectue en présence d'un acide tel que l'acide chlorhydrique, dans un solvant tel que la pyridine et à une température comprise entre la température ambiante et la température de reflux du solvant. On peut également effectuer la réaction en présence d'une base telle que le *tert*-butylate de potassium, dans un solvant tel que l'éthanol et à une température comprise entre la température ambiante et la température de reflux du solvant.

Les composés de formule (III) sont connus, disponibles dans le commerce ou se préparent selon des méthodes connues à partir des composés de formule :

HO-CH₂-R₁ (V)

dans laquelle R₁ est tel que défini pour un composé de formule (I). Ainsi par exemple, lorsque dans un composé de formule (III) Y représente un atome d'halogène, on traite un composé de formule (V) par un agent d'halogénation tel que SOCl₂, PCl₅, PBr₃, HBr ou BBr₃, dans un solvant tel que le dichlorométhane ou l'éther et à une température comprise entre 0°C et la température ambiante. On peut également utiliser comme agent d'halogénation le chlorure de méthanesulfonyle, en présence d'une base telle que la triéthylamine, dans un solvant tel que le dichlorométhane, et à une température comprise entre 0°C et la température ambiante.

Lorsque dans un composé de formule (III), Y représente un méthanesulfonate, un benzènesulfonate, un p-toluènesulfonate ou un trifluorométhanesulfonate, on fait réagir un composé de formule (V) avec un chlorure de sulfonyle de formule W-SO₂-Cl dans laquelle W représente un méthyle, un phényle, un p-tolyle ou un trifluorométhyle. La réaction s'effectue en présence d'une base telle que la triéthylamine, la pyridine ou la N,N-diisopropyléthylamine, dans un solvant tel que le dichloroinéthane ou le toluène et à une température comprise entre -20°C et la température de reflux du solvant.

Les composés de formule (IV) se préparent par réaction d'un composé de formule : 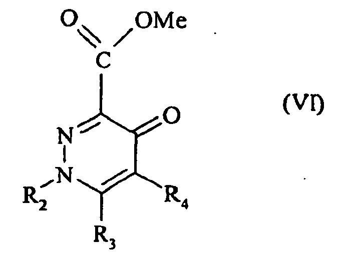 dans laquelle R₂, R₃ et R₄ sont tels que définis pour un composé de formule (I) avec l'hydrazine monohydrate, dans un solvant tel que le dioxane, le dichlorométhane, le méthanol, ou un mélange de ces solvants et à une température comprise entre 0°C et la température ambiante.

Les composés de formule (V) sont connus, disponibles dans le commerce ou se préparent selon des méthodes connues à partir des composés de formule : 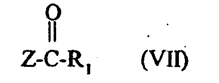 dans laquelle R₁ est tel que défini pour un composé de formule (I) et Z représente un hydroxy ou un (C₁-C₂)alcoxy. La réaction s'effectue en présence d'un agent réducteur tel que le borane, l'hydrure d'aluminium et de lithium dans un solvant tel que l'éther diéthylique ou le tétrahydrofurane et à une température comprise entre -20°C et la température de reflux du solvant.

Les composés de formule (VI) se préparent par estérification des composés de formule : 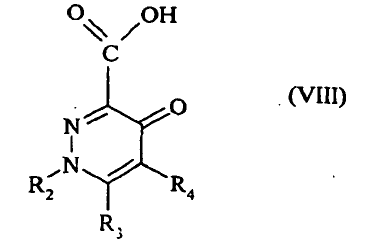 dans laquelle R₂, R₃ et R₄ sont tels que définis pour un composé de formule (I) selon des méthodes classiques. Par exemple la réaction s'effectue par action d'un alcool, en présence de chlorure d'isocyanatosulfuryle et de triéthylamine selon la méthode décrite dans Synthesis, 1982, 506-508.

Les composés de formule (VII) sont connus, disponibles dans le commerce ou se préparent selon des méthodes connues.

Les composés de formule (VIII) se préparent selon des méthodes connues telles que celles décrites dans le brevet US 4,345,934.

Ainsi, par exemple, on fait réagir un dérivé de pyrone de formule : 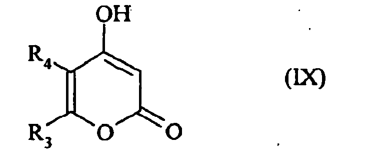 dans laquelle R₃ et R₄ sont tels que définis pour un composé de formule (I), ou un sel du composé de formule (IX) préparé en traitant le dérivé de pyrone avec une base soluble dans l'eau telle que un carbonate de sodium ou de potassium, avec un sel de diazonium, de préférence le chlorure, préparé par réaction classique de diazotation (action du nitrite de sodium en milieu acide dans l'eau à 0°C) d'une amine de formule :

R₂-NH₂ (X)

dans laquelle R₂ est tel que défini pour un composé de formule (I). La réaction s'effectue en milieu aqueux, à une température comprise entre -10°C et la température ambiante. Le composé hydrazinone ainsi obtenu de formule : 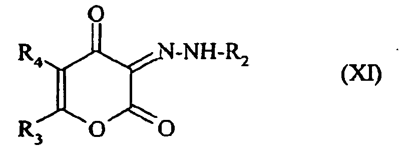 est ensuite traité par un acide fort tel que l'acide chlorhydrique, en milieux aqueux, à une température comprise entre 0°C et 100°C, pour obtenir, après réarrangement, le composé de formule (VIII) attendu.

Les composés de formule (IX) se préparent selon des méthodes connues telles que celles décrites dans le brevet US 5,808,062. Ainsi, par exemple, les composés de formule (IX) se préparent selon le SCHEMA I ci-après dans lequel R₃ et R₄ sont tels que définis pour un composé de formule (I) : 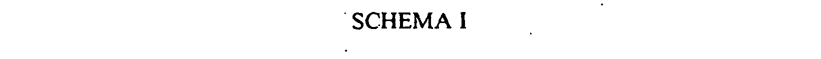 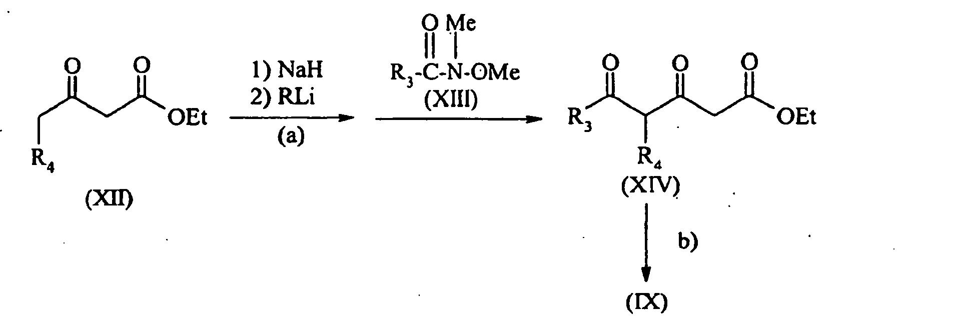 A l'étape a) un β-cétoester de formule (XII) est déprotoné par action d'une base telle qu'un hydrure de métal alcalin, l'hydrure de sodium par exemple, dans un solvant tel que l'éther diéthylique où le tétrahydrofurane, à une température comprise entre -10°C et 0°C. L'action d'une deuxième base forte telle qu'un alkylithium (Rli), le lithium diisopropylamide par exemple, permet d'obtenir un dianion qui est ensuite mis en réaction avec un agent acylant, un amide de formule (XIII) par exemple, à une température comprise entre 0°C et la température ambiante pour obtenir un composé de formule (XIV).

A l'étape b), le composé de formule (XIV) est ensuite cyclisé en composé de formule (IX) par action d'un acide fort tel que l'acide sulfurique à une température comprise entre 0°C et la température ambiante.

Les composés de formule (X) sont connus.

Les composés de formule (XII) sont connus.

Les composés de formule (XIII) se préparent par réaction de la N-méthoxyméthanamine avec un halogénure d'acide de formule R₃COHal (XV) dans laquelle R₃ est tel que défini pour un composé de formule (I) et Hal représente un atome d'halogène, de préférence le chlore, en présence d'une base telle que le triéthylamine, dans un solvant tel que le dichlorométhane et à une température comprise entre 0°C et la température ambiante.

Les composés de formule (XV) sont connus ou se préparent selon des méthodes connues.

L'invention, selon un autre des ses aspects, a également pour objet les composés de formule (II). Ces composés sont utiles comme intermédiaires de synthèse des composés de formule (I).

Ainsi, l'invention a pour objet des composés de formule : 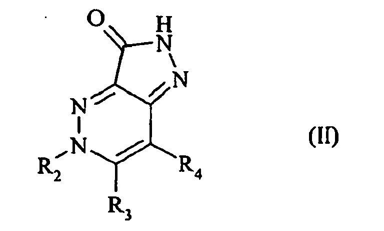 dans laquelle :
- R₂ représente un phényle non substitué ou substitué une ou plusieurs fois par des substituants choisis indépendamment parmi un atome d'halogène, un hydroxy, un groupe Alk, un groupe OAlk, un groupe S(O)ₙAlk ou un groupe OS(O)ₙAlk ;
- R₃ représente un phényle non substitué ou substitué une ou plusieurs fois par des substituants choisis indépendamment parmi un atome d'halogène, un hydroxy, un groupe Alk, un groupe OAlk, un groupe S(O)ₙAlk ou un groupe OS(O)ₙAlk ;
- R₄ représente un atome d'hydrogène ou un (C₁-C₄)alkyle, un (C₁-C₄)alcoxy, un hydroxy ;
- n représente 0, 1 ou 2 ;
- Alk représente un (C₁-C₄)alkyle non substitué ou substitué une ou plusieurs fois par un atome de fluor ;

Les EXEMPLES suivants décrivent la préparation de certains composés conformes à l'invention. Ces exemples ne sont pas limitatifs et ne font qu'illustrer la présente invention. Les numéros des composés exemplifiés renvoient à ceux donnés dans le TABLEAU (VI) ci-après, qui illustre les structures chimiques et les propriétés physiques de quelques composés selon l'invention.

Dans les Préparations et dans les Exemples on utilise les abréviations suivantes :
éther : éther diéthylique
éther iso : éther diisopropylique
DMSO : diméthylsulfoxyde
DMF : N,N-diméthylformamide
THF : tétrahydrofurane
DCM : dichlorométhane
AcOEt : acétate d'éthyle
DIPEA : diisopropyléthylamine
TFA : acide trifluoroacétique
Pd₂(dba)₃: tris(dibenzylidèneacétone)dipalladium(0)
Xantphos: 4,5-bis(diphénylphosphino)-9,9-diméthylxanthène;

Ether chlorhydrique 2N : solution 2N d'acide chlorhydrique dans l'éther diéthylique
F : point de fusion
TA : température ambiante
Eb : température d'ébullition
CLHP : chromatographie liquide haute performance
Silice H : gel de silice 60 H commercialisé par Merck (DARMSTAD)
Solution tampon pH = 2 : solution de 16,66 g de KHSO₄ et 32,32 g de K₂SO₄ dans 1 litre d'eau.

Les spectres de résonance magnétique nucléaire du proton (RMN ¹H) sont enregistrés dans le DMSO-d₆. Les déplacements chimiques δ sont exprimés en parties par million (ppm). Pour l'interprétation des spectres, on utilise les abréviations suivantes : s : singulet, d : doublet, t : triplet, q : quadruplet, m : massif, mt : multiplet, se : singulet élargi, dd : doublet dédoublé, spt : septuplet.

Les composés selon l'invention sont analysés par couplage LC/UV/MS (chromatographie liquide/détection UV/spectrométrie de masse). On mesure le pic moléculaire (MH⁺) et le temps de rétention (tr) en minutes.

### Méthode 1 : M1

On utilise une colonne Symmetry C18 de 2,1 x 50 mm, 3,5 µm, à 30°C, débit 0,4 ml/minute.

L'éluant est composé comme suit :
- solvant A : 0,005 % d'acide trifluoroacétique (TFA) dans l'eau à pH 3,15 ;
- solvant B : 0,005 % de TFA dans l'acétonitrile.

Gradient :

| Temps (mn) | % A | % B |
|---|---|---|
| 0 | 100 | 0 |
| 10 | 10 | 90 |
| 15 | 10 | 90 |
| 16 | 100 | 0 |
| 20 | 100 | 0 |

La détection UV est effectuée à λ = 210 nm -220 nm et la détection de masse en mode ionisation chimique ESI positif afin d'observer les ions issus de la protonation des composés analysés (MH⁺).

### Méthode 2 : M2

On utilise une colonne XTerra MS C18 de 2,1 x 50 mm, 3,5 µm, à 30°C, débit 0,4 ml/minute.

L'éluant est composé comme suit :
- solvant A : AcONH₄ 10 mM à pH = 7 ;
- solvant B : acétonitrile.

### Gradient :

| Temps (mn) | % A | % B |
|---|---|---|
| 0 | 100 | 0 |
| 10 | 10 | 90 |
| 15 | 10 | 90 |
| 16 | 100 | 0 |
| 20 | 100 | 0 |

La détection UV est effectuée λ = 220 nm et la détection de masse en mode ionisation chimique ESI positif afin d'observer les ions issus de la protonation des composés analysés (MH⁺).

### Méthode 3 : M3

On utilise une colonne Varian C18 de 2 x 100 mm, 3,5 µm, débit 0,3 ml/minute. L'éluant est composé comme suit :
- solvant A : AcONH₄ 10 mM à pH = 6,5 ;
- solvant B : acétonitrile.

### Gradient :

| Temps (mn) | % A | % B |
|---|---|---|
| 0 | 95 | 5 |
| 25 | 10 | 90 |
| 30 | 10 | 90 |
| 32 | 95 | 5 |
| 40 | 95 | 5 |

La détection UV est effectuée λ = 220 nm et la détection de masse en mode ionisation chimique ESI positif afin d'observer les ions issus de la protonation des composés analysés (MH⁺).

### Méthode 4 : M4

On utilise une colonne XTerra C18 de 2,1 x 50 mm, 3,5 µm, à 30°C, débit 0,3 ml/minute.

L'éluant est composé comme suit :
- solvant A : 0,005 % d'acide trifluoroacétique dans l'eau ;
- solvant B : acétonitrile.

### Gradient :

| Temps (mn) | % A | % B |
|---|---|---|
| 0 | 95 | 5 |
| 17 | 10 | 90 |
| 22 | 10 | 90 |
| 23 | 95 | 5 |
| 30 | 95 | 5 |

La détection UV est effectuée λ = 220 nm et la détection de masse en mode ionisation chimique ESI positif afin d'observer les ions issus de la protonation des composés analysés (MH⁺).

### Méthode 5 : M5

On utilise une colonne Symmetry C18 de 2,1 x 50 mm, 3,5 µm, débit 0,4 ml/minute.

L'éluant est composé comme suit :
- solvant A : 0,005 % d'acide trifluoroacétique dans l'eau ;
- solvant B : 0,005 % d'acide trifluoroacétique dans acétonitrile.

### Gradient :

| Temps (mn) | % A | % B |
|---|---|---|
| 0 | 100 | 0 |
| 10 | 10 | 90 |
| 15 | 10 | 90 |
| 16 | 100 | 0 |
| 20 | 100 | 0 |

La détection UV est effectuée λ = 220 nm et la détection de masse en mode ionisation chimique ESI positif afin d'observer les ions issus de la protonation des composés analysés (MH⁺).

### Méthode 6 : M6

On utilise une colonne Symmetry C18 de 2,1 x 50 mm, 3,5µ, débit 0,4m1/minute. L'éluant est composé comme suit :
- Solvant A : 0,005 % d'acide trifluoroacétique dans l'eau à pH = 3,1 ;
- Solvant B : 0,005 % d'acide trifluoroacétique dans l'acétonitrile.

### Gradient :

| Temps (mn) | % A | % B |
|---|---|---|
| 0 | 100 | 0 |
| 20 | 10 | 90 |
| 30 | 10 | 90 |
| 31 | 100 | 0 |
| 35 | 100 | 0 |

La détection UV est effectuée λ = 210 - 220 nm et la détection de masse en mode ionisation chimique ESI positif afin d'observer les ions issus de la protonation des composés analysés (MH⁺).

### PREPARATIONS

### 1. Préparations des composés de formule (XIII) :

### Préparation 1.1

### 2,4-Dichloro-N-méthoxy-N-méthylbenzamide

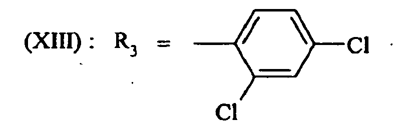

On refroidit au bain de glace un mélange de 32,6 g de chlorhydrate de N-méthoxyméthanamine et de 91,2 ml de triéthylamine dans 500 ml de DCM, puis ajoute lentement 47 ml de chlorure de 2,4-dichlorobenzoyle et laisse sous agitation en laissant remonter la température à T.A. Après 1 heure d'agitation à T.A, on ajoute de l'eau au mélange réactionnel, extrait au DCM, sèche la phase organique sur Na₂SO₄ et évapore le solvant sous vide. On obtient 53 g du composé attendu.

### Préparation 1.2

### 4-Chloro-N-méthoxy-N-méthylbenzamide

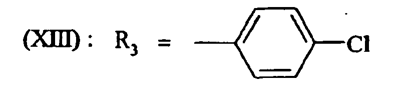

On refroidit au bain de glace un mélange de 31,2 g de chlorhydrate de N-méthoxyméthanamine et de 91,2 ml de triéthylamine dans 500 ml de DCM, puis ajoute lentement 56 g de chlorure de 4-chlorobenzoyle et laisse 4 heures sous agitation à T.A. On ajoute un mélange de glace/eau, extrait au DCM, lave la phase organique par une solution tampon pH = 2, sèche sur Na₂SO₄ et évapore le solvant sous vide. On obtient 60 g du composé attendu.

### Préparation 1.3

### 2-Chloro-N-méthoxy-N-méthylbenzamide

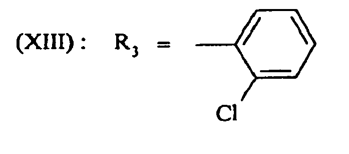

A un mélange de 37,33 g de chlorhydrate de N-méthoxy méthanamine et de 104 ml de triéthylamine dans 500 ml de DCM, on ajoute lentement 50 ml de chlorure de 2-chlorobenzoyle et laisse une nuit sous agitation à T.A. On ajoute de l'eau au mélange réactionnel, extrait au DCM, lave la phase organique par une solution tampon pH = 2, sèche sur Na₂SO₄ et évapore le solvant sous vide. On obtient 78,57 g du composé attendu.

### 2. Préparations des composés de formule (IX) :

### Préparation 2.1

### 6-(2,4-Dichlorophényl)-4-hydroxy-2H-pyran-2-one

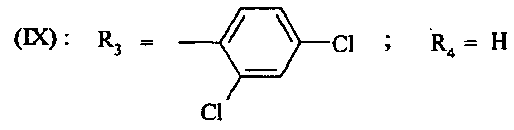

On refroidit à 0°C, sous atmosphère d'azote, une suspension de 5,8 g d'hydrure de sodium à 60% dans l'huile dans 300 ml de THF, puis ajoute lentement, 18,9 g de 3-oxobutanoate d'éthyle et laisse 5 minutes sous agitation à 0°C. On ajoute ensuite, goutte à goutte à 0°C, 97 ml d'une solution 1,5 M de lithium diisopropylamide mono(tétrahydrofurane) dans le cyclohexane et laisse 20 minutes sous agitation à 0°C. On ajoute enfin, lentement 34 g du composé de la Préparation 1.1, laisse sous agitation en laissant remonter la température à T.A et agite une nuit à T.A. On ajoute un mélange de glace/HCl 10%, extrait le mélange réactionnel à l'AcOEt, sèche la phase organique sur Na₂SO₄ et évapore le solvant sous vide. On reprend le résidu avec 300 ml d'H₂SO₄ concentré et laisse 4 heures sous agitation à T.A. On verse le mélange réactionnel sur de la glace, laisse sous agitation, essore le précipité formé et le lave à l'éther iso puis au pentane. On obtient 26 g du composé attendu après séchage.

### Préparation 2.2

### 6-(4-Chlorophényl)-4-hydroxy-5-méthyl-2H-pyran-2-one

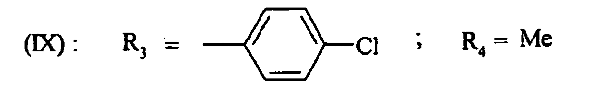

On refroidit à 0°C, sous atmosphère d'azote, une suspension de 4,7 g d'hydrure de sodium à 60% dans l'huile dans 100 ml de THF, puis ajoute lentement, une solution de 17 g de 3-oxopentanoate d'éthyle dans 100 ml de THF et laisse 5 minutes sous agitation à 0°C. On ajoute ensuite, goutte à goutte, à 0°C, 86,5 ml d'une solution 1,5 M de lithium diisopropylamide mono(tétrahydrofurane) dans le cyclohexane et laisse 20 minutes sous agitation à 0°C. On ajoute enfin, lentement une solution de 23,5 g du composé de la Préparation 1.2 dans 50 ml de THF laisse sous agitation en laissant remonter la température à T.A et agite une nuit à T.A. On ajoute un mélange de glace/HCl 10%, extrait le mélange réactionnel à l'AcOEt, sèche la phase organique sur Na₂SO₄ et évapore le solvant sous vide. On reprend le résidu avec 150 ml d'H₂SO₄ concentré et laisse 3 heures sous agitation à T.A. On verse le mélange réactionnel sur de la glace, laisse sous agitation, essore le précipité formé et le lave à l'éther iso puis au pentane. On obtient 25 g du composé attendu après séchage.

### Préparation 2.3

### 6-(2,4-Dichlorophényl)-4-hydroxy-5-méthoxy-2H-pyran-2-one

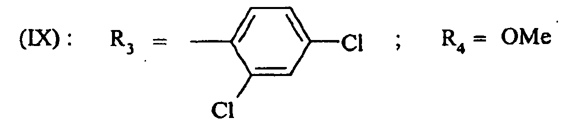

On refroidit à 0°C sous atmosphère d'azote, une suspension de 6 g d'hydrure de sodium à 60 % dans l'huile dans 300 ml de THF, ajoute lentement une solution de 21,9 g de 4-méthoxy-3-oxobutanoate de méthyle dans 100 ml de THF et laisse 5 minutes sous agitation à 0°C. On ajoute ensuite, goutte à goutte et à 0°C, 100 ml d'une solution 1,5 M de lithium diisopropylamide mono(tétrahydrofurane) dans le cyclohexane et laisse 2 heures sous agitation à 0°C. On ajoute enfin lentement une solution de 35,11 g du composé de la Préparation 1.1 dans 100 ml de THF, laisse sous agitation en laissant remonter la température à T.A et agite une nuit à T.A. On concentre le mélange réactionnel sous vide, reprend le résidu dans un mélange eau/HCl 10%, extrait à l'AcOEt, sèche la phase organique sur Na₂SO₄ et évapore le solvant sous vide. On reprend le résidu avec 200 ml d'H₂SO₄ concentré et laisse 3 heures sous agitation à T.A. On verse le mélange réactionnel sur de la glace, extrait au DCM, sèche la phase organique sur Na₂SO₄ et évapore le solvant sous vide. On dissout le résidu dans une solution de K₂CO₃ à 10%, lave à l'éther, acidifie la phase aqueuse par ajout d'une solution d'HCl à 10% et essore le produit cristallisé formé. On obtient 8,5 g du composé attendu après séchage.

### Préparation 2.4

### 6-(2-Chlorophényl)-4-hydroxy-2H-pyran-2-one

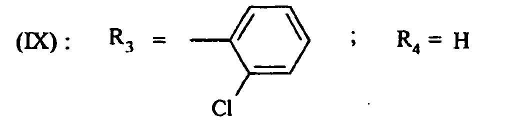

On refroidit à 0°C sous atmosphère d'azote, une suspension de 6 g d'hydrure de sodium à 60 % dans l'huile dans 50 ml de THF, puis ajoute lentement 19,18 ml de 3-oxobutanoate d'éthyle et laisse 5 minutes sous agitation à 0°C. On ajoute ensuite, goutte à goutte à 0°C, 100 ml d'une solution 1,5 M de lithium diisopropylamide mono(tétrahydrofurane) dans le cyclohexane et laisse 20 minutes sous agitation à 0°C. On ajoute enfin lentement 34 g du composé de la Préparation 1.3, laisse sous agitation en laissant remonter la température à T.A et agite 48 heures à T.A. On ajoute un mélange de glace/tampon pH = 2, extrait le mélange réactionnel à l'AcOEt, sèche la phase organique sur Na₂SO₄ et évapore le solvant sous vide. On reprend le résidu avec 140 ml dH₂SO₄ concentré et laisse 4 heures sous agitation à T.A. On verse le mélange réactionnel sur de la glace, laisse sous agitation, essore le précipité formé et le lave à l'éther iso puis au pentane. On obtient 28,44 g du composé attendu après séchage.

### 3. Préparations des composés de formule (VIII) :

### Préparation 3.1

### Acide 1-(4-chlorophényl)-6-(2,4-dichlorophényl)-4-oxo-1,4-dihydropyridazine-3-carboxylique.

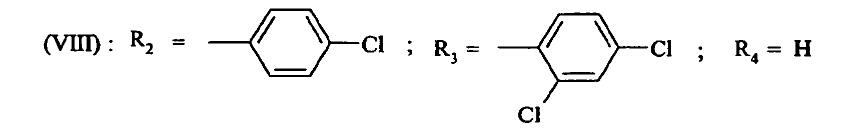

A une solution de 34,7 g de 4-chloroaniline dans 70 ml d'acide acétique on ajoute 300 ml d'HCl concentré, refroidit à 0°C, ajoute lentement une solution de 18,8 g de nitrite de sodium dans 60 ml d'eau et laisse 1 heure 30 minutes sous agitation à 0°C pour obtenir une solution de chlorure de diazonium. En parallèle on laisse sous agitation une solution de 70 g du composé de la Préparation 2.1 et 420 g de Na₂CO₃ dans 500 ml d'eau, ajoute lentement la solution de chlorure de diazonium froide et laisse 2 heures sous agitation à T.A. On essore le précipité formé et le lave à l'éther iso puis au pentane. On reprend le précipité dans 1 litre d'HCl concentré, laisse 1 heure sous agitation à T.A., et chauffe à reflux pendant 5 heures. Après refroidissement du mélange réactionnel, on ajoute 2 litres d'eau, laisse une nuit sous agitation à T.A essore le précipité formé et le lave à l'éther iso puis au pentane. On obtient 27,8 g du composé attendu.

### Préparation 3.2

### Acide 1-(4-bromophényl)-6-(2,4-dichiorophényl)-4-oxo-1,4-dihydropyridazine-3-carboxylique.

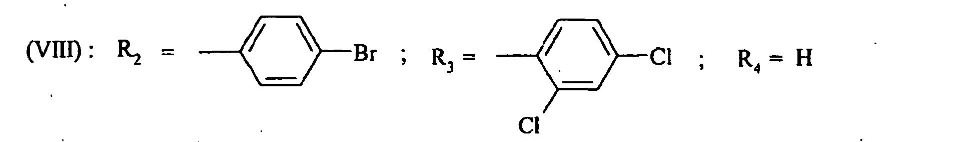

A une solution de 17,4 g de 4-bromoaniline dans 50 ml d'acide acétique on ajoute 200 ml d'HCl concentré, refroidit à 0°C, ajoute lentement une solution de 7 g de nitrite de sodium dans 30 ml d'eau et laisse 1 heure 30 minutes sous agitation à 0°C pour obtenir une solution de chlorure de diazonium. En parallèle on laisse sous agitation une solution de 36 g du composé de la Préparation 2.1 et 200 g de Na₂CO₃ dans 300 ml d'eau et 100 ml de THF, ajoute lentement la solution de chlorure de diazonium froide et laisse 2 heures sous agitation à T.A. On essore le précipité formé et le lave à l'éther iso puis au pentane. On reprend le précipité dans 300 ml d'HCl concentré, laisse 1 heure sous agitation à T.A., et chauffe à reflux pendant 4 heures. Après refroidissement du mélange réactionnel, on ajoute 2 litres d'eau, laisse une nuit sous agitation à T.A essore le précipité formé et le lave à l'éther iso puis au pentane. On obtient 18 g du composé attendu.

### Préparation 3.3

### Acide 6-(2,4-dichlorophényl)-1-[4-(méthylthio)phényl])-4-oxo-1,4-dihydropyridazine-3-carboxylique.

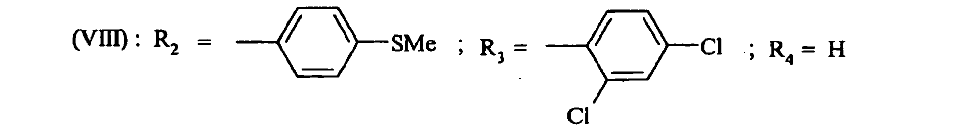

A une solution de 19,8 g de 4-(méthylthio)aniline dans 50 ml d'acide acétique on ajoute 200 ml d'HCl concentré, refroidit à 0°C, ajoute lentement une solution de 9,9 g de nitrite de sodium dans 30 ml d'eau et laisse 2 heures sous agitation à 0°C pour obtenir une solution de chlorure de diazonium. En parallèle on laisse sous agitation une solution de 35,8 g du composé de la Préparation 2.1 et 650 g de Na₂CO₃ dans 650 ml d'eau et 250 ml de THF, ajoute lentement la solution de chlorure de diazonium froide et laisse 2 heures sous agitation à T.A. On essore le précipité formé et le lave à l'éther iso puis au pentane. On reprend le précipité dans 700 ml d'HCl concentré, laisse 1 nuit sous agitation à T.A., et chauffe à reflux pendant 1 heure. Après refroidissement du mélange réactionnel, on ajoute 1,5 litre d'eau, laisse une nuit sous agitation à T.A essore le précipité formé et le lave à l'éther iso puis au pentane. On obtient 41,7 g du composé attendu.

### Préparation 3.4

### Acide 6-(4-chlorophényl)-1-(2,4-dichlorophényl)-5-méthyl-4-oxo-1,4-dihydropyridazine-3-carboxylique.

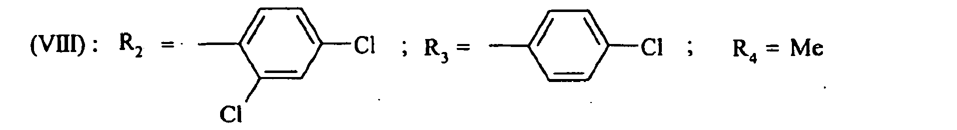

A une solution de 17,1 g de 2,4-dichloroaniline dans 20 ml d'acide acétique on ajoute 120 ml d'HCl concentré, refroidit à 0°C, ajoute lentement une solution de 7,3 g de nitrite de sodium dans 30 ml d'eau et laisse 1 heure sous agitation à 0°C pour obtenir une solution de chlorure de diazonium. En parallèle on laisse sous agitation une solution de 25 g du composé de la Préparation 2.2, 100 g de Na₂CO₃ et 30 ml de NaOH concentré dans 200 ml d'EtOH et 200 ml d'eau, ajoute lentement la solution de chlorure de diazonium froide et laisse 1 heure sous agitation à T.A. On essore le précipité formé et le lave à l'éther iso puis au pentane. On reprend le précipité dans 300 ml d'HCl concentré, laisse 30 minutes sous agitation à T.A et chauffe à reflux pendant 3 heures. Après refroidissement du mélange réactionnel à TA, on essore le précipité formé et le lave à l'eau. On obtient 16,5 g du composé attendu après séchage.

### Préparation 3.5

### Acide 1-(4-chlorophényl)-6-(2,4-dichlorophényl)-5-méthoxy-4-oxo-1,4-dihydropyridazine-3-carboxylique.

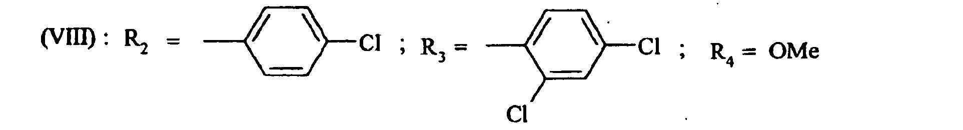

A une solution de 5,8 g de 4-chloroaniline dans 20 ml d'acide acétique on ajoute 60 ml d'HCl concentré, refroidit à 0°C, ajoute lentement une solution de 3,2 g de nitrite de sodium dans 10 ml d'eau et laisse 2 heures sous agitation à 0°C pour obtenir une solution de chlorure de diazonium. En parallèle on laisse sous agitation une solution de 13 g du composé de la Préparation 2.3 et 150 g de Na₂CO₃ dans 200 ml d'eau, ajoute lentement la solution de chlorure de diazonium froide et laisse 2 heures sous agitation à T.A. On essore le précipité formé et le lave à l'éther iso puis au pentane. On reprend le précipité dans 200 ml d'HCl concentré et laisse une nuit sous agitation à T.A., essore le précipité formé et le lave à l'éther iso puis au pentane. On obtient 16 g du composé attendu après séchage.

En suivant les modes opératoires décrits dans les Préparations 3, on prépare les composés de formule (VIII) rassemblés dans le TABLEAU I ci-après :

**TABLEAU I**

| | | | |
|---|---|---|---|
| 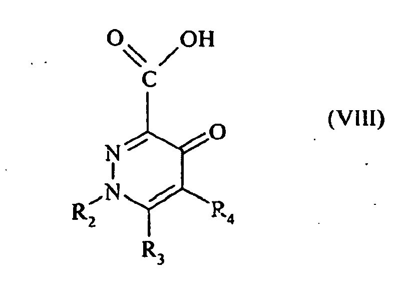 | | | |

| Préparations | R₂ | R₃ | R₄ |
|---|---|---|---|
| 3.6 | 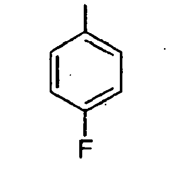 | 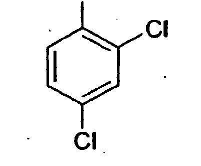 | H |
| 3.7 | 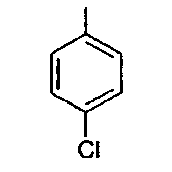 | 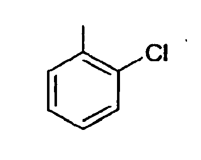 | H |
| 3.8 | 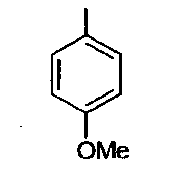 | 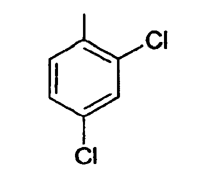 | H |
| 3.9 | 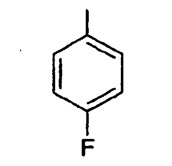 | 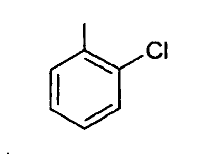 | H |
| 3.10 | 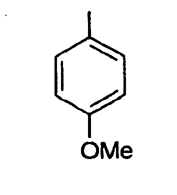 | 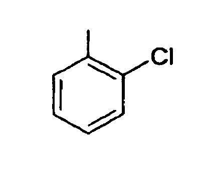 | H |
| 3.11 | 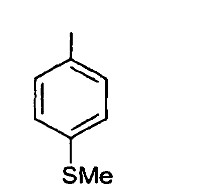 | 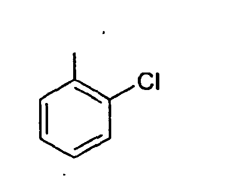 | H |
| 3.12 | 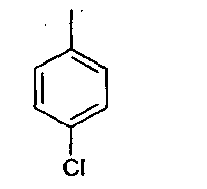 | 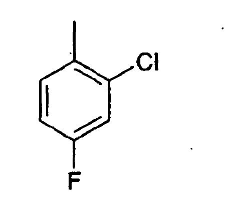 | H |
| 3.13 | 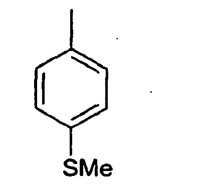 | 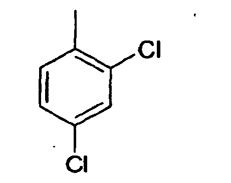 | OMe |
| 3.14 | 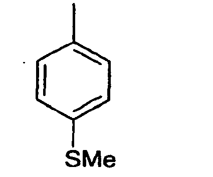 | 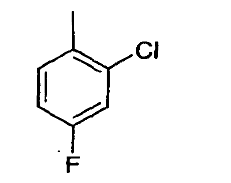 | H |
| 3.15 | 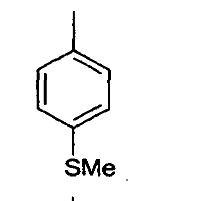 | 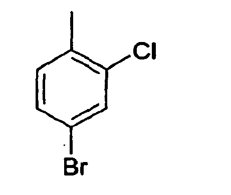 | H |
| 3.16 | 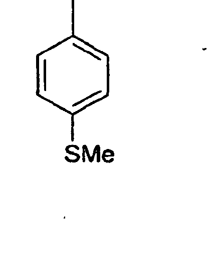 | 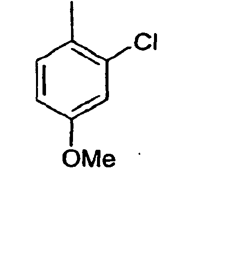 | H |
| 3.17 | 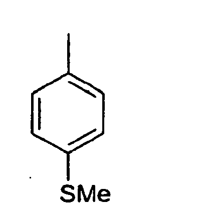 | 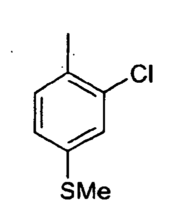 | H |
| 3.18 | 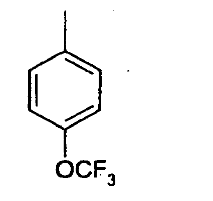 | 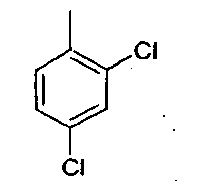 | H |
| 3.19 | 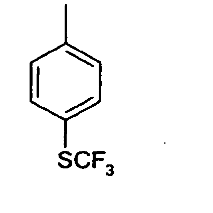 | 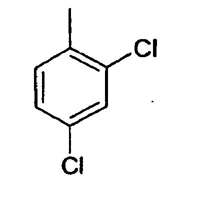 | H |

### 4. Préparations des composés de formule (VI) :

### Préparation 4.1

### 1-(4-Chlorophényl)-6-(2,4-dichlorophényl)-4-oxo-1,4-dihydropyndazine-3-carboxylate de méthyle.

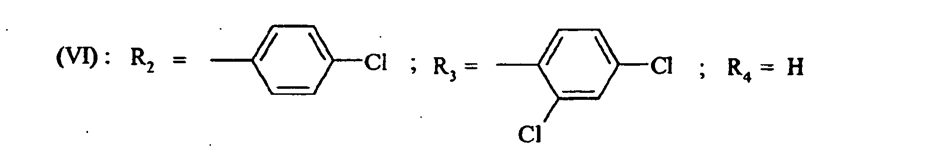

On refroidit au bain de glace un mélange de 27,8 g du composé de la Préparation 3.1 et 30 ml de triéthylamine dans 200 ml de DCM et 100 ml de THF, ajoute 6,14 ml de chlorure d'isocyanatosulfuryle, laisse 30 minutes sous agitation à 0°C puis laisse remonter à T.A. On ajoute ensuite 300 ml de MeOH, chauffe pendant 1 heure à reflux, ajoute 10 ml de triéthylamine et poursuit le reflux pendant 2 heures. On concentre le mélange réactionnel sous vide, reprend le résidu dans le mélange glace/eau, acidifie à pH = 3 par ajout d'HCl 2N, extrait au DCM, sèche la phase organique sur Na₂SO₄ et évapore le solvant sous vide. On obtient 26,5 g du composé attendu.

### Préparation 4.2

### 1-(4-Bromophényl)-6-(2,4-dichlorophényl)-4-oxo-1,4-dihydropyridazine-3-carboxylate de méthyle.

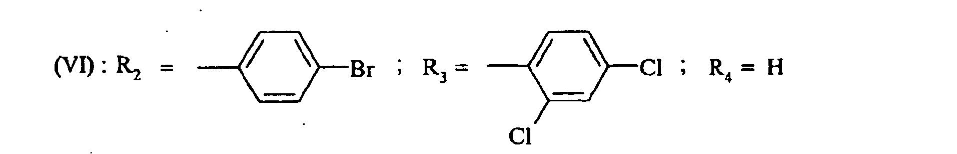

On refroidit au bain de glace un mélange de 18 g du composé de la Préparation 3.2 et dans 200 ml de DCM et 150 ml de THF, ajoute 3,57 ml de chlorure d'isocyanatosulfuryle puis 5,85 ml de triéthylamine, laisse 30 minutes sous agitation à 0°C puis laisse remonter à T.A. On ajoute ensuite 300 ml de MeOH et 10 ml de triéthylamine puis chauffe à 100°C pendant 3 heures. On concentre le mélange réactionnel sous vide, reprend le résidu à l'eau, acidifie à pH = 3 par ajout d'HCl 2N, extrait au DCM, sèche la phase organique sur Na₂SO₄ et évapore le solvant sous vide. On chromatographie le résidu sur gel de silice en éluant par le mélange DCM/propan-2-ol (100/2;v/v). On obtient 8 g du composé attendu après cristallisation dans le mélange EtOH/ether iso.

### Préparation 4.3

### 6-(2,4-Dichlorophényl)-1-[4-(méthylthio)phényl]-4-oxo-1,4-dihydropyridazine-3-carboxylate de méthyle.

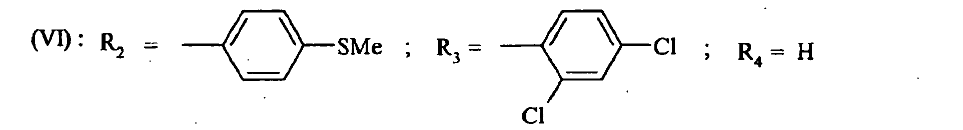

On refroidit à 0°C un mélange de 36,14 g du composé de la Préparation 3.3 dans 300 ml de DCM et 100 ml de THF, ajoute 37 ml de triéthylamine puis 7,88 ml de chlorure d'isocyanatosulfuryle, laisse 30 minutes sous agitation à 0°C puis laisse remonter à T.A. On ajoute ensuite 350 ml de MeOH, chauffe à reflux pendant 1 heure, ajoute 35 ml de triéthylamine et poursuit le reflux pendant 2 heures. On concentre le mélange réactionnel sous vide, reprend le résidu par un mélange glace/eau, acidifie à pH = 3 par ajout d'une solution tampon pH=2, extrait au DCM, sèche la phase organique sur Na₂SO₄ et évapore le solvant sous vide. On obtient 30,6 g du composé attendu.

### Préparation 4.4

### 6-(4-Chlorophényl)-1-(2,4-dichlorophényl)-5-méthyl-4-oxo-1,4-dihydropyridazine-3-carboxylate de méthyle.

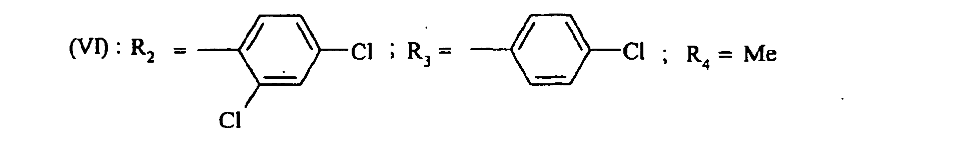

On refroidit à 0°C un mélange de 15 g du composé de la Préparation 3.4 dans 150 ml de DCM et 50 ml de THF, ajoute 3,20 ml de chlorure d'isocyanatosulfuryle puis 5,15 ml de triéthylamine et laisse 2 heures sous agitation à 0°C. On ajoute ensuite 400 ml de MeOH et 25 ml de triéthylamine et chauffe à reflux pendant 3 heures. On concentre le mélange réactionnel sous vide, reprend le résidu par un mélange éther/EtOH, essore le composé cristallisé formé, le lave au pentane et le sèche. On obtient 15 g du composé attendu.

### Préparation 4.5

### 1-(4-Chlorophényl)-6-(2,4-dichlorophényl)-5-méthoxy-4-oxo-1,4-dihydropyridazine-3-carboxylate de méthyle.

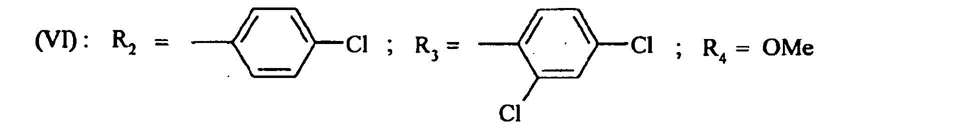

On refroidit à 0°C un mélange de 8 g du composé de la Préparation 3.5 dans 150 ml de DCM et 150 ml de THF, ajoute 8 ml de triéthylamine puis 1,64 ml de chlorure d'isocyanatosulfuryle, laisse 30 minutes sous agitation à 0°C puis laisse remonter à T.A. On ajoute ensuite 200 ml de MeOH et chauffe à reflux pendant 2 heures. On concentre le mélange réactionnel sous vide, reprend le résidu à l'eau, essore le précipité formé et le sèche. On obtient 7,4 g du composé attendu.

En suivant les modes opératoires décrits dans les Préparations 4, on prépare les composés de formule (VI) rassemblés dans le TABLEAU II ci-après :

**TABLEAU II**

| Préparations | R₂ | R₃ | R₄ |
|---|---|---|---|
| 4.6 | 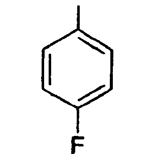 | 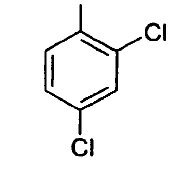 | H |
| 4.7 | 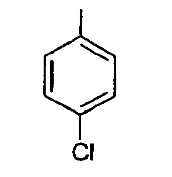 | 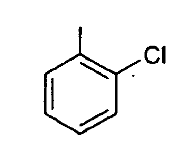 | H |
| 4.8 | 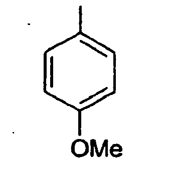 | 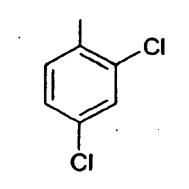 | H |
| 4.9 | 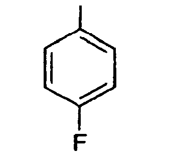 | 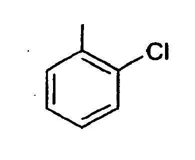 | H |
| 4.10 | 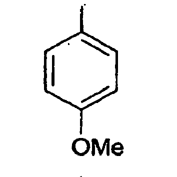 | 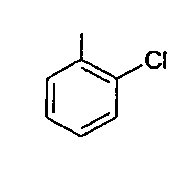 | H |
| 4.11 | 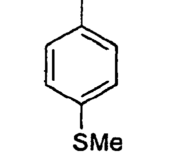 | 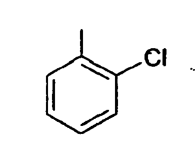 | H |
| 4.12 | 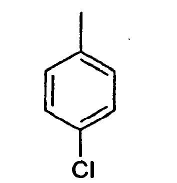 | 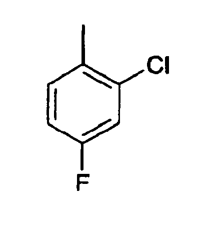 | H |
| 4.13 | 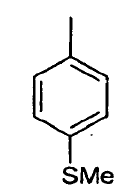 | 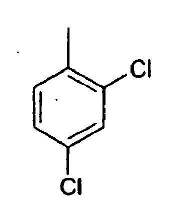 | OMe |
| 4.14 | 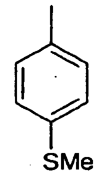 | 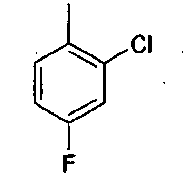 | H |
| 4.15 | 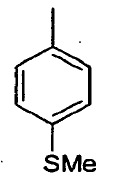 | 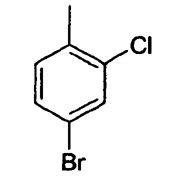 | H |
| 4.16 | 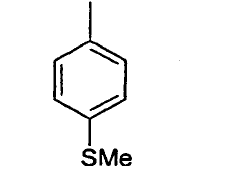 | 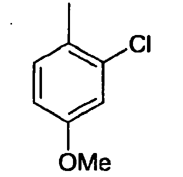 | H |
| 4.17 | 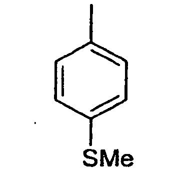 | 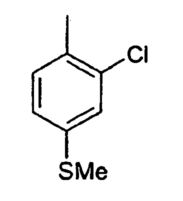 | H |
| 4.18 | 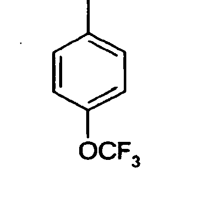 | 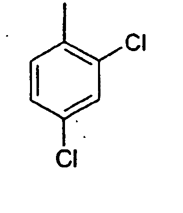 | H |
| 4.19 | 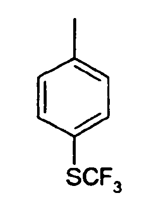 | 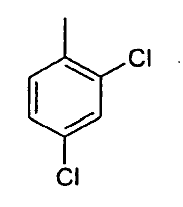 | H |

### 5. Préparations des composés de formule(IV) :

### Préparation 5.1

### Chlorhydrate de 1-(4-chlorophényl)-6-(2,4-dichlorophényl)-4-oxo-1,4-dihydropyridazine-3-carbohydrazide.

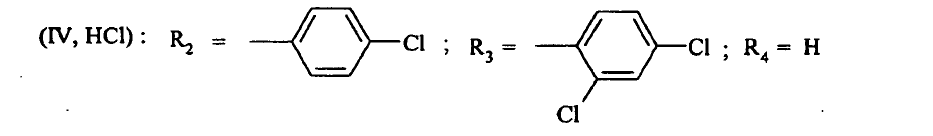

On laisse 3 heures sous agitation à T.A un mélange de 26,5 g du composé de la Préparation 4.1 et 5,5 ml d'hydrazine monohydrate dans 400 ml de dioxane. On concentre le mélange réactionnel sous vide, reprend le résidu avec 100 ml d'EtOH, ajoute 100 ml d'éther chlorhydrique 2N puis 200 ml d'éther, laisse sous agitation et essore le précipité formé. On obtient 23 g du composé attendu.

### Préparation 5.2

### 1-(4-Bromophényl)-6-(2,4-dichlorophényl)-4-oxo-1,4-dihydropyridazine-3-carbohydrazide.

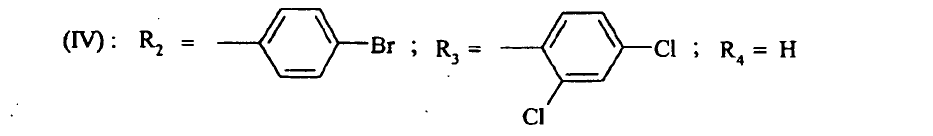

On laisse 3 heures sous agitation à T.A un mélange de 8 g du composé de la Préparation 4.2 et 3 ml d'hydrazine monohydrate dans 150ml de dioxane. On concentre le mélange réactionnel sous vide, reprend le résidu dans l'éther, laisse une nuit sous agitation à T.A. et essore le précipité formé. On obtient 5,5 g du composé attendu.

### Préparation 5.3

### Chlorhydrate de 6-(2,4-dichlorophényl)-1-[4-(méthylthio)phényl]-4-oxo-1,4-dihydropyridazine-3-carbohydrazide.

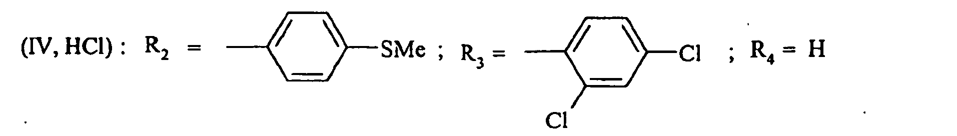

On laisse 2 heures sous agitation à T.A. un mélange de 30,6 g du composé de la Préparation 4.3 et 7 ml d'hydrazine monohydrate dans 400 ml de dioxane. On concentre le mélange réactionnel sous vide, reprend le résidu avec 100 ml d'EtOH, ajoute 100 ml d'éther chorhydrique 2N puis 200 ml d'éther, laisse sous agitation et essore le précipité formé. On obtient 32 g du composé attendu.

### Préparation 5.4

### 6-(4-Chlorophényl)-1-(2,4-dichlorophényl)-5-méthyl-4-oxo-1,4-dihydropyridazine-3-carbohydrazide.

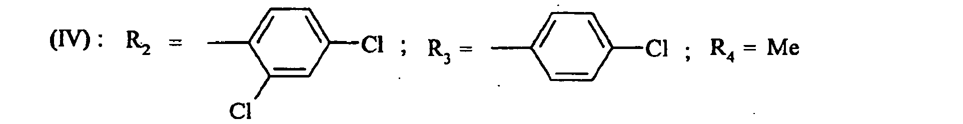

On laisse 24 heures sous agitation à T.A un mélange de 15 g du composé de la Préparation 4.4 et 7 ml d'hydrazine monohydrate dans 400 ml de dioxane et 300 ml de DCM. On concentre le mélange réactionnel sous vide, reprend le résidu dans un mélange éther/EtOH, essore le précipité formé et le sèche. On obtient 11,5 g du composé attendu.

### Préparation 5.5

### Chlorhydrate de 1-(4-chlorophényl)-6-(2,4-dichlorophényl)-5-méthoxy-4-oxo-1,4-dihydropyridazine-3-carbohydrazide.

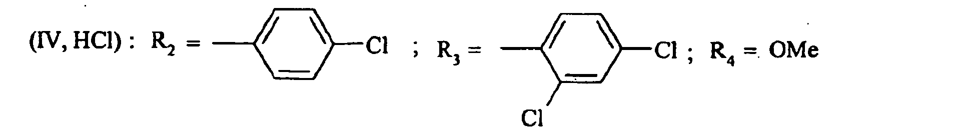

On laisse 16 heures sous agitation à T.A un mélange de 7,4 g du composé de la Préparation 4.5 et 1 g d'hydrazine monohydrate dans 200 ml de dioxane et 50 ml de MeOH. On concentre le mélange réactionnel sous vide, reprend le résidu dans 50 ml d'EtOH, ajoute de l'éther chlorhydrique 2N puis de l'éther iso, essore le précipité formé, le lave à l'éther iso puis au pentane et le sèche. On obtient 8,2 g du composé attendu.

En suivant les modes opératoires décrits dans les Préparations 5, on prépare les composés de formule (IV) rassemblés dans le TABLEAU III ci-après :

**TABLEAU III**

| | | | |
|---|---|---|---|
| 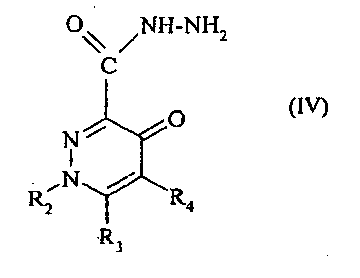 | | | |

| Préparations | R₂ | R₃ | R₄ |
|---|---|---|---|
| 5.6 (HCl) | 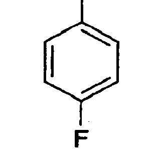 | 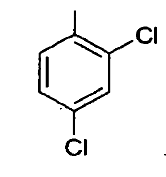 | H |
| 5.7 (HCl) | 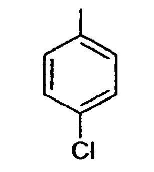 | 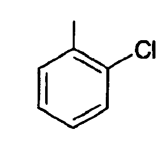 | H |
| 5.8 (HCl) | 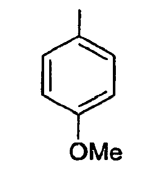 | 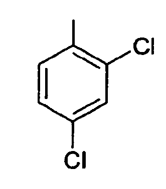 | H |
| 5.9 (HCl) | 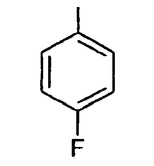 | 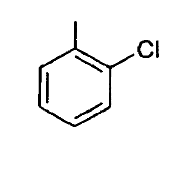 | H |
| 5.10 (HCl) | 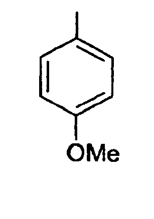 | 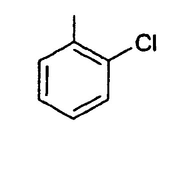 | H |
| 5.11 (HCl) | 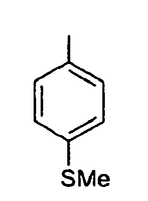 | 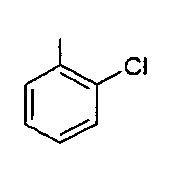 | H |
| 5.12 (HCl) | 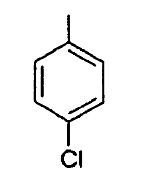 | 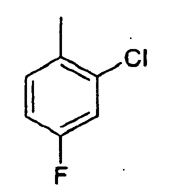 | H |
| 5.13 (HCl) | 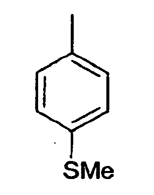 | 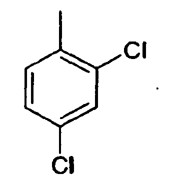 | OMe |
| 5.14 (HCl) | 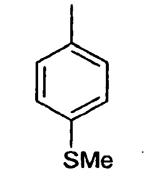 | 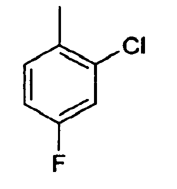 | H |
| 5.15 (HCl) | 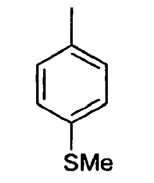 | 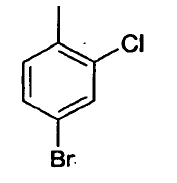 | H |
| 5.16 (HCl) | 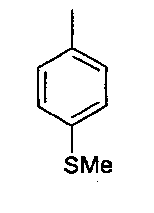 | 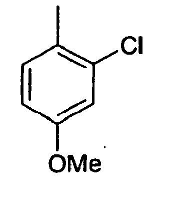 | H |
| 5.17 (HCl) | 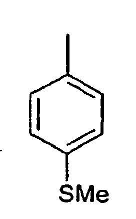 | 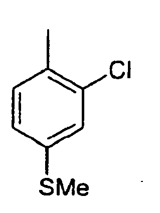 | H |
| 5.18 (HCl) | 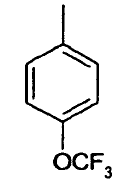 | 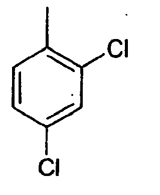 | H |
| 5.19 (HCl) | 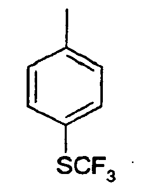 | 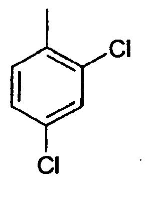 | H |

### 6. Préparations des composés de formule (II)

### Préparation 6.1

### 5-(4-Chlorophényl)-6-(2,4-dichlorophényl)-2,5-dihydro-3H-pyrazolo[4,3-c]pyridazin-3-one.

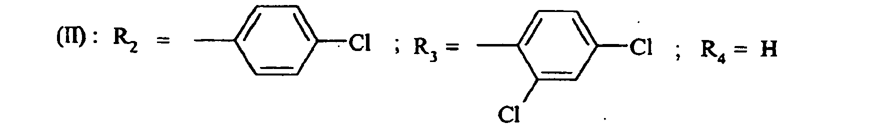

On chauffe à 135°C pendant 36 heures un mélange de 5 g du composé de la Préparation 5.1 et 500 ml de pyridine. On concentre le mélange réactionnel sous vide, reprend le résidu à l'eau, laisse une nuit sous agitation à T.A, essore le précipité formé et le sèche. On obtient 3,5 g du composé attendu.

### Préparation 6.2

### 5-(4-Bromophényl)-6-(2,4-dichlorophényl)-2,5-dihydro-3H-pyrazolo[4,3-c]pyridazin-3-one.

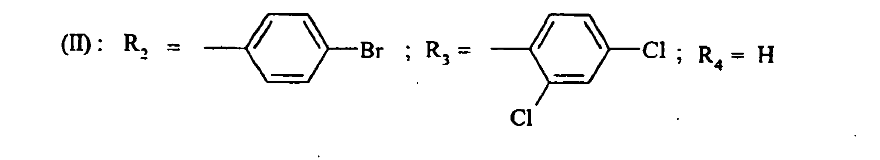

On chauffe à 120°C pendant une nuit un mélange de 5,5 g du composé de la Préparation 5.2, 500 ml de pyridine et 10 ml d'HCl concentré. On concentre le mélange réactionnel sous vide, reprend le résidu à l'eau, essore le précipité formé et le sèche. On obtient 5 g du composé attendu.

### Préparation 6.3

### 6-(2,4-Dichlorophényl)-5-[4-(méthylthio)phényl]-2,5-dihydm-3H-pyrazolo[4,3-c]pyridazin-3-one.

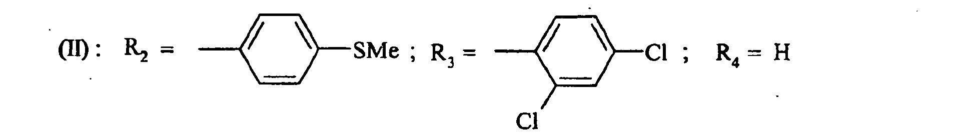

On chauffe à reflux pendant 72 heures un mélange de 5 g du composé de la Préparation 5.3 et 486 ml de pyridine. On concentre le mélange réactionnel sous vide, reprend le résidu à l'eau, laisse 1 heure sous agitation à T.A et essore le précipité formé (4,31 g). On chromatographie 0,2 g de précipité sur gel de silice H en éluant au DCM puis par le mélange DCM/MeOH (100/2,5 ; v/v). On obtient 0,07g du composé attendu.

### Préparation 6.4

### 6-(4-Chloronhényl)-5-(2,4-dichloronhényl)-7-méthyl-2,5-dihvdro-3H-pyrazolo[4,3-c]pyridazin-3-one.

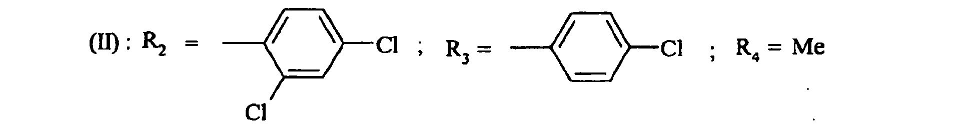

On chauffe à reflux pendant 3 jours un mélange de 5,2 g du composé de la Préparation 4.4, 50 ml de pyridine et 2 ml d'HCl concentré. Après refroidissement à T.A, on ajoute de l'eau au mélange réactionnel et essore le précipité formé. On dissout le précipité dans l'AcOEt et filtre un insoluble. On extrait les jus d'essorage aqueux à l'AcOEt, lave la phase organique par une solution d'HCl à 10%, sèche les phases organiques jointes sur Na₂SO₄ et évapore sous vide le solvant. On obtient 2,3 g du composé attendu.

### Préparation 6.5

### 5-(4-Chlorophényl)-6-(2,4-dichlorophényl)-7-méthoxy-2,5-dihydro-3H-pyrazolo[4,3-c]pyridazin-3-one.

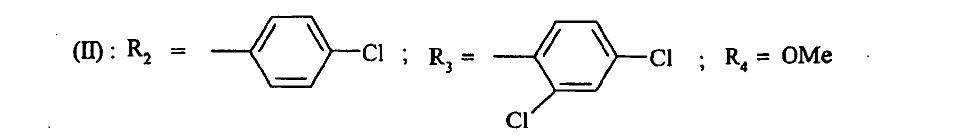

On chauffe à reflux pendant 4 heures un mélange de 3,6 g du composé de la Préparation 5.5 et 1,7 g de *tert*-butylate de potassium dans 400 ml d'EtOH. On concentre le mélange réactionnel sous vide, reprend le résidu par une solution d'HCl à 10%, essore le précipité formé et le lave à l'éther iso puis au pentane. On obtient 2,5 g du composé attendu.

En suivant les modes opératoires décrits dans les Préparations 6, on prépare les composés de formule (II) rassemblés dans le TABLEAU IV ci-après :

**TABLEAU IV**

| | | | |
|---|---|---|---|
| 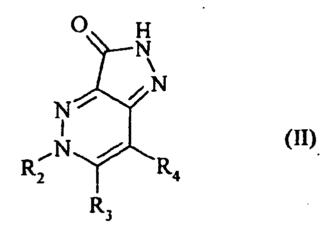 | | | |

| Préparations | R₂ | R₃ | R₄ |
|---|---|---|---|
| 6.6 | 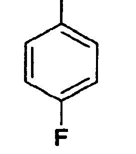 | 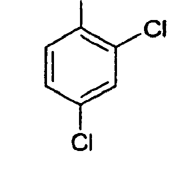 | H |
| 6.7 | 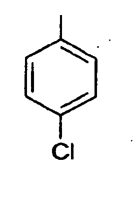 | 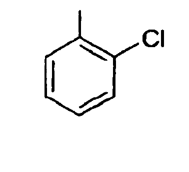 | H |
| 6.8 | 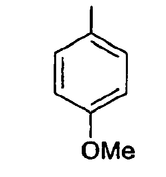 | 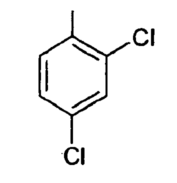 | H |
| 6.9 | 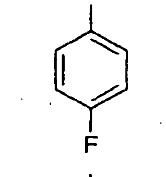 | 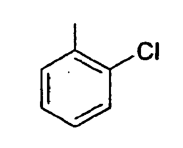 | H |
| 6.10 | 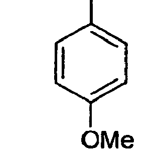 | 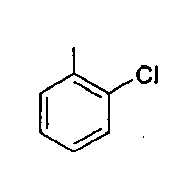 | H |
| 6.11 | 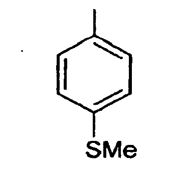 | 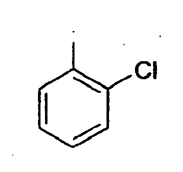 | H |
| 6.12 | 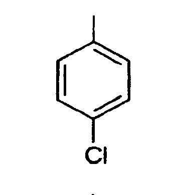 | 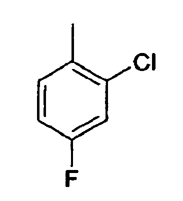 | H |
| 6.13 | 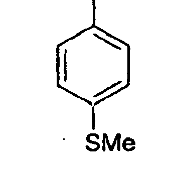 | 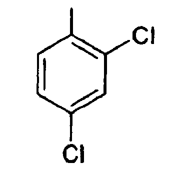 | OMe |
| 6.14 | 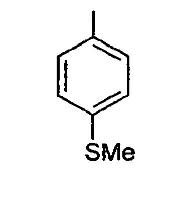 | 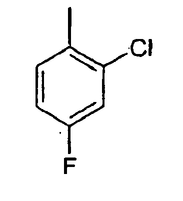 | H |
| 6.15 | 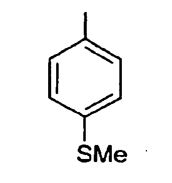 | 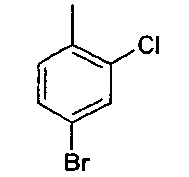 | H |
| 6.16 | 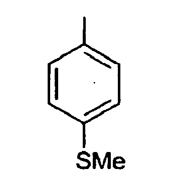 | 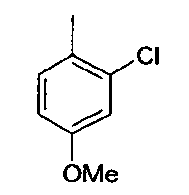 | H |
| 6.17 | 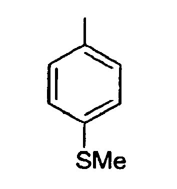 | 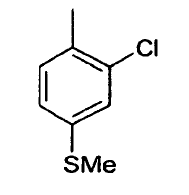 | H |
| 6.18 | 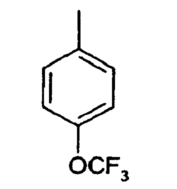 | 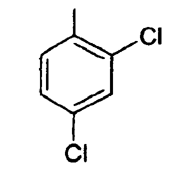 | H |
| 6.19 | 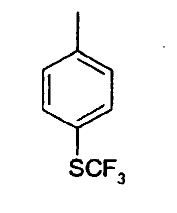 | 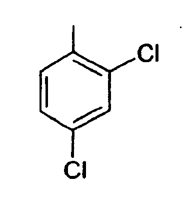 | H |

### 7. Préparations des composés de formule (III)

### Préparation 7.1

### [4-(Trifluorométhyl)cyclohexyl]méthyl méthanesulfonate, mélange de cis et trans.

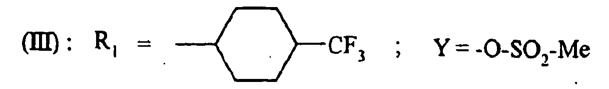

### A) [4-(Trifluorométhyl)cyclohexyl]méthanol, mélange de cis et trans.

A un mélange de 5,15 g d'acide 4-(trifluorométhyl)cyclohexanecarboxylique mélange de cis et trans dans 150 ml de THF, on ajoute goutte à goutte et à T.A, 65 ml d'une solution 1M de borane dans le THF, laisse 1 heure sous agitation à T.A puis chauffe à 100°C pendant 4 heures. On ajoute ensuite 150 ml de MeOH et chauffe à reflux pendant 1 heure. On ajoute enfin, goutte à goutte 100 ml d'éther chlorhydrique 2N et chauffe à reflux pendant 45 minutes. Après refroidissement à T.A, on concentre le mélange réactionnel sous vide, reprend le résidu par une solution de NaOH à 10%, laisse 30 minutes sous agitation, extrait à l'AcOEt, sèche la phase organique sur Na₂SO₄ et évapore le solvant sous vide. On obtient 4,35 g du composé attendu.

### B) [4-(Trifluorométhyl)cyclohexyl]méthyl méthanesulfonate, mélange de cis et trans.

On refroidit à 0°C un mélange de 4,35 g du composé de l'étape A et 5,1 ml de triéthylamine dans 50 ml de DCM, ajoute, goutte à goutte, 1,85 ml de chlorure de méthanesulfonyle et laisse sous agitation en laissant remonter la température à T.A. Après 4 heures d'agitation à T.A, on ajoute de la glace au mélange réactionnel, extrait au DCM, sèche la phase organique sur Na₂SO₄ et évapore le solvant sous vide. On obtient 4 g du composé attendu sous forme d'huile.

### Préparation 7.2

### Adamant-1-ylméthylméthanesulfonate

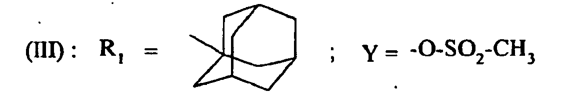

On refroidit à 0°C un mélange de 3 g d'adamantan-1-yl méthanol et 5 ml de triéthylamine dans 50 ml de DCM, ajoute, goutte à goutte, 2 ml de chlorure de méthanesulfonyle et laisse sous agitation en laissant remonter la température à T.A. On ajoute un mélange de eau/glace au mélange réactionnel, extrait au DCM, sèche la phase organique sur Na₂SO₄ et évapore le solvant sous vide. On obtient 2 g du composé attendu.

### Préparation 7.3

### 4-(Chlorométhyl)-2-fluoro-1-propoxybenzène

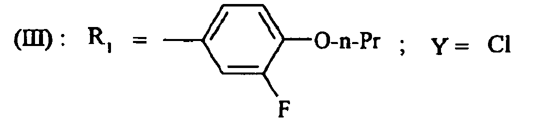

### A) Acide 3-fluoro-4-propoxybenzoïque

On chauffe à reflux pendant 24 heures un mélange de 2 g d'acide 3-fluoro-4-hydroxybenzoïque et 5,31 g de K₂CO₃ dans 50 ml d'acétonitrile, ajoute 0,5 ml de 1-iodopropane et poursuit le reflux pendant 7 heures. Après refroidissement à T.A, on concentre le mélange réactionel sous vide, reprend le résidu à l'eau, extrait à l'AcOEt et évapore le solvant sous vide. On reprend le résidu dans 30 ml d'EtOH, ajoute 5 ml d'une solution concentrée de NaOH et laisse 1 heure sous agitation à 60°C. On concentre sous vide, reprend le résidu par une solution d'HCl 10%, extrait au DCM, sèche la phase organique sur Na₂SO₄ et évapore le solvant sous vide. On obtient 0,8 g du composé attendu.

### B) (3-Fluoro-4-propoxyphényl)méthanol

A un mélange de 0,8 g du composé de l'étape précédente dans 300 ml de THF, on ajoute goutte à goutte 8 ml d'une solution 1M de borane dans le THF, laisse 30 minutes sous agitation à T.A puis chauffe à 100°C pendant 2 heures. On ajoute ensuite 100 ml de MeOH et chauffe à 100°C pendant 1 heure. On ajoute enfin 20 ml d'éther chlorhydrique 2 N et chauffe à 100°C pendant 1 heure. On concentre le mélange réactionnel sous vide, reprend le résidu à l'eau, extrait au DCM, sèche la phase organique sur Na₂SO₄ et évapore le solvant sous vide. On obtient 0,8 g du composé attendu.

### C) 4-(Chlorométhyl)-2-fluoro-1-propoxybenzène

On laisse une nuit sous agitation à T.A. un mélange de 0,8 g du composé de l'étape précédente, 0,4 ml de chlorure de méthanesulfonyle et 0,2 ml de triéthylamine dans 10 ml de DCM. On ajouté de la glace au mélange réactionnel, puis du tampon pH = 2, extrait au DCM, sèche la phase organique sur Na₂SO₄ et évapore le solvant sous vide. On obtient 1,2 g du composé attendu.

### Préparation 7.4

### Chlorhydrate de N-[4-(chlorométhyl)benzyl]-N-éthylpropan-1-amine

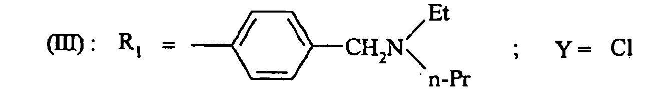

### A) [4-[[éthyl(propyl)amino]méthyl]phényl]méthanol

On laisse 48 heures sous agitation à T.A. un mélange de 5 g de [4-(chlorométhyl)phényl]méthanol, 5,56 g de N-éthylpropan-1-amine, 5,29 g d'iodure de potassium et 4,41 g de K₂CO₃ dans 200 ml d'acétonitrile. On concentre le mélange réactionnel sous vide, reprend le résidu à l'eau, extrait au DCM, sèche la phase organique sur Na₂SO₄ et évapore le solvant sous vide. On obtient 3,5 g du composé attendu.

### B) Chlorhydrate de N-[4-(chlorométhyl)benzyl]-N-éthylpropan-1-amine

A un mélange de 0,5 g du composé de l'étape précédente dans 5 ml d'éther chlorhydrique 2N on ajoute goutte à goutte, 2,87 g de chlorure de thionyle et laisse 2 heures sous agitation à T.A. On concentre le mélange réactionnel sous vide, reprend le résidu au DCM et évapore le solvant sous vide. On obtient 0,6 g du composé attendu.

### Préparation 7.5

### Chlorhydrate de N-[4-(chlorométhyl)benzyl]-2,2,2-trifluoro-N-méthyléthanamine

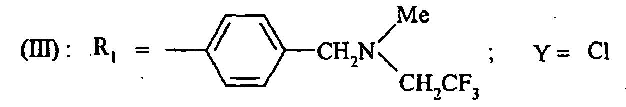

### A) 4-[(méthylamino)méthyl]benzoate de méthyle

A une solution de 5 g de 4-[[(*tert*-butoxycarbonyl)amino]méthyl]benzoate de méthyle dans 150 ml de THF on ajoute 1,13 g de NaH à 60% dans l'huile, puis 2,35 ml d'iodure de méthyle et laisse une nuit sous agitation à T.A. On concentre le mélange réactionnel sous vide, reprend le résidu par un mélange eau/glace, extrait à l'AcOEt, sèche la phase organique sur Na₂SO₄ et évapore le solvant sous vide. On reprend le résidu par 80 ml d'éther chlorhydrique 2N, ajoute 1 ml d'eau et laisse une nuit sous agitation à T.A. On concentre le mélange réactionnel sous vide, reprend le résidu par une solution de NaHCO₃ à 5%, extrait au DCM, sèche la phase organique sur Na₂SO₄ et évapore le solvant sous vide. On obtient 2,2 g du composé attendu.

### B) 4-[[méthyl(2,2,2-trifluoroéthyl)amino]méthylbenzoate de méthyle.

On chauffe à 80°C pendant 5 heures un mélange de 2,2 g du composé de l'étape précédente, 3,13 g de 2,2,2-trifluoroéthyltrifluorométhanesulfonate et 2,06 g de NaHCO₃ dans 20 ml d'EtOH. On concentre le mélange réactionnel sous vide, reprend le résidu à l'eau, extrait au DCM, sèche la phase organique sur Na₂SO₄ et évapore le solvant sous vide. On obtient 2 g du composé attendu.

### C) [4-[[méthyl(2,2,2-trifluoroéthyl)amino]méthyl]phényl]méthanol.

On refroidit à 0°C une suspension de 0,581 g de LiAlH₄ dans 30 ml d'éther, ajoute goutte à goutte une solution de 2 g du composé de l'étape précédente dans 10 ml d'éther et laisse sous agitation en laissant remonter la température à T.A. On ajoute ensuite 1,4 ml d'eau, puis 1,4 ml de NaOH à 15% et 4,4 ml d'eau, laisse sous agitation et filtre les sels minéraux. On concentre le filtrat sous vide, reprend le résidu à l'eau, extrait à l'éther, sèche la phase organique sur Na₂SO₄ et évapore le solvant sous vide. On obtient 2 g du composé attendu.

### D) [Chlorhydrate de N-[4-(chlorométhyl)benzyl]-2,2,2-trifluoro-N-méthyléthanamine

A un mélange de 0,5 g du composé de l'étape précédente dans 10 ml d'éther chorhydrique 2N, on ajoute 2 ml de chlorure de thionyle et laisse une nuit sous agitation à T.A. On concentre sous vide et obtient 0,5 g du composé attendu.

En suivant les modes opératoires décrits dans les Préparations 7, on prépare les composés de formule (III) rassemblés dans le TABLEAU V ci-après :

**TABLEAU V**

| Y-CH₂-R₁ (III) | | |
|---|---|---|
| Préparations | Y | R₁ |
| 7.6 | -O-SO₂-CH₃ | 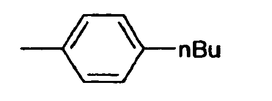 |
| 7.7 | -Cl | 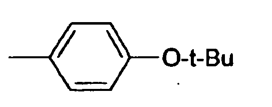 |
| 7.8 | -Cl | 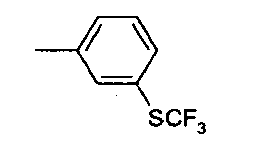 |
| 7.9 | -Cl | -CH(nPr)₂ |
| 7.10 | -O-SO₂-CH₃ | 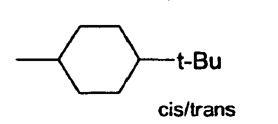 |
| 7.11 | -Cl | 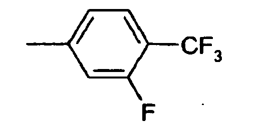 |
| 7.12 | -Cl | 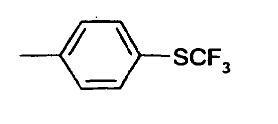 |

### EXEMPLE 1 : Composé N° 1

### 5-(4-Chlorophényl)-2-(cyclohexylméthyl)-6-(2,4-dichlorophényl)-2,5-dihydro-3H-pyrazolo[4,3-c]pyridazin-3-one.

On chauffe à 130°C pendant 48 heures un mélange de 0,5 g du composé de la Préparation 6.1, 0,27 ml de (bromométhyl)cyclohexane et 0,27 g de K₂CO₃ dans 20ml de dioxane et 5 ml de DMF. On concentre le mélange réactionnel sous vide, reprend le résidu à l'eau, extrait au DCM, sèche la phase organique sur Na₂SO₄ et évapore le solvant sous vide. On obtient 0,22 g après cristallisation dans l'éther iso.
MH⁺ : 487 ; tr = 11,24 (M1) ;
RMN ¹H : DMSO-d₆ (250 MHz): δ (ppm) : 0,75-1,95 : m : 11H ; 3,70 : mt : 2H ; 7,29-7,74 : m : 8H.

### EXEMPLE 2 : Composé N° 2 et composé N° 3

### 5-(4-Chlorophényl)-6-(2,4-dichlorophényl)-2-[[4-(trifluorométhyl)cyclohexyl] méthyl]-2,5-dihydro-3H-pyrazolo[4,3-c]pyridazin-3-one, isomère le moins polaire et isomère le plus polaire.

On chauffe à 130°C pendant 48 heures un mélange de 0,5 g du composé de la Préparation 6.1, 0,664 g du composé de la Préparation 7.1 et 0,352 g de K₂CO₃ dans 20ml de dioxane et 5 ml de DMF. On concentre le mélange réactionnel sous vide, reprend le résidu à l'eau, extrait au DCM, sèche la phase organique sur Na₂SO₄ et évapore le solvant sous vide. On chromatographie le résidu sur gel de silice en éluant par le mélange DCM/MeOH (100/2;v/v). On sépare les deux isomères :
- composé N°2 : le moins polaire : m = 0,12 g après cristallisation dans l'éther iso ;
   MH⁺ : 555 ; tr = 11,45 (M1) ;
   RMN ¹H : DMSO-d₆ (500 MHz): δ (ppm) : 1,42-1,72 : m : 8H ; 2,24 : mt : 1H ; 2,31 : mt : 1H ; 3,94 : mt : 2H ; 7,37-7,76 : m : 8H.
- composé N°3 : le plus polaire : m = 0,025 g après cristallisation dans l'éther iso.
MH⁺ : 555 ; tr= 11,42 (M1) ;
RMN ¹H : DMSO-d₆ (500 MHz): δ (ppm) : 0,99-1,34 : m : 4H ; 1,73 : mt : 2H ; 1,78-1,94 : m : 3H ; 2,24 : mt : 1H ; 3,78 : mt : 2H ; 7,40-7,58 : m : 6H.

### EXEMPLE 3 : Composé N° 4

### 2-(Adamantan-1-ylméthyl)-5-(4-chlorophényl)-6-(2,4-dichlorophényl)-2,5-dihydro-3H-pyrazolo[4,3-c]pyridazin-3-one.

On chauffe à 140°C pendant une nuit un mélange de 0,5 g du composé de la Préparation 6.1, 0,62 g du composé de la Préparation 7.2 et 0,624 g de Cs₂CO₃ dans 20ml de dioxane et 5 ml de DMF. On concentre le mélange réactionnel sous vide, reprend le résidu à l'eau, extrait au DCM, sèche la phase organique sur Na₂SO₄ et évapore le solvant sous vide. On chromatographie le résidu sur gel de silice en éluant par le mélange DCM/propan-2-ol (97/3;v/v). On obtient 0,025 g du composé attendu après cristallisation dans l'éther iso.

### EXEMPLE 4 : Composé N° 7

### 5-(4-Chlorophényl)-6-(2,4-dichlorophényl)-2-(4-isopropylbenzyl)-2,5-dihydro-3H-pyrazolo[4,3-c]pyridazin-3-one.

On refroidit à 0°C un mélange de 0,5 g du composé de la Préparation 6.1 dans 30 ml de THF et 5 ml de DMF, ajoute 0,052 g de NaH à 60% dans l'huile puis 0,26 ml de bromure de 4-isopropylbenzyle et laisse sous agitation en laissant remonter la température à T.A. On chauffe ensuite à 60°C pendant 6 heures puis agite une nuit à T.A. On concentre le mélange réactionnel sous vide, reprend le résidu à l'eau, extrait au DCM, sèche la phase organique sur Na₂SO₄ et évapore le solvant sous vide. On chromatographie le résidu sur gel de silice en éluant par le mélange DCM/propan-2-ol (97/3;v/v). On obtient 0,06 g du composé attendu.

### EXEMPLE 5 : Composé N° 36 et composé N° 37

### 5-(4-Bromophényl)-2-[(4-tert-butylcyclohexyl)méthyl]-6-(2,4-dichlorophényl)-2,5-dihydro-3H-pyrazolo[4,3-c]pyridazin-3-one, isomère le moins polaire et isomère le plus polaire.

On chauffe à 140°C pendant 5 heures un mélange de 0,5 g du composé de la Préparation 6.2, 0,57 g du composé de la Préparation 7.10 et 0,32 g de K₂CO₃ dans 30 ml de dioxane et 5 ml de DMF. On concentre le mélange réactionnel sous vide, reprend le résidu à l'eau, extrait à l'AcOEt, sèche la phase organique sur Na₂SO₄ et évapore le solvant sous vide. On chromatographie le résidu sur gel de silice en éluant par le mélange DCM/MeOH (100/2;v/v). On sépare les deux isomères :
- composé N° 36: le moins polaire : m = 0,035g après cristallisation dans l'éther iso ;
   MH⁺ : 587 ; tr = 13,05 (M1);
   RMN ¹H : DMSO-d₆ (250 MHz): δ (ppm) : 0,77-1,67 : m : 18H ; 2,26 : se : 1H ; 3,94 : mt : 2H ; 7,32-7,77 : m : 8H.
- composé N° 37 : le plus polaire : m = 0,065 g après cristallisation dans l'éther iso.
   MH⁺ : 587 ; tr = 13,03 (M1) ;
RMN ¹H : DMSO-d₆ (250 MHz): δ (ppm) : 0,81 : s : 9H ; 0,86-1,12 : m : 4H ; 1,58-1,90: m : 4H ; 3,73 : mt : 2H ; 7,29-7,79 : m : 8H.

### EXEMPLE 6 : Composé N° 51

### 5-(4-Chlorophényl)-6-(2,4-dichlorophényl)-7-hydroxy-2-[4-(trifluorométhyl)benzyl]-2,5-dihydro-3H-pyrazolo[4,3-c]pyridazin-3-one.

On chauffe à 80°C pendant 5 heures un mélange de 0,6 g du composé N° 50 et 30 ml d'une solution de HBr à 45% dans l'acide acétique. On concentre le mélange réactionnel sous vide, reprend le résidu par une solution saturée de NaHCO₃, laisse 1 heure sous agitation à T.A. et essore le précipité formé. On reprend le précipité au MeOH, acidifie par ajout d'HCl concentré, essore le précipité formé et le sèche. On obtient 0,09 g du composé attendu.

### EXEMPLE 7 : Composé N° 57

### 6-(2,4-Dichlorophényl)-5-(4-fluorophényl)-2-[4-(trifluorométhoxy)benzyl]-2,5-dihydro-3H-pyrazolo[4,3-c]pyridazin-3-one.

On chauffe à 160°C pendant 2 heures un mélange de 0,53 g du composé de la Préparation 6.6, 0,56 g de 1-(bromométhyl)4-(trifluorométhoxy)benzène et 1,88 g de Cs₂CO₃ dans 30 ml de dioxane et 5 ml de DMF. On concentre le mélange réactionnel sous vide, reprend le résidu à l'eau, extrait au DCM, sèche la phase organique sur Na₂SO₄ et évapore le solvant sous vide. On chromatographie le résidu sur gel de silice H en éluant au DCM puis par le mélange DCM/MeOH (100/5;v/v). On obtient 0,04 g du composé attendu après cristallisation dans l'éther iso.

### EXEMPLE 8 : Composé N° 65

### 6-(2,4-Dichlorophényl)-5-(4-méthoxyphényl)-2-[4-[(trifluorométhyl)thio]benzyl]-2,5-dihydro-3H-pyrazolo[4,3-c]pyridazin-3-one.

On chauffe à reflux pendant 2 heures un mélange de 2 g du composé de la Préparation 6.8, 1,73 g de 1-(bromométhyl)-4-[(trifluorométhyl)thio]benzène et 6,87 g de Cs₂CO₃ dans 103 ml de dioxane. On concentre le mélange réactionnel sous vide, reprend le résidu à l'eau, extrait au DCM, sèche la phase organique sur Na₂SO₄ et évapore le solvant sous vide. On chromatographie le résidu sur gel de silice H en éluant au DCM puis par le mélange DCM/MeOH (100/S;v/v). On obtient 1,164 g du composé attendu.

### EXEMPLE 9 : Composé N° 76

### 6-(2,4-Dichlorophényl)-5-(4-hydroxyphényl)-2-[4-[(trifluorométhyl)thio]benzyl]-2,5-dihydro-3H-pyrazolo[4,3-c]pyridazin-3-one.

On refroidit à -20°C une solution de 0,91 g du composé N° 65 dans 50 ml de DCM, ajoute sous atmosphère d'azote, 6,3 ml d'une solution 1M de BBr₃ dans le DCM et laisse 48 heures sous agitation en laissant remonter la température à T.A. On verse le mélange réactionnel dans de l'eau glacée, extrait au DCM, sèche la phase organique sur Na₂SO₄ et évapore le solvant sous vide. On chromatographie le résidu sur gel de silice H en éluant au DCM puis par le mélange DCM/MeOH (100/1;v/v). On obtient 0,49g du composé attendu.

### EXEMPLE 10 : Composé N° 77

### 4-[6-(2,4-dichlorophényl)-3-oxo-2-[4[(trifluorométhyl)thio]benzyl]-2,3-dihydro-5H-pyrazolo[4,3-c]pyridazin-5-yl]phényl-3,3,3-trifluoropropane-1-sulfonate.

On refroidit à 0°C une solution de 0,245 g du composé N° 76 et 0,12 ml de triéthylamine dans 100 ml de DCM, ajoute 0,17 g de chlorure de 3,3,3-trifluoropropane-1-sulfonyle et laisse sous agitation en laissant remonter la température à T.A. On ajoute de l'eau au mélange réactionnel, extrait au DCM, lave la phase organique à l'eau, sèche sur Na₂SO₄ et évapore le solvant sous vide. On obtient 0,25 g du composé attendu après cristallisation dans l'éther iso.

### EXEMPLE 11 : Composé N° 78

### 4-[6-(2,4-dichlorophényl)-3-oxo-2-[4[(trifluorométhyl)thio]benzyl]-2,3-dihydro-5H-pyrazolo[4,3-c]pyridazin-5-yllphénylpropane-1-sulfonate.

On refroidit à 0°C une solution de 0,17 g du composé N° 76 et 0,08 ml de triéthylamine dans 100 ml de DCM, ajoute 0,07 ml de chlorure de propane-1-sulfonyle et laisse 2 heures sous agitation en laissant remonter la température à T.A. On ajoute de l'eau au mélange réactionnel, extrait au DCM, lave la phase organique à l'eau, sèche sur Na₂SO₄ et évapore le solvant sous vide. On obtient 0,167 g du composé attendu après cristallisation dans l'éther iso.

### EXEMPLE 12 : Composé N° 90 - SAR 117027

### N-[2-[[3-Chloro-4-[5-[4-(méthylthio)phényl]-3-oxo-2-[4-(trifluorométhyl) benzyl]-3,5-dihydro-2H-pyrazolo[4,3-c]pyridazin-6-yl]phényl]thio]éthyl] acétamide.

On laisse 1 heure sous agitation à 60°C un mélange de 0,46ml de N-(2-mercaptoéthyl) acétamide et 0,066g de NaH à 60% dans l'huile dans 25ml de xylène. On ajoute ensuite 1g du composé N°89, 0,12g de Pd₂(dba)₃ et 0,088g de xantphos puis chauffe à reflux pendant une nuit. On concentre le mélange réactionnel sous vide, reprend le résidu par une solution d'HCl à 10%, extrait à l'AcOEt, filtre un insoluble, décante, sèche la phase organique sur Na₂SO₄ et évapore le solvant sous vide. On chromatographie le résidu sur gel de silice en éluant par le mélange DCM/MeOH ( 100/5 ; V/V). On obtient 0,86g du composé attendu après cristallisation dans l'éther iso.
MH⁺ = 644 ; tr = 9,48 (M1).

### EXEMPLE 13 : Composé N° 91 - SAR 115935

6-[2-Chloro-4-[(3-hydroxypropyl)thio]phényl]-5-[4-(méthylthio)phényl]-2-[4-(trifluorométhyl)benzyl]-2,5-dihydro-3*H*-pyrazolo[4,3-c]pyridazin-3-one On laisse 1 heure sous agitation là 60°C un mélange de 0,24g de 3-mercaptopropan-1-ol et de 0,04gde NaH à 60% dans l'huile dans 34ml de xylène. On ajoute ensuite 0,61g du composé N° 89, 0,074g de Pd₂(dba)₃ et 0,055 g de xantphos puis chauffe à reflux pendant une nuit. On concentre le mélange réactionnel sous vide, reprend le résidu par une solution d'HCl à 10%, extrait à l'AcOEt, filtre un insoluble, décante, sèche la phase organique sur Na₂SO₄ et évapore le solvant sous vide. On chromatographie le résidu sur gel de silice en éluant par le mélange DCM/AcOEt (100/30 ; V/V). On obtient 0,68g du composé attendu.
MH⁺ = 617 ; tr = 9,96 (M1).

### EXEMPLE 14 : Composé N° 92 - SAR 117026

### N-[3-[(3-Chloro-4-[5-[4-(méthylthio)phényl]-3-oxo-2-[4-(trifluorométhyl)benzyl]-3,5-dihydro-2H-pyrazolo[4,3-c]pyridazin-6-yl]phényl)thio]propyl]méthane sulfonamide.

### A) 3-[[3-Chloro-4-[5-[4-(méthylthio)phényl]-3-oxo-2-[4-(trifluorométhyl)benzyl]-3,5-dihydro-2H-pyrazolo[4,3-c]pyridazin-6-yl]phényl]thio] propylméthanesulfonate.

On refroidit à 0°C une solution de 0,58g du composé N° 91 et 0,26ml de triéthylamine dans 10ml de DCM, ajoute 0,11ml de chlorure de méthanesulfonyle et laisse une nuit sous agitation en laissant remonter la température à TA. On concentre le mélange réactionnel sous vide, reprend le résidu par une solution tampon pH = 2, extrait au DCM, sèche la phase organique sur Na₂SO₄ et évapore sous vide le solvant. On chromatographie le résidu sur gel de silice en éluant par le mélange DCM/AcOEt (100/35 ; V/V). On obtient 0,53g du composé attendu après cristallisation dans l'éther iso.

### B) N-[3-[(3-Chloro-4-[5-[4-(méthylthio)phényl]-3-oxo-2-[4-(trifluorométhyl)benzyl]-3,5-dihydro-2H-pyrazolo[4,3-c]pyridazin-6-yl]phényl)thio]propyl]méthane sulfonamide.

On refroidit à 0°C une solution de 0,3g de méthanesulfonamide et 0,12g de NaH à 60% dans l'huile dans 15ml de DMF, ajoute 0,52g du composé de l'étape précédente et laisse 5 heures sous agitation en laissant remonter la température à TA. On ajoute ensuite 0,006g de NaI et laisse 30 minutes sous agitation à TA. On concentre le mélange réactionnel sous vide, reprend le résidu à l'eau, extrait à l'AcOEt, sèche la phase organique sur Na₂SO₄ et évapore le solvant sous vide. On chromatographie le résidu sur gel de silice en éluant par le mélange DCM/MeOH (100/5 ; V/V). On obtient 0,19g du composé attendu après cristallisation dans l'éther iso.
MH⁺ = 694 ; tr = 9,98 (M1).

### EXEMPLE 15: Composé N° 94 - SAR 125856

6-[2-Chloro-4-[(3,3,3-trifluoropropyl)thio]phényl]-5-[4-(méthylthio)phényl]-2-[4-(trifluorométhyl)thio]benzyl]-2,5-dihydro-3*H*-pyrazolo[4,3-c]pyridazin-3-one On chauffe à reflux pendant une nuit un mélange de 0,32g du composé N° 93, 0,1g de 3,3,3-trifluoropropane-1-thiolate de sodium, 0,036g de Pd₂(dba)₃ et 0,027g de xantphos dans 20ml de xylène. On concentre le mélange réactionnel sous vide, reprend le résidu par une solution d'HCl à 10%, extrait à l'AcOEt, filtre un insoluble, décante, sèche la phase organique sur Na₂SO₄ et évapore le solvant sous vide. On chromatographie le résidu sur gel de silice en éluant par le mélange DCM/AcOEt (100/20 ; V/V). On obtient 0,11 g du composé attendu.
MH⁺ = 687 ; tr = 11,82 (M1)

### EXEMPLE 16 : Composé N° 95 - SAR 119436

### N-[2-[[3-Chloro-4-[5-[4-(méthylthio)phényl]-3-oxo-2-[4-[(trifluorométhyl)thio]benzyl]-3,5-dihydro-2H-pyrazolo[4,3-c]pyridazin-6-yl]phényl]thio]éthyl]acétamide

On prépare ce composé selon le mode opératoire décrit à l'exemple 12 à partir de 1g du composé N° 93, 0,44ml de N-(2-mercaptoéthyl) acétamide, 0,06g de NaH à 60% dans l'huile, 0,11g de Pd₂(dba)₃ et 0,084g de xantphos dans 25 ml de xylène. On obtient 0,57g du composé attendu.
MH⁺ = 676 ; tr = 9,94 (M1)

### EXEMPLE 17 : Composé N° 96 - SAR 124029

6-[2-Chloro-4-(éthylthio)phényl]-5-[4-(méthylthio)phényl]-2-[4-[(trifluorométhyl)thio]benzyl]-2,5-dihydro-3*H*-pyrazolo[4,3-c]pyridazin-3-one On prépare ce composé selon le mode opératoire décrit à l'exemple 12 à partir de 1g du composé N° 93, 0,07g de NaH à 60% dans l'huile, 0,13ml d'éthanethiol, 0,11g de Pd₂(dba)₃ et 0,084g de xantphos dans 25ml de xylène. On obtient 0,489g du composé attendu.
MH⁺=619;tr= 11,80 (M1)

### EXEMPLE 18 : Composé N° 102 - SAR 100912

6-[2-Chloro-4-[2-(diméthylamino)éthoxy]phényl]-5-[4-(méthylthio)phényl]-2-[4-(trifluorométhyl)benzyl]-2,5-dihydro-3*H*-pyrazolo[4,3-c]pyridazin-3-one A) 6-(2-Chloro-4-hydroxyphényl)-5-[4-(méthythio)phényl]-2-[4-(trifluorométhyl)benzyl]-2,5-dihydro-3*H*-pyrazolo[4,3-c]pyridazin-3-one On refroidit à -20°C une solution de 2g du composé N° 99 dans 40ml de DCM, ajoute sous atmosphère d'azote 10,77ml d'une solution 1 M de BBr₃ dans le DCM et laisse 48 heures sous agitation en laissant remonter la température à TA. On verse le mélange réactionnel sur la glace, extrait au DCM, sèche la phase organique sur Na₂SO₄ et évapore le solvant sous vide. On obtient 2,047g du composé attendu. B) 6-[2-Chloro-4-[2-(diméthylamino)éthoxy]phényl]-5-[4-(méthylthio)phényl]-2-[4-(trifluorométhyl)benzyl]-2,5-dihydro-3H-pyrazolo[4,3-c]pyridazin-3-one On chauffe à reflux pendant une nuit un mélange de 0,5g du composé de l'étape précédente, 0,27g de chlorhydrate de 2-chloro-N,N-diméthyléthanamine et 1,5g de Cs₂SO₃ dans 30ml d'acétonitrile. On concentre sous vide le mélange réactionnel, reprend le résidu à l'eau, extrait au DCM, sèche la phase organique sur Na₂SO₄ et évapore le solvant sous vide. On chromatographie le résidu sur gel de silice en éluant par le mélange DCM/AcOEt (100/30 ; V/V). On obtient 0,05g du composé attendu.
MH⁺ = 614 ; tr = 7,14 (M1)

### EXEMPLE 19 : Composé N° 103 - SAR 157102

6-[2-Chloro-4-(2,2,2-trifluoroéthoxy)phényl]-5-[4-(méthylthio)phényl]-2-[4-(trifluorométhyl)benzyl]-2,5-dihydro-3*H*-pyrazolo[4,3-c]pyàdazin-3-one On chauffe à reflux pendant 2 heures un mélange de 0,45g du composé obtenu à l'étape A de l'exemple 18, 0,38g de 2,2,2-trifluoroéthyltrifluorométhanesulfonate et 0,28g de NaHCO₃ dans 14 ml d'EtOH. On concentre le mélange réactionnel sous vide, reprend le résidu à l'eau, extrait au DCM, sèche la phase organique sur Na₂SO₄ et évapore le solvant sous vide. On chromatographie le résidu sur gel de silice en éluant par le mélange DCM/AcOEt (100/30 ; V/V). On obtient 0,145g du composé attendu après cristallisation dans l'éther iso.
MH⁺ = 625 ; tr = 10,87 (M1)

### EXEMPLE 20 : Composé N° 111 - SSR 154266

### 6-(2,4-Dichlorophényl)-5-[4-[2-(diméthylamino)éthoxy]phényl]-2-[4-(trifluorométhyl)benzyl]-2,5-dihydro-3H-pyrazolo[4,3-c]pyridazin-3-one A) 6-(2,4-Dichlorophényl)-5-(4-hydroxyphényl)-2-[4-(trifluorométhyl)benzyl]-2,5-dihydro-3H-pyrazolo[4,3-c]pyridazin-3-one

On refroidit à -20°C une solution de 4,5g du composé N° 64 dans 83ml de DCM, ajoute sous atmosphère d'azote 24,75ml d'une solution 1M de BBr₃ dans le DCM et laisse 48 heures sous agitation en laissant remonter la température à TA. On verse le mélange réactionnel sur de la glace, extrait au DCM, sèche la phase organique sur Na₂SO₄ et évapore le solvant sous vide. On obtient 4,55g du composé attendu.

### B) 6-(2,4-Dichlorophényl)-5-[4-[2-(diméthylamino)éthoxy]phényl]-2-[4-(trifluorométhyl)benzyl]-2,5-dihydro-3H-pyrazolo[4,3-c]pyridazin-3-one

A un mélange de 0,2g du composé de l'étape précédente dans 20ml de dioxane, on ajoute 0,02g de NaH à 60% dans l'huile et laisse 1 heure 30 minutes sous agitation à TA. On ajoute 0,37g de Cs₂CO₃ puis 0,1g de chlorhydrate de 2-chloro-N,N-diméthyléthanamine et chauffe à reflux pendant 2 heures. On concentre le mélange réactionnel sous vide, reprend le résidu à l'eau, extrait au DCM, sèche la phase organique sur Na₂SO₄ et évapore le solvant sous vide. On chromatographie le résidu sur gel de silice en éluant par le mélange DCM/MeOH (100/5 ; V/V). On obtient 0,24g du composé attendu après cristallisation dans l'éther iso.
MH⁺ = 602 ; tr = 8,67 (M1)

### EXEMPLE 21 : Composé N° 121-SAR137338 A

### Chlorhydrate de 6-[4-[(2-aminoéthyl)thio]-2-chlorophényl]-5-[4-(méthylthio)phényl]-2-[4-(trifluorométhyl)benzyl]-2,5-dihydro-3H-pyrazolo[4,3-c]pyridazin-3-one. -

### A) [2-[(3-Chloro-4-[5-[4-(méthylthio)phényl]-3-oxo-2-[4-(trifluorométhyl)benzyl]-3,5-dihydro-2H-pyrazolo[4,3-c]pyridazin-6-yl]phényl]thio]éthyl]carbamate de tert-butyle.

On laisse 1 heure sous agitation à 60°C un mélange de 3,33ml de (2-mercaptoéthyl)carbamate de *tert*-butyle et 0,31g de NaH à 60% dans l'huile dans 50ml de xylène. On ajoute ensuite 4,63g du composé N° 89, 0,56g de Pd₂(dba)₃ et 0,4g de xantphos puis chauffe à reflux pendant une nuit. On concentre le mélange réactionnel sous vide, reprend le résidu par une solution à 10% d'HCl, extrait à l'AcOEt, filtre un insoluble, décante, sèche la phase organique sur Na₂SO₄ et évapore le solvant sous vide. On chromatographie le résidu sur gel de silice en éluant par le mélange DCM/AcOEt (100/40 ; V/V). On obtient 4,15g du composé attendu après cristallisation dans l'éther iso.

### B) Chlorhydrate de 6-[4-[(2-aminoéthyl)thio]-2-chlorophényl]-5-[4-(méthylthio)phényl]-2-[4-(trifluorométhyl)benzyl]-2,5-dihydro-3H-pyrazolo[4,3-c]pyridazin-3-one.

On laisse 2 heures sous agitation à TA un mélange de 4,15g du composé de l'étape précédente et 43,27ml d'une solution d'éther chlorhydrique 2N dans 15ml de DCM. On concentre le mélange réactionnel sous vide, reprend le résidu à l'éther et essore le précipité formé. On obtient 256g du composé attendu.

### EXEMPLE 22 : Composé N° 122 - SAR137529

### 6-[2-Chloro-4-[[2-(diméthylamino)éthyl]thio]phényl]-5-[4-(méthylthio)phényl]-2-[4-(trifluorométhyl)benzyl]-2,5-dihydro-3H-pyrazolo[4,3-c]pyridazin-3-one.

On laisse une nuit sous agitation à TA un mélange de 0,4g du composé N° 121 sous forme de base libre 0,11 ml d'une solution à 37% de formaldéhyde dans l'eau et 0,89g de triacétoxyborohydrure de sodium dans 25ml de THF. On ajoute ensuite 30ml de MeOH et chauffe à reflux pendant 30 minutes. On concentre le mélange réactionnel sous vide, reprend le résidu à l'eau, alcalinise la phase aqueuse par ajout de NaOH 30%, extrait au DCM, sèche la phase organique sur Na₂SO₄ et évapore le solvant sous vide. On chromatographie le résidu sur gel de silice en éluant par le mélange DCM/MeOH (100/5 ; V/V). On obtient 0,277g du composé attendu après cristallisation dans l'éther iso.

### EXEMPLE 23 : Composé N° 123 - SAR 139296

### 6-(2-chloro-4-{[2-(diethylamino)ethyl]thio}phenyl)-5-[4-(methylthio)phenyl]-2-[4-(trifluoromethyl)benzyl]-2,5-dihydro-3H-pyrazolo[4,3-c]pyridazin-3-one.

On laisse une nuit sous agitation à TA un mélange de 0,45g du composé N°121 sous forme de base libre 0,11ml d'acétaldéhyde et 0,33g de triacétoxyborohydrure de sodium dans 25ml de THF. On ajoute ensuite 30ml de MeOH et chauffe à reflux pendant 30 minutes. On concentre le mélange réactionnel sous vide, reprend le résidu à l'eau, alcanilise la phase aqueuse par ajout de NaOH 30%, extrait au DCM, sèche la phase organique sur Na₂SO₄ et évapore le solvant sous vide. On chromatographie le résidu sur gel de silice en éluant par le mélange DCM/MeOH (100/5 ; V/V). On obtient 0,061 g du composé attendu.

### EXEMPLE 24 : Composé N° 125 - SAR139298

### N-{2-[(3-chloro-4-{5-[4-(methylthio)phenyl]-3-oxo-2-[4-(trifluoromethyl)benzyl]-3,5-dihydro-2H-pyrazolo[4,3-c]pyridazin-6-yl}phenyl)thio]ethyl}methanesulfonamide.

A un mélange de 0,4g du composé N° 121 sous forme de base libre et 0,18ml de triéthylamine dans 15ml de DCM on ajoute 0,06ml de chlorure de méthanesulfonyle et laisse 3 heures sous agitation à TA. On concentre le mélange réactionnel sous vide, reprend le résidu par une solution à 10 % d'HCl, extrait au DCM, sèche la phase organique sur Na₂SO₄ et évapore sous vide le solvant. On chromatographie le résidu sur gel de silice en éluant par le mélange DCM/MeOH (100/5; V/V). On obtient 0,294g du composé attendu.

### EXEMPLE 25 : Composé N° 126 - SAR 140559

### N-{2-[(3-chloro-4-{5-[4-(methylthio)phenyl]-3-oxo-2-[4-(trifluoromethyl)benzyl]-3,5-dihydro-2H-pyrazolo[4,3-c]pyridazin-6-yl}phenyl)thio]ethyl}formamide,

A une solution de 0,45g du composé N°121 dans 1,69ml d'acide formique on ajoute goutte à goutte 0,71ml d'anhydride acétique et laisse une nuit sous agitation à TA. On concentre le mélange réactionnel sous vide, reprend le résidu par de l'eau glacée, alcalinise la phase aqueuse par ajout d'une solution concentrée de NaOH, extrait au DCM, sèche la phase organique sur Na₂SO₄ et évapore le solvant sous vide. On chromatographie le résidu sur gel de silice en éluant par le mélange DCM/MeOH (100/5 ; V/V). On obtient 0,32g du composé attendu après cristallisation dans l'éther iso.

Le tableau qui suit illustre les structures chimiques et les propriétés physiques de quelques exemples de composés selon l'invention. Dans ce tableau :
- Me, Et, nPr, iPr, nBu, tBu représente respectivement des groupes méthyle, éthyle, n-propyle, n-butyle et *tert*-butyle.

**TABLEAU VI**

| | | | | | |
|---|---|---|---|---|---|
| 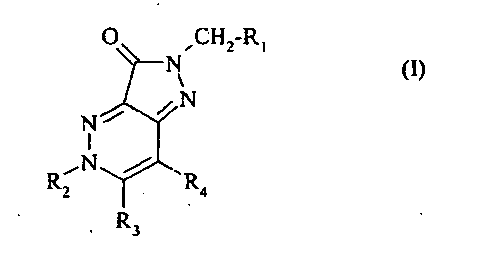 | | | | | |

| Composés N° | R₁ | R₂ | R₃ | R₄ | MH+;tr(mn) (Méthode) RMN |
|---|---|---|---|---|---|
| 1 | 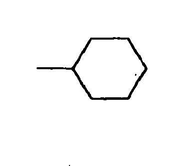 | 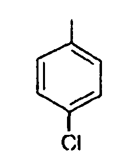 | 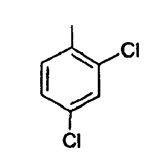 | H | 487 ; 11,24 (M1) |
| 2 | 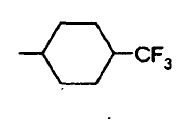 | 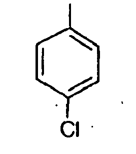 | 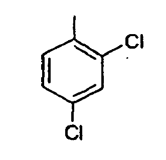 | H | 555 ; 11,45 (M1) Le moins polaire |
| 3 | 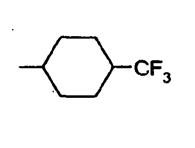 | 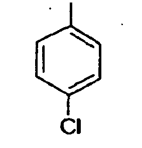 | 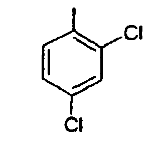 | H | 555 ; 11,42 (M1) Le plus polaire |
| 4 | 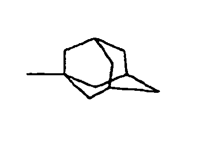 | 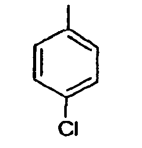 | 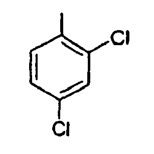 | H | 539 ; 12,40 (M1) |
| 5 | 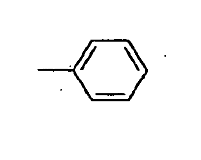 | 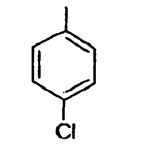 | 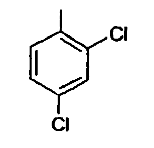 | H | 481 ; 10,53 (M1) |
| 6 | 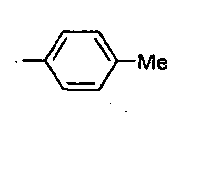 | 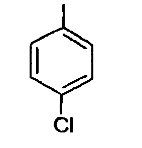 | 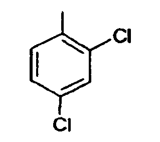 | H | 495 ; 10,79 (M1) |
| 7 | 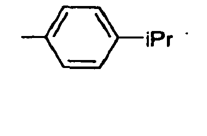 | 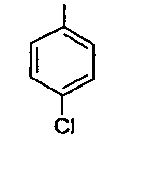 | 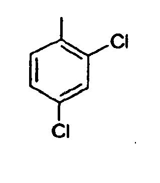 | H | 523 ; 11,69 (M1) RMN |
| 8 | 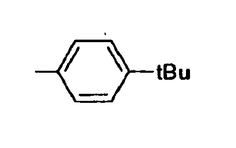 | 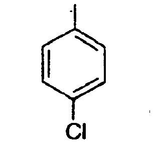 | 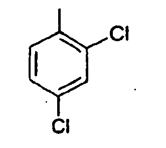 | H | 537 ; 11,92 (M1) RMN |
| 9 | 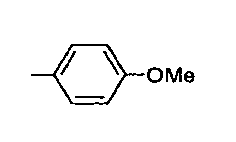 | 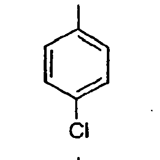 | 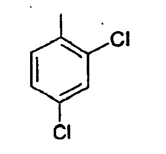 | H | 511 ; 10,45 (M1) |
| 10 | 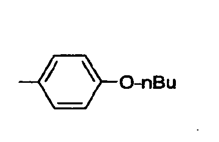 | 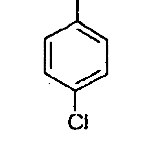 | 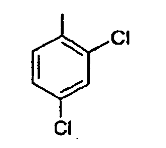 | H | 553; 11,81 (M1) |
| 11 | 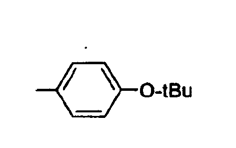 | 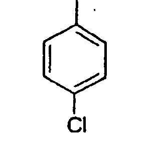 | 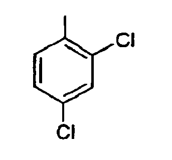 | H | 553; 11,35 (M1) |
| 12 | 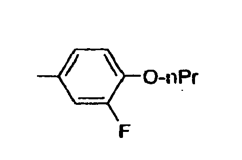 | 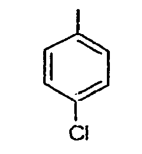 | 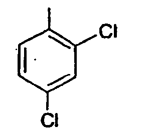 | H | 557 ; 23,75 (M3) |
| 13 | 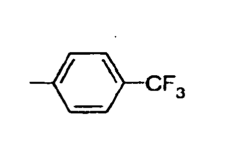 | 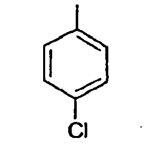 | 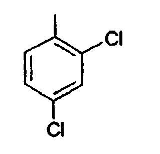 | H | 549 ; 11,17 (M1) RMN |
| 14 | 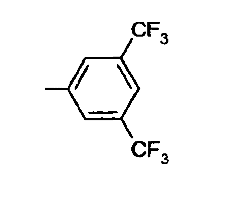 | 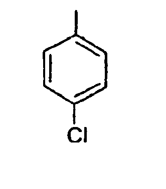 | 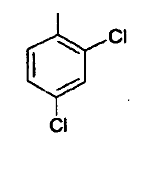 | H | 617 ; 12,09 (M1) |
| 15 | 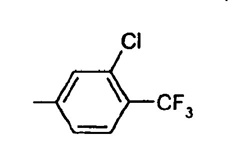 | 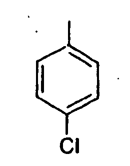 | 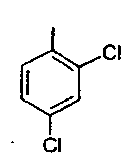 | H | 583;11,71 (M1) |
| 16 | 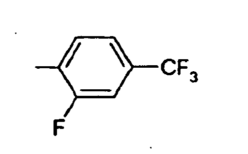 | 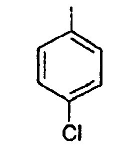 | 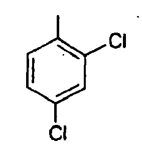 | H | 567; 11,52 (M1) RMN |
| 17 | 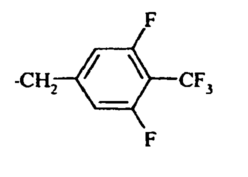 | 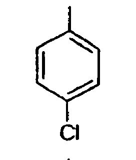 | 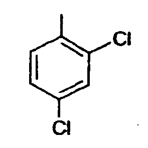 | H | 599 ; 11,59 (M1) |
| 18 | 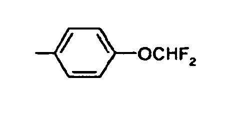 | 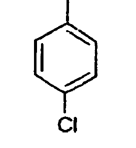 | 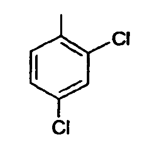 | H | 547; 10,91 (M1) |
| 19 | 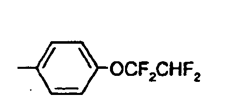 | 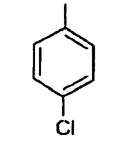 | 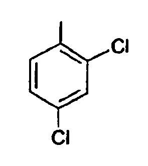 | H | 597 ; 11,31 (M1) |
| 20 | 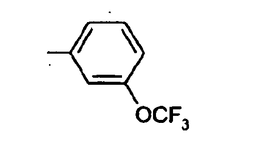 | 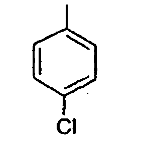 | 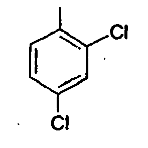 | H | 565 ; 11,38 (M1) |
| 21 | 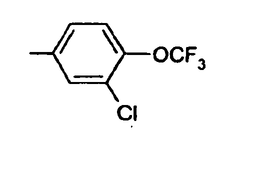 | 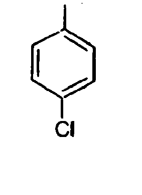 | 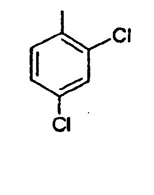 | H | 599; 12,01 (M1) |
| 22 | 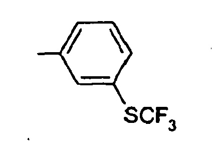 | 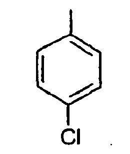 | 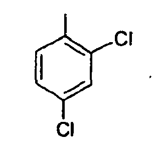 | H | 581 ; 11,93 (M1) |
| 23 | 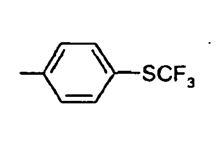 | 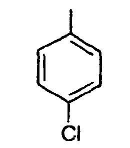 | 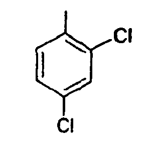 | H | 581 ; 11,98 (M1) RMN |
| 24 | 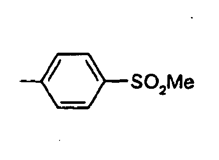 | 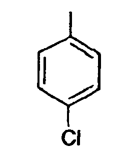 | 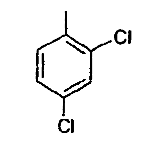 | H | 559 ; 9,52 (M1) |
| 25 | 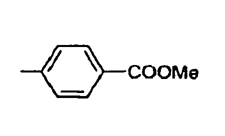 | 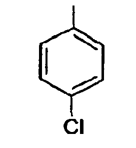 | 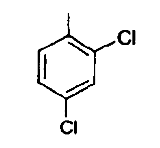 | H | 539 ; 10,30 (M1) |
| 26 | 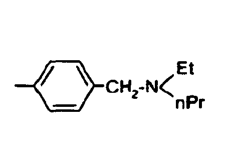 | 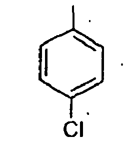 | 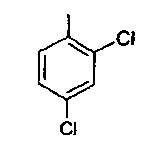 | H | 580 ; 7,69 (M1) HCl |
| 27 | 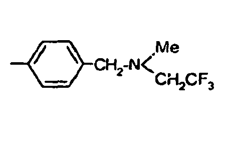 | 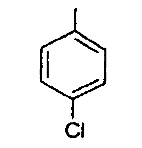 | 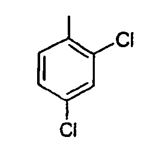 | H | 606 ; 11,32 (M1) HCl |
| 28 | 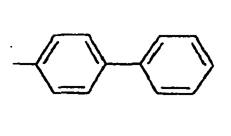 | 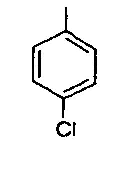 | 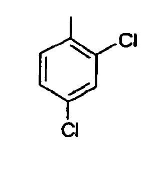 | H | 557 ; 11,60 (M1) |
| 29 | 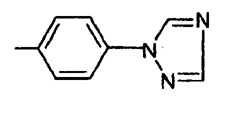 | 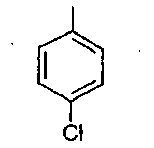 | 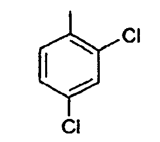 | H | 548 ; 9,50 (M1) |
| 30 | 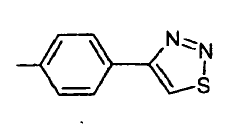 | 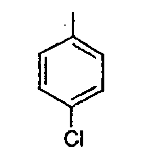 | 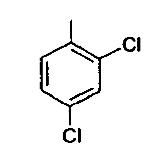 | H | 565 ; 10,36 (M1) |
| 31 | 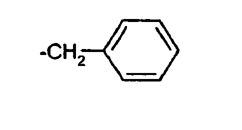 | 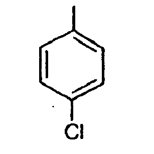 | 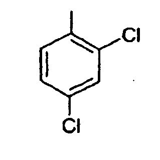 | H | 495 ; 10,72 (M1) |
| 32 | 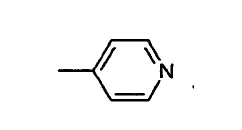 | 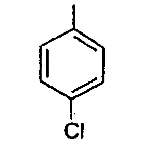 | 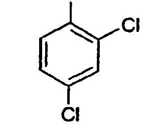 | H | 482 ; 7,54 (M1) |
| 33 | 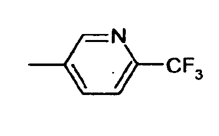 | 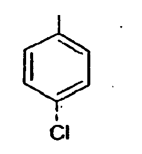 | 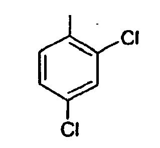 | H | 550 ; 10,46 (M1) |
| 34 | 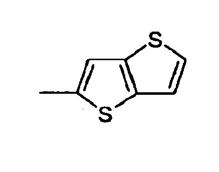 | 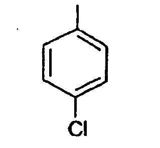 | 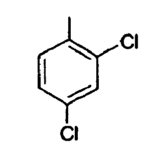 | H | 543 ; 11,16 (M1) |
| 35 | -CH(nPr)₂ | 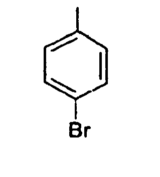 | 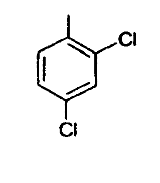 | H | 547 ; 12,29 (M1) |
| 36 | 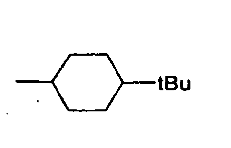 | 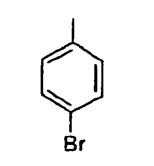 | 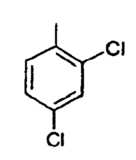 | H | 587 ; 13,05 (M1) Le moins polaire |
| 37 | 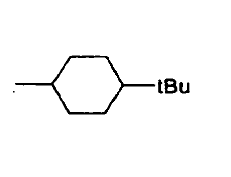 | 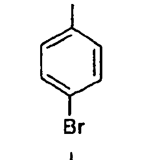 | 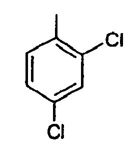 | H | 587 ; 13,03 (M1) Le plus polaire |
| 38 | 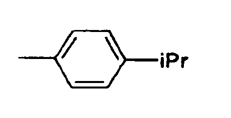 | 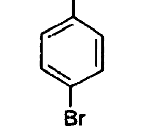 | 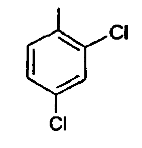 | H | 567; 11,74 (M1) RMN |
| 39 | 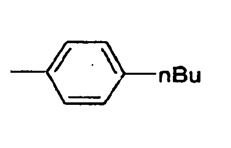 | 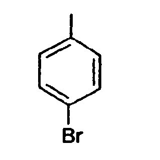 | 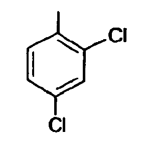 | H | 581 ; 12,27 (M1) RMN |
| 40 | 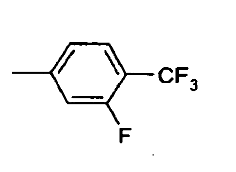 | 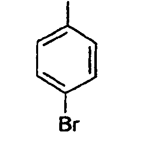 | 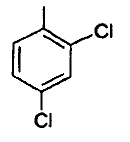 | H | 611 ; 11,47 (M1) RMN |
| 41 | 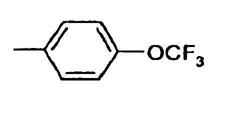 | 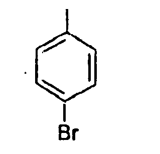 | 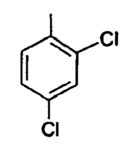 | H | 609; 11,52 (M1) RMN |
| 42 | 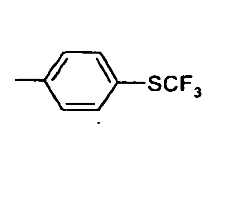 | 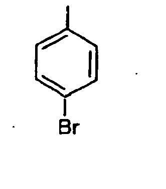 | 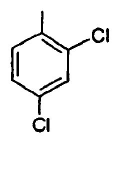 | H | 625; 11,81 (M1) RMN |
| 43 | 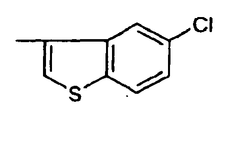 | 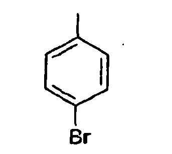 | 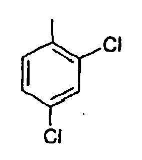 | H | 615 ; 11,86 (M1) |
| 44 | 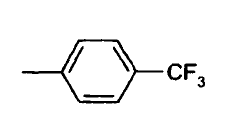 | 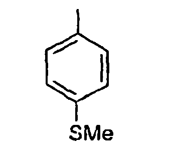 | 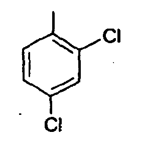 | H | 561 ; 11,27 (M1) RMN |
| 45 | 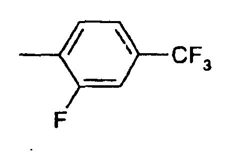 | 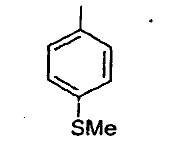 | 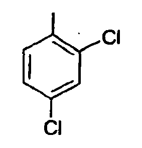 | H | 579 ; 11,05 (M5) RMN |
| 46 | 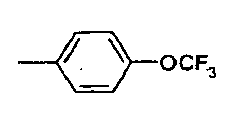 | 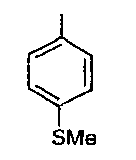 | 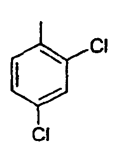 | H | 577 ; 15,07 (M4) RMN |
| 47 | 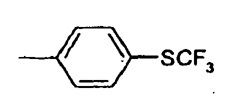 | 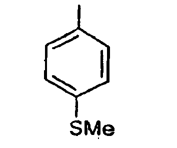 | 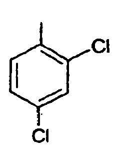 | H | 593 ; 11,70 (M1) |
| 48 | 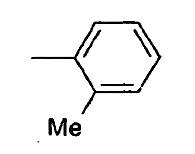 | 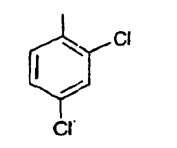 | 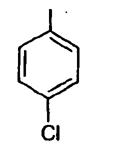 | Me | 509 ; 11,07 (M1) |
| 49 | 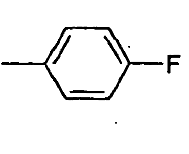 | 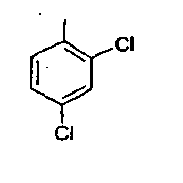 | 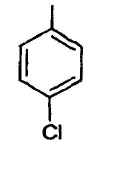 | Me | 513 ; 10,76 (M1) |
| 50 | 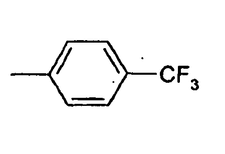 | 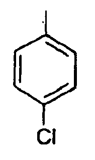 | 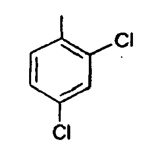 | OMe | 579 ; 11,70 (M1) |
| 51 | 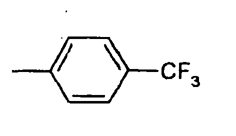 | 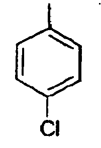 | 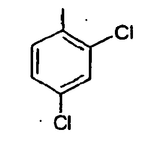 | OH | 565 ; 10,47 (M1) |
| 52 | 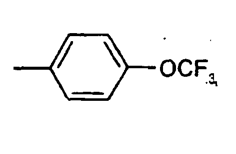 | 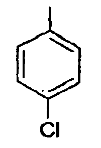 | 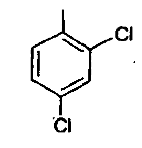 | OMe | 595 ; 11,83 (M1) |
| 53 | 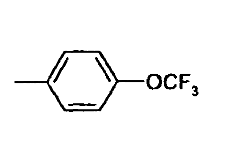 | 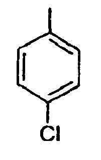 | 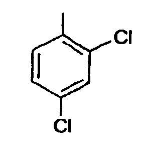 | OH | 581 ; 10,57 (M1) |
| 54 | 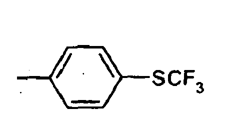 | 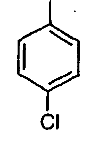 | 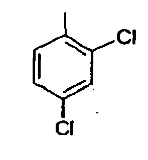 | OMe | 611 ; 12,13 (M1) |
| 55 | 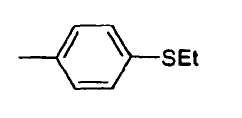 | 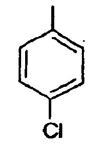 | 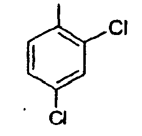 | H | 541; 11,11 (M5) |
| 56 | 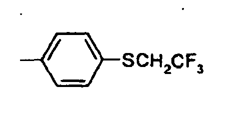 | 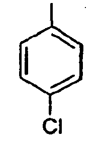 | 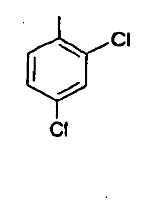 | H | 595 ; 11,45 (M1) |
| 57 | 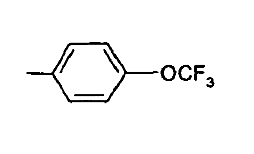 | 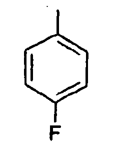 | 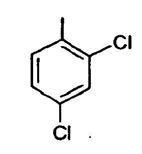 | H | 549 ; 10,97 (M1) |
| 58 | 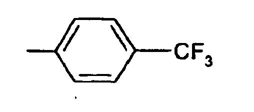 | 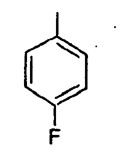 | 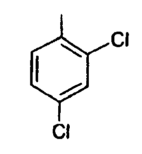 | H | 533 ; 10,83 (M1) |
| 59 | 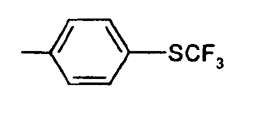 | 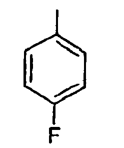 | 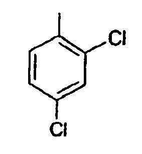 | H | 565; 11,32 (M1) |
| 60 | 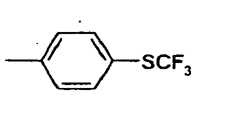 | 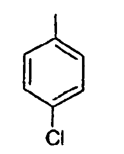 | 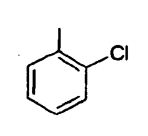 | H | 547 ; 11,10 (M1) RMN |
| 61 | 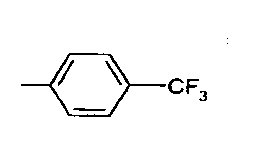 | 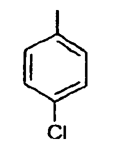 | 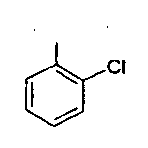 | H | 515 ; 10,60 (M1) |
| 62 | 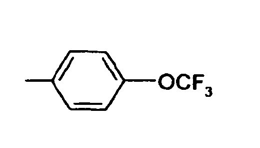 | 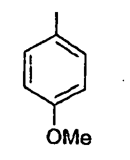 | 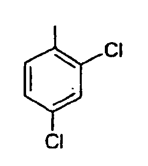 | H | 561 ; 10,60 (M2) |
| 63 | 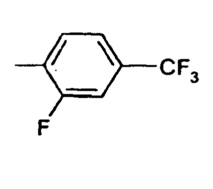 | 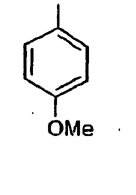 | 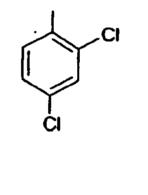 | H | 563; 10,91 (M1) |
| 64 | 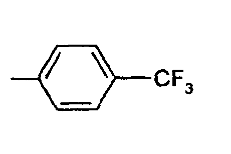 | 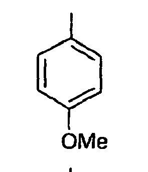 | 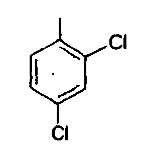 | H | 545 ; 10,77 (M1) |
| 65 | 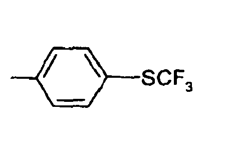 | 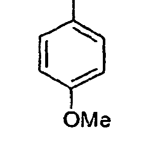 | 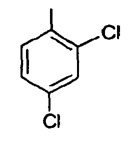 | H | 577 ; 10,90 (M2) RMN |
| 66 | 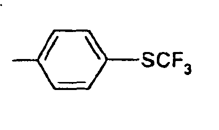 | 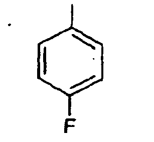 | 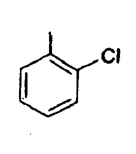 | H | 531 ; 10,70 (M1) RMN |
| 67 | 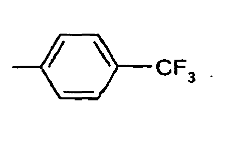 | 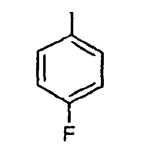 | 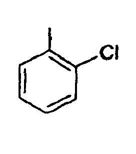 | H | 499 ; 10,22 (M1) |
| 68 | 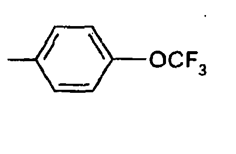 | 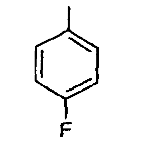 | 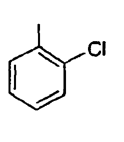 | H | 515 ; 10,38 (M1) |
| 69 | 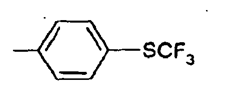 | 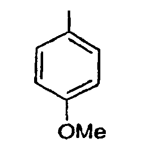 | 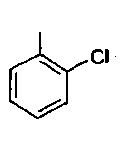 | H | 543 ; 10,43 (M2) |
| 70 | 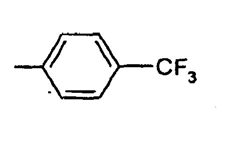 | 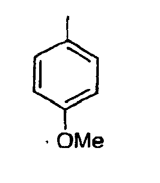 | 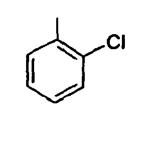 | H | 511 ; 9,92 (M2) |
| 71 | 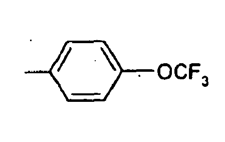 | 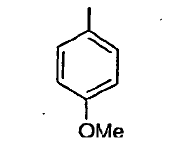 | 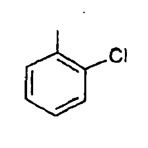 | H | 527 ; 10,08 (M2) |
| 72 | 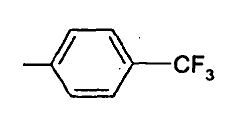 | 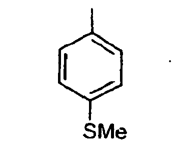 | 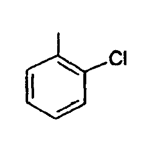 | H | 527; 10,51 (M1) |
| 73 | 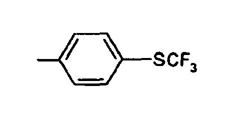 | 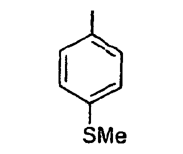 | 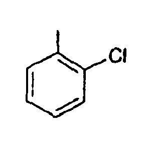 | H | 559 ; 10,97 (M1) |
| 74 | 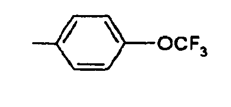 | 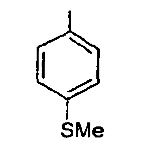 | 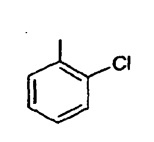 | H | 543 ; 10,62 (M1) |
| 75 | 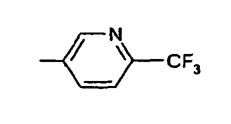 | 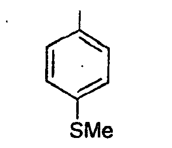 | 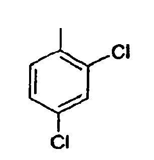 | H | 562 ; 10,34 (M1) |
| 76 | 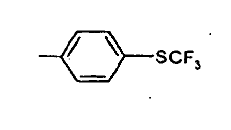 | 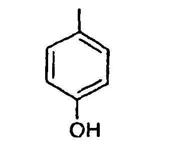 | 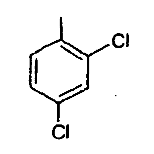 | H | 563; 10,10 (M2) |
| 77 | 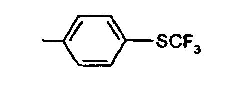 | 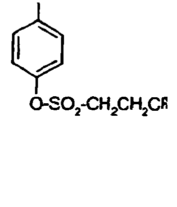 | 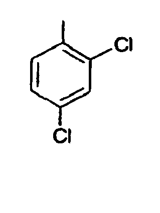 | H | 723 ; 11,49 (M1) RMN |
| 78 | 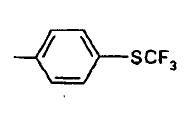 | 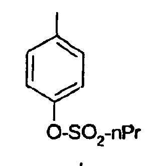 | 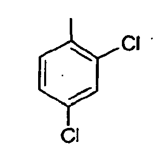 | H | 669 ; 11,39 (M1) |
| 79 | 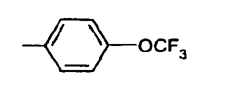 | 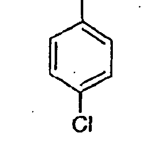 | 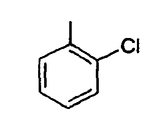 | H | 531 ; 10,73 (M1) |
| 80 SSR15661 2 | 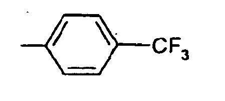 | 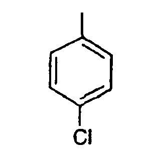 | 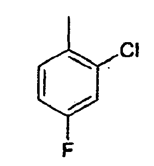 | H | 533 ; 10,68 (M1) |
| 81 SSR15661 3 | 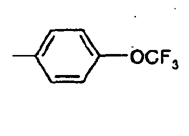 | 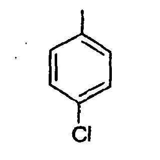 | 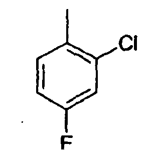 | H | 549 ; 10,83 (M1) |
| 82 SSR15661 4 | 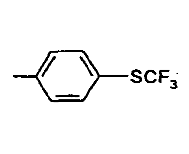 | 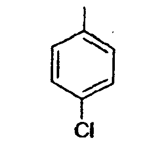 | 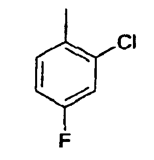 | H | 565 ; 11,16 (M1) |
| 83 SAR11378 7 | 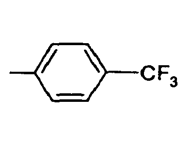 | 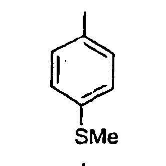 | 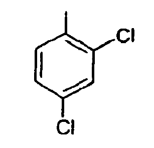 | OMe | 591 ; 11,59 (M1) |
| 84 SAR11395 3 | 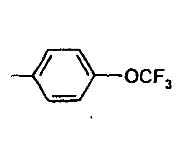 | 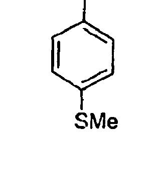 | 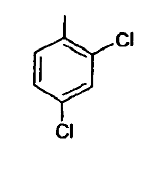 | OMe | 607 ; 11,73 (M1) |
| 85 SAR11378 6 | 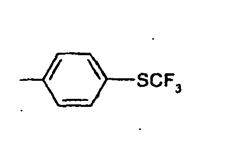 | 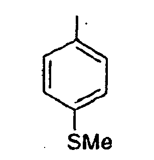 | 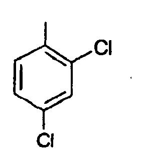 | OMe | 623 ; 12,06 (M1) |
| 86 SSR15680 7 | 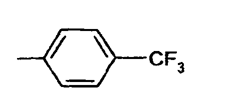 | 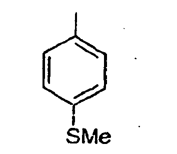 | 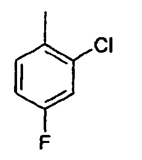 | H | 545 ; 10,32 (M2) |
| 87 SSR15680 8 | 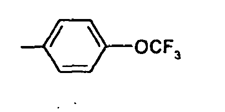 | 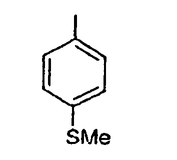 | 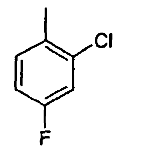 | H | 561 ; 10,46 (M2) |
| 88 SSR15680 9 | 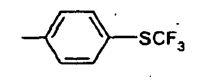 | 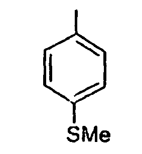 | 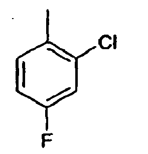 | H | 577 ; 11,03 (M1) |
| 89 SAR11275 3 | 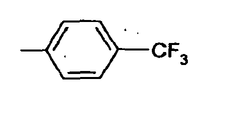 | 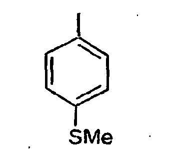 | 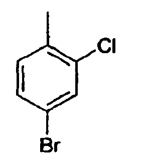 | H | 605 ; 11,18 (M1) RMN |
| 90 SAR11702 7 | 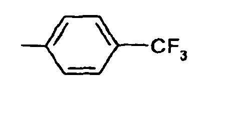 | 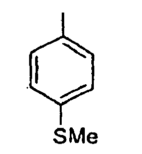 | 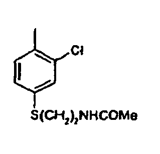 | H | 644 ; 9,48 (M1) |
| 91 SAR11593 5 | 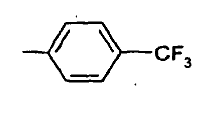 | 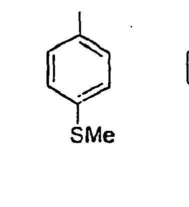 | 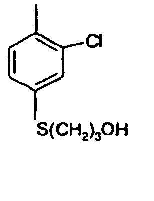 | H | 617 ; 9 ,96 (M1) RMN |
| 92 SAR11702 6 | 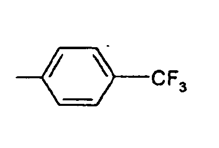 | 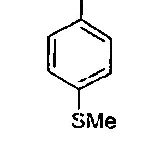 | 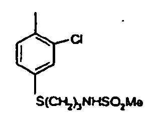 | H | 694 ; 9,98 (M1) |
| 93 SAR11866 6 | 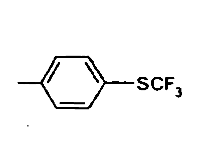 | 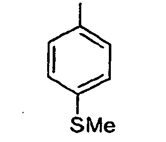 | 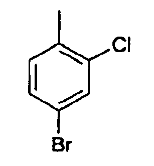 | H | 637 ; 18,70 (M6) RMN |
| 94 SAR12585 6 | 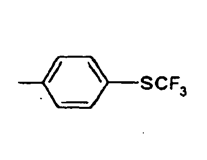 | 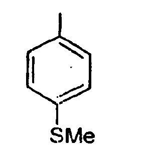 | 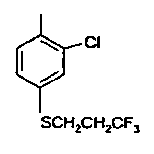 | H | 687; 11,82 (M1) |
| 95 SAR11943 6 | 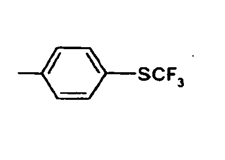 | 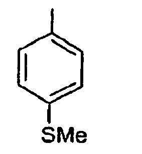 | 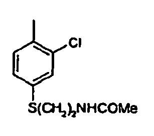 | H | 676 ; 9,94 (M1) RMN |
| 96 SAR12402 9 | 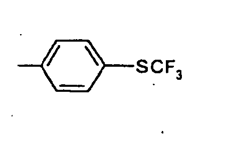 | 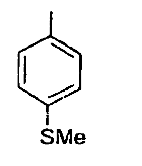 | 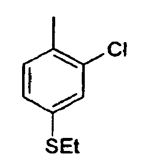 | H | 619 ; 11,80 (M1) |
| 97 SAR11943 5 | 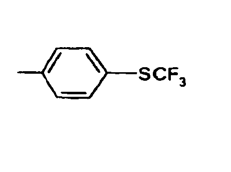 | 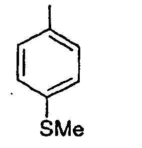 | 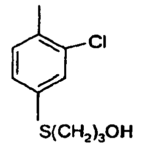 | H | 649 ; 10,40 (M1) RMN |
| 98 SAR12305 7 | 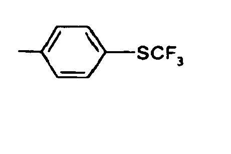 | 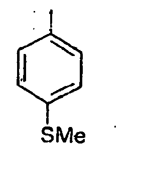 | 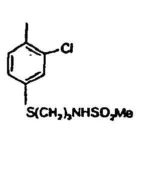 | H | 726; 10,43 (M1) |
| 99 SSR15651 5 | 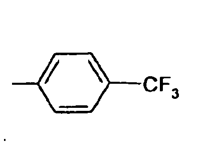 | 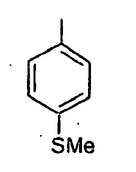 | 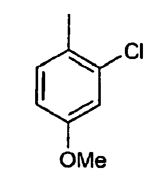 | H | 557 ; 10,52 (M1) RMN |
| 100 SSR15651 6 | 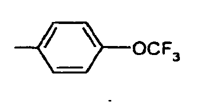 | 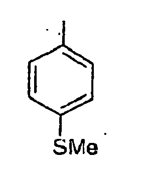 | 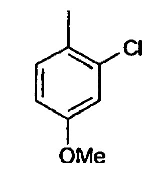 | H | 573 ; 10,28 (M2) RMN |
| 101 SSR15651 7 | 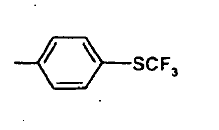 | 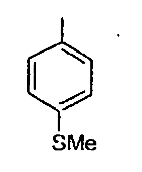 | 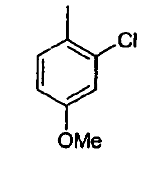 | H | 589 ; 10,62 (M2) RMN |
| 102 SAR10091 2 | 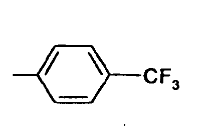 | 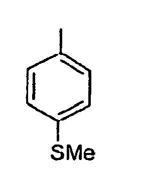 | 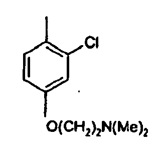 | H | 614 ; 7,14 (M1) RMN |
| 103 SSR15710 2 | 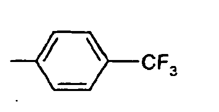 | 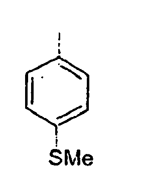 | 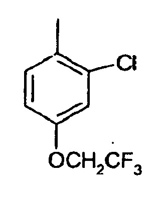 | H | 625 ; 10,87 (M1) |
| 104 SAR10138 4 | 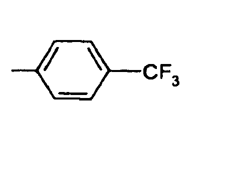 | 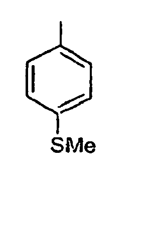 | 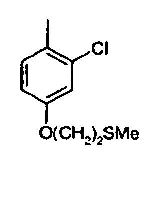 | H | 617; 11,06 (M1) RMN |
| 105 SAR10556 7 | 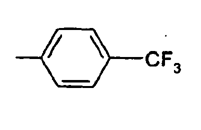 | 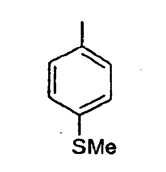 | 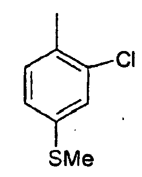 | H | 573 ; 10,91 (M1) RMN |
| 106 SAR10556 6 | 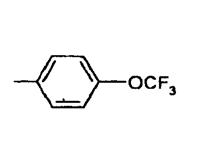 | 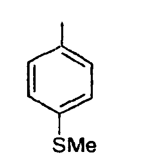 | 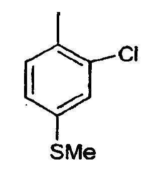 | H | 589;11,07 (M1) |
| 107 SAR10556 5 | 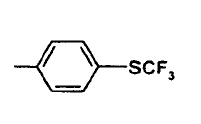 | 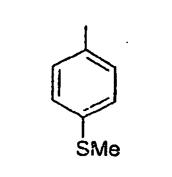 | 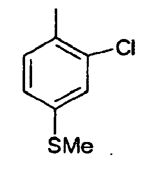 | H | 605 ; 11,40 (M1) RMN |
| 108 SAR12652 6 | 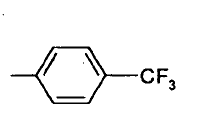 | 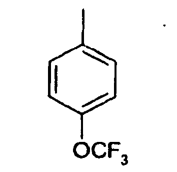 | 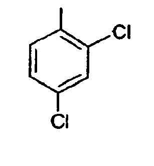 | H | 599; 11,41 (mol) |
| 109 SAR12729 3 | 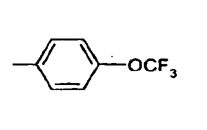 | 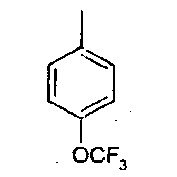 | 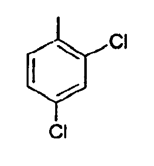 | H | 615 ; 11,54 (M1) |
| 110 SAR12718 3 | 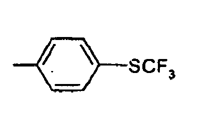 | 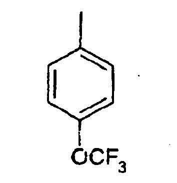 | 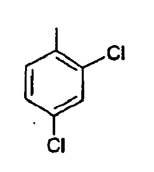 | H | 631 ; 11,85 (M1) |
| 111 SSR15426 6 | 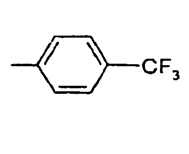 | 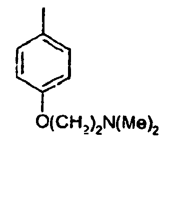 | 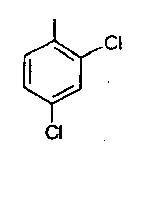 | H | 602 ; 8,67 (M1) |
| 112 SAR12305 8 | 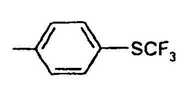 | 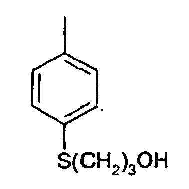 | 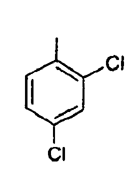 | H | 637 ; 10,66 (M1) |
| 113 SAR12403 0 | 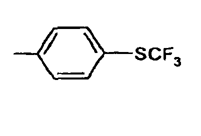 | 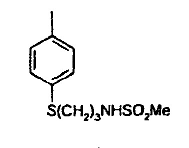 | 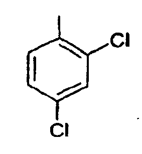 | H | 714 ; 10,75 (M1) RMN |
| 114 | 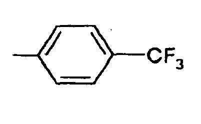 | 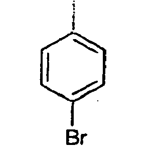 | 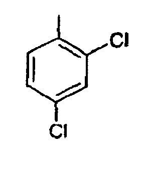 | H | |
| 115 SAR12177 9 | 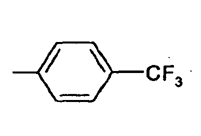 | 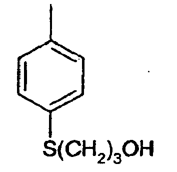 | 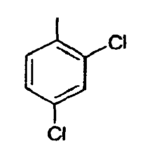 | H | 605 ; 10,17 (M1) |
| 116 SAR12284 8 | 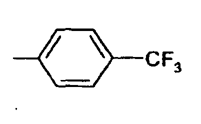 | 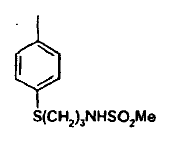 | 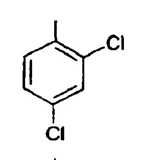 | H | 682 ; 10,28 (M1) RMN |
| 117 SAR12284 7 | 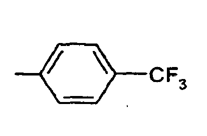 | 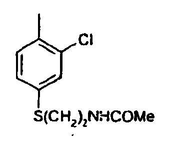 | 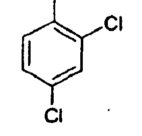 | H | 632 ; 9,77 (M1) |
| 118 SAR 13553 5 | 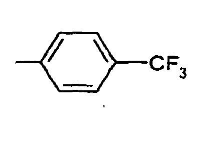 | 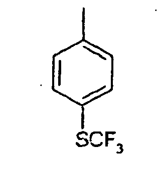 | 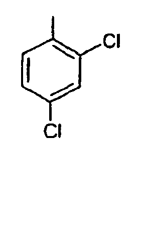 | H | 615 ; 11,76 (M1) |
| 119 SAR13553 7 | 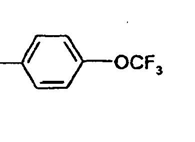 | 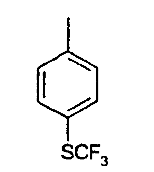 | 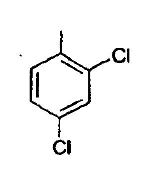 | H | 631 ; 11,86 (M1) |
| 120 SAR13553 6 | 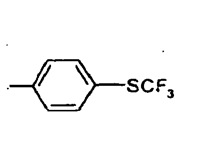 | 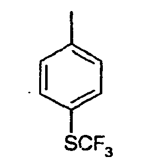 | 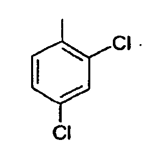 | H | 647 ; 12,18 (M1) |
| 121 SAR13733 8A | 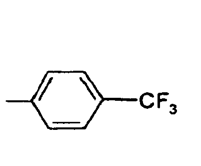 | 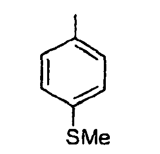 | 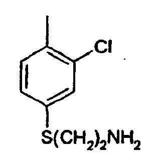 | H | 602 ; 7,22 (M1) RMN |
| 122 SAR13752 9 | 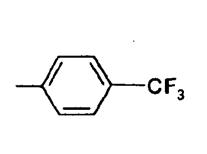 | 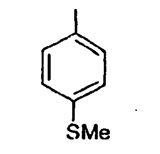 | 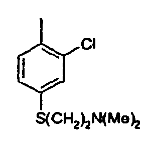 | H | 630; 10,04 (M2) RMN |
| 123 SAR13929 6 | 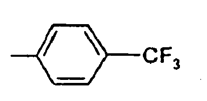 | 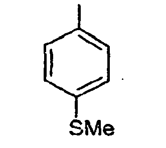 | 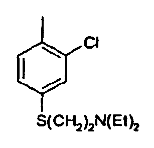 | H | 658 ; 7,48 (M1) RMN |
| 124 SAR14030 8 | 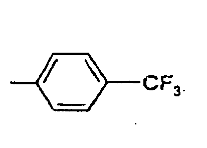 | 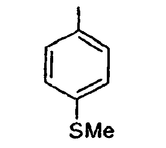 | 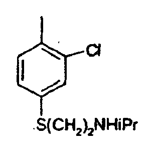 | H | 644 ; 7,49 (M1) RMN |
| 125 SAR13929 8 | 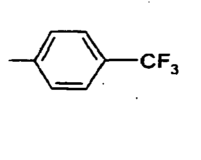 | 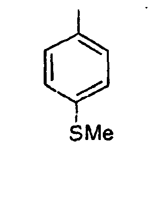 | 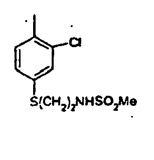 | H | 680 ; 9,8 (M1) RMN |
| 126 SAR14055 9 | 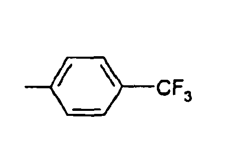 | 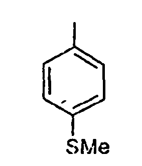 | 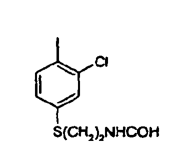 | H | 630 ; 9,41 (M1) |
| 127 SAR14206 1 | 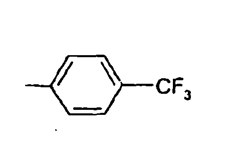 | 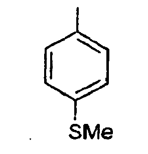 | 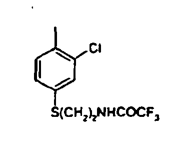 | H | 698 ; 10,54 (M1) RMN |
| 128 SAR14206 2 | 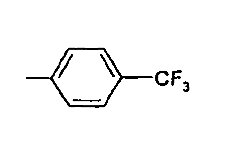 | 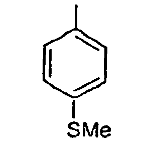 | 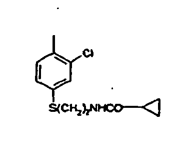 | H | 670 ; 9,97 (M1) RMN |

Composé N° 7 : RMN¹H : DMSO-d₆ (400 MHz) : δ (ppm) : 1,18 : d : 6H ; 2,86 : spt : 1H ; 5,07 : mt : 2H ; 7,22 : mt : 4H ; 7,37-7,77 : m : 8H.
Composé N° 8 : RMN¹H : DMSO-d₆ (200 MHz) : δ (ppm) : 1,25 : s : 9H ; 5,06 : mt : 2H ; 7,14-7,81 : m : 12H.
Composé N° 13 : RMN¹H : DMSO-d₆ (250 MHz) : δ (ppm) : 5,23 : mt : 2H ; 7,40-7,80 : m : 12H.
Composé N° 16 : RMN¹H : DMSO-d₆ (250 MHz) : δ (ppm) : 5,25 : mt : 2H ; 7,37-7,82 : m : 11H.
Composé N° 23 : RMN¹H : DMSO-d₆ (250 MHz) : δ (ppm) : 5,21 : mt : 2H ; 7,28-7,94 : m : 12H.
Composé N° 38 : RMN¹H : DMSO-d₆ (400 MHz) : δ (ppm) : 1,18 : d : 6H ; 2,86 : spt : 1H ; 5,07 : mt : 2H ; 7,14-7,74 : m : 12H.
Composé N° 39 : RMN¹H : DMSO-d₆ (400 MHz) : δ (ppm) : 0,88 : t : 3H ; 1,29 : mt : 2H ; 1,52 : mt : 2H ; 2,55 : mt : 2H ; 7,01-7,85 : m : 12H.
Composé N° 40 : RMN¹H : DMSO-d₆ (250 MHz) : δ (ppm) : 5,24 : mt : 2H ; 7,22-7,88 : m : 11H.
Composé N° 41 : RMN¹H DMSO-d₆ (250 MHz) : δ (ppm) : 5,16 : mt : 2H ; 7,30-7,73 : m : 12H.
Composé N° 42 : RMN¹H : DMSO-d₆ (250 MHz) : δ (ppm) : 5,16 : mt : 2H ; 7,21-7,80 : m : 12H.
Composé N° 44 : RMN¹H : DMSO-d₆ (250 MHz) : δ (ppm) : 2,45 : s: 3H ; 5,23 : mt : 2H ; 7,23 : d : 2H ; 7,36 : d : 2H ; 7,46 : s : 1H ; 7,53 : mt : 3H ; 7,65-7,78 : m : 4H. Composé N° 45 : RMN¹H : DMSO-d₆ (250 MHz) : δ (ppm) : 2,45 : s: 3H ; 5,26 : m : 2H ; 7,23 : d : 2H ; 7,36 : d : 2H ; 7,46 : s : 1H ; 7,49-7,79 : m : 6H.
Composé N° 46 : RMN¹H : DMSO-d₆ (250 MHz) : δ (ppm) : 2,45 : s: 3H ; 5,16: mt : 2H ; 7,23 : d : 2H ; 7,31-7,40 : m : 4H ; 7,42-7,57 : m : 4H ; 7,65-7,72 : m: 2H. Composé N° 60 : RMN¹H : DMSO-d₆ (250 MHz) : δ (ppm) : 5,21 : mt : 2H ; 7,32-7,53 : m : 10H ; 7,61-7,70 : m : 2H.
Composé N° 62 : RMN¹H : DMSO-d₆ (400 MHz) : δ (ppm) : 3,74 : s: 3H ; 5,16: mt : 2H ; 6,91 : d : 2H ; 7,30-7,55 : m : 8H ; 7,61-7,70 : m : 2H.
Composé N° 65 : RMN¹H : DMSO-d₆ (250 MHz) : δ (ppm) : 3,73 : s: 3H ; 5,20 : mt : 2H ; 6,92 : d : 2H ; 7,36 : d : 2H ; 7,42-7,56 : m : 4H ; 7,61-7,79 : m : 4H.
Composé N° 66 : RMN¹H : DMSO-d₆ (250 MHz) : δ (ppm) : 5,20 : mt : 2H ; 7,21 : t : 2H ; 7,31-7,55 : m : 8H ; 7,63 : mt : 1H ; 7,72 : d : 2H.
Composé N° 77 : RMN¹H : DMSO-d₆ (250 MHz) : δ (ppm) : 2,92 : mt : 2H ; 3,88 : mt : 2H ; 5,21 : mt : 2H ; 7,37-7,79 : m : 12H.
Composé N°89 : SAR112753 : RMN¹H : DMSO-d₆ (250 MHz) : δ (ppm) : 2,40 : s : 3H ; 5,18 : dd : 2H ; 7,18 : d : 2H ; 7,31 : d : 2H ; 7,40 : s : 1H ; 7,48 : d : 2H ; 7,55 : d : 1H ; 7,61 : dd : 1H ; 7,69 : d : 2H ; 7,73 : d : 1H.
Composé N°91 : SAR115935 : RMN¹H : DMSO-d₆ (400 MHz) : δ (ppm) : 1,67 : t : 2H ; 2,45 : s : 3H ; 3,03 : t : 2H ; 3,47 : mt : 2H ; 4,61 : t : 1H ; 5,22 : dd : 2H ; 7,22 : d : 2H ; 7,28 : dd : 1H ; 7,31-7,36 : 2mt : 3H ; 7,40 : s : 1H ; 7,53 : d : 3H ; 7,73 : d : 2H.
Composé N°93 : SAR118666 : RMN¹H : DMSO-d₆ (250 MHz) : δ (ppm) : 2,40 : s : 3H ; 5,15 : dd : 2H ; 7,18 : d : 2H ; 7,32 : d : 2H ; 7,42 : 2mt : 3H ; 7,55 : d : 1H ; 7,61 : dd : 1H ; 7,69 : d : 2H ; 7,73 : d : 1H.
Composé N°95 : SAR119436 : RMN₁H : DMSO-d₆ (250 MHz) : δ (ppm) : 1,72 : s : 3H ; 2,39 : s : 3H ; 3,00 : mt : 2H ; 3,17 : mt : 2H ; 5,15 : dd : 2H ; 7,18 : d : 2H ; 7,29 : 2mt : 3H ; 7,35 : s : 1H ; 7,37 : d : 1H ; 7,41 : d : 2H ; 7,49 : d : 1H ; 7,67 : d : 2H ; 8,05 : t : 1H.
Composé N°97 : SARI 19435 : RMN¹H : DMSOd₆ (250 MHz) : δ (ppm) : 1,61 : mt : 2H ; 2,40 : s : 3H ; 2,97 : t : 2H ; 3,42 : mt : 2H ; 4,53: t : 1H ; 5,15 : dd : 2H ; 7,13-7,33 : m : 6H ; 7,36 : s : 1H ; 7,42 : d : 2H ; 7,48 : d : 1H ; 7 ;67 : d : 2H.
Composé N°99 : SSR156515 : RMN¹H : DMSO-d₆ (250 MHz) : δ (ppm) : 2,40 : s : 3H ; 3,70 : s : 3H ; 5,20 : dd : 2H ; 6,92 : d : 1H ; 6,98 : d : 1H ; 7,17 : d : 2H ; 7,29 : d+mt : 3H ; 7,48 : d+mt : 3H ; 7,69 : d : 2H.
Composé N°100 : SSR156516 : RMN¹H : DMSO-d₆ (250 MHz) : δ (ppm) : 2,40 : s : 3H ; 3,71 : s : 3H ; 5,1 : dd : 2H ; 6,97 : dd : 1H ; 6,97 : d : 1H ; 7,16 : d : 2H ; 7,23-7,54 : m : 8H.
Composé N°101 : SSR156517 : RMN¹H : DMSO-d₆ (250 MHz) : δ (ppm) : 2,42 : s : 3H ; 3,71 : s : 3H ; 8,14 : dd : 2H ; 6,92 : dd : 1H ; 6,98 : d : 1H ; 7,17 : mt : 2H ; 7,28 : 2mt : 3H ; 7,42 : d : 2H ; 7,49 : d : 1H ; 7,67 : d : 2H.
Composé N°102 : SAR100912 : RMN¹H : DMSO-d₆ (250 MHz) : δ (ppm) : 2,12 : s : 6H ; 2,39 : s : 3H ; 2,51 : t : 2H ; 3,99 : t : 2H ; 5,17 : dd : 2H ; 6,91 : dd : 1H ; 6,98 : d : 1H ; 7,17 : d : 2H ; 7,29 : d+mt : 3H ; 7,43-7,53 : m : 3H ; 7,69 : d : 2H.
Composé N°104 : SAR101384 : RMN¹H : DMSO-d₆ (250 MHz) : δ (ppm) : 2,06 : s : 3H ; 2,41 : s : 3H ; 2,75 : t : 2H ; 4,10 : t : 2H ; 5,7 : dd : 2H ; 6,93 : dd : 1H ; 7,00 : d : 1H ; 7,16 : d : 2H ; 7,29 : d+mt : 3H ; 7,48 : d : 3H ; 7,68 : d : 2H.
Composé N°105 : SAR105567 : RMN¹H : DMSO-d₆ (400 MHz) : δ (ppm) : 2,46 : s : 3H ; 2,48 : s : 3H ; 5,22 : dd : 2H ; 7,18-2,30 : m : 4H ; 7,31-7,41 : m : 3H ; 7,53 : d : 3H ; 7,73 : d : 2H.
Composé N°107 : SAR105565 : RMN¹H : DMSO-d₆ (400 MHz) : δ (ppm) : 2,46 : s : 3H ; 2,48 : s : 3H ; 5,20 : dd : 2H ; 7,20-7,32 : m : 3H ; 7,33-7,39 : m : 3H ; 7,46 : d : 2H ; 7,53 : d : 1H ; 7,72 : d : 2H.
Composé N°113: SAR124030 : RMN¹H : DMSO-d₆ (250 MHz) : δ (ppm) : 1,65 : m : 2H ; 2,81 : s : 3H ; 2,96 : mt : 4H ; 5,17 : dd : 2H ; 7,02 : t : 1H ; 7,27 : 2d : 4H ; 7,37-7,53 : m : 4H ; 7,56-7,75 : m : 4H.
Composé N°116 : SAR122848 : RMN¹H : DMSO-d₆ (250 MHz) : δ (ppm) : 1,66 : mt : 2H ; 2,83 : s : 3H ; 2,97 : mt : 4H ; 5,18 : dd : 2H ; 7,01 : t : 1H ; 7,28 : 2d : 4H ; 7,41 : s : 3H ; 7,44-7,53 : m : 3H ; 7,63 : mt : 1H ; 7,69 : d : 1H.
Composé N°121 : SAR137338A : RMN¹H : DMSO-d₆ (400 MHz) : δ (ppm) : 2,23 : se : 2H ; 2,45 : s : 3H ; 2,7 : t : 2H ; 3,01 : t : 2H ; 5,23 : dd : 2H ; 7,22 : d : 2H ; 7,31 : d : 1H ; 7,32-7,37 : m : 3H ; 7 ;41 : s : 1H ; 7,5-7,56 : m : 3H ; 7,73 : d : 2H.
Composé N°122 : SAR137529: RMN¹H : DMSO-d₆ (250 MHz) : δ (ppm) : 2,08 : s : 6H ; 2,37 : t : 2H ; 2,40 : s : 3H ; 3,05 : t : 2H ; 5,17 : dd : 2H ; 7,17 : d : 2H ; 7,25 : d : 1H ; 7,27-7,34 : m : 3H ; 7,36 : s : 1H ; 7,45-7,51 : m : 3H ; 7,69 : d : 2H.
Composé N°123 SAR139296 : RMN¹H : DMSO-d₆ (250 MHz) : δ (ppm) : 0,85 : t : 6H ; 2,32-2,5 : m : 4H ; 2,4 : s : 3H ; 2,52 : t : 2H ; 3,02 : t : 2H ; 5,18 : dd : 2H ; 7,17 : d : 2H ; 7,22-7,41 : m : 5H ; 7,42-7,54 : m : 3H ; 7,69 : d : 2H.
Composé N°124 : SAR140308 : RMN¹H : DMSO-d₆ (250 MHz) : δ (ppm) : 0,86 : d : 6H ; 2,4 : s : 3H ; 2,6-2,74 : m : 3H ; 3,02 : t : 2H ; 5,18 : dd : 2H ; 7,17 : d : 2H ; 7,23-7,34 : m : 4H ; 7,36 : s : 1H ; 7,48 : d : 3H ; 7,69 : d : 2H.
Composé N°125 : SAR139298: RMN¹H : DMSO-d₆ (250 MHz) : δ (ppm) : 2,4 : s : 3H ; 2,85 : s : 3H ; 3,08 : s : 4H ; 5,2 : dd : 2H ; 7,18 : d : 2H ; 7,22-7,4 : m : 6H ; 7,44-7,61 : m : 3H ; 7,69 : d : 2H.
Composé N°126 : SAR140559 : RMN¹H : DMSO-d₆ (250 MHz) : δ (ppm) : 2,4 : s : 3H ; 3,03 : t : 2H ; 3,2 : t : 2H ; 5,18 : dd : 2H ; 7,17 : d : 2H ; 7,17 : d : 2H ; 7,25-7,39 : m : 5H ; 7,44-7,55 : m : 3H ; 7,69 : d : 2H ; 7,97 : s : 1H ; 8,17 : t : 1H.
Composé N°127 : SAR142061 : RMN¹H : DMSO-d₆ (250 MHz) : δ (ppm) : 2,39 : s : 3H ; 3,12 : t : 2H ; 3,32 : q : 2H ; 5,18 : dd : 2H ; 7,17 : d : 2H ; 7,26-7,39 : m : 5H ; 7,45-7,54 : m : 3H ; 7,69 : d : 2H ; 9,58 : t : 1H.
Composé N°128 : SAR142062 : RMN¹H : DMSO-d₆ (250 MHz) : δ (ppm) : 0,55-0,66 : m : 4H; 1,45: mt: 1H; 2,4 : s : 3H ; 3,01 : t : 2H ; 3,17 : t : 2H ; 5,18 : dd 2H;7,17 : d : 2H ; 7,26-7,38 : m : 5H ; 7,44-7,55 : m : 3H ; 7,69 : d : 2H ; 8,28 : t : 1H.

Les composés de formule (I) possèdent une très bonne affinité in vitro (IC50 ≤ 5.10-7M) pour les récepteurs aux cannabinoïdes CB1 humain ou de rongeur, dans les conditions expérimentales décrites par M. Rinaldi-Carmona et al. (FEBS Letters, 1994, 350, 240-244).

La nature antagoniste des composés de formule (I) a été démontrée par les résultats obtenus dans les modèles de l'inhibition de l'adénylate-cyclase comme décrits dans M. Bouaboula et al., .J. Biol. Chem., 1995, 270, 13973-13980, M. Rinaldi-Carmona et al., J. Pharmacol. Exp. Ther., 1996, 278, 871-878 et M. Bouaboula et al., J. Biol. Chem., 1997, 272, 22330-22339.

L'interaction d'un composé de formule (I) avec les récepteurs CB1 présents dans le cerveau est déterminée chez la souris avec le test de binding ex vivo du [3H]-CP55940 après une injection intraveineuse ou une administration orale comme décrit dans Rinaldi-Carmona M. et al., FEBS Letters 1994, 350, 240-244, Rinaldi-Carmona M. et al., Life Sciences 1995, 56, 1941-1947 et Rinaldi-Carmona M. et al., J. Pharmacol. Exp. Ther. 2004, 310, 905-914.

L'interaction d'un composé de formule (I) avec les récepteurs CB1 présents à la périphérie est déterminée chez la souris avec le test de réversion de l'effet inhibiteur du CP55940 sur transit gastrointestinal après une administration orale comme décrit dans Rinaldi-Carmona M. et al., J. Pharmacol. Exp. Ther. 2004, 310, 905-914.

La toxicité des composés de formule (I) est compatible avec leur utilisation en tant que médicament.

Ainsi, selon un autre de ses aspects, l'invention a pour objet des médicaments pour la médecine humaine ou vétérinaire qui comprennent un composé de formule (I), ou un sel d'addition de ce dernier à un acide pharmaceutiquement acceptable, ou encore un solvat ou un hydrate du composé de formule (I).

Ainsi les composés selon l'invention peuvent être utilisés chez l'homme ou chez l'animal (notamment chez les mammifères incluant de façon non limitative les chiens, les chats, les chevaux, les bovins, les moutons) dans le traitement ou la prévention de maladies impliquant les récepteurs aux cannabinoïdes CB₁.

Par exemple et de manière non limitative, les composés de formule (I) sont utiles comme médicaments psychotropes, notamment pour le traitement des désordres psychiatriques incluant l'anxiété, la dépression, les troubles de l'humeur, l'insomnie, les troubles délirants, les troubles obsessionnels, les psychoses en général, la schizophrénie, les troubles de l'attention et de l'hyperactivité (TDAH) chez les enfants hyperkinétiques ainsi que pour le traitement des troubles liés à l'utilisation de substances psychotropes, notamment dans le cas d'un abus d'une substance et/ou de dépendance à une substance, y compris la dépendance alcoolique et la dépendance nicotinique.

Les composés de formule (I) selon l'invention peuvent être utilisés comme médicaments pour le traitement de la migraine, du stress, des maladies d'origine psychosomatique, des crises d'attaques de panique, de l'épilepsie, des troubles du mouvement, en particulier des dyskinésies ou de la maladie de Parkinson, des tremblements et de la dystonie.

Les composés de formule (I) selon l'invention peuvent également être utilisés comme médicaments dans le traitement des troubles mnésiques, des troubles cognitifs, en particulier dans le traitement des démences séniles, de la maladie d'Alzheimer, ainsi que dans le traitement des troubles de l'attention ou de la vigilance.

De plus, les composés de formule (I) peuvent être utiles comme neuroprotecteurs, dans le traitement de l'ischémie, des traumatismes crâniens et le traitement des maladies neurodégénératives aigües ou chroniques : incluant la chorée, la chorée de Huntington, le syndrome de Tourrette.

Les composés de formule (I) selon l'invention peuvent être utilisés comme médicaments dans le traitement de la douleur: les douleurs neuropathiques, les douleurs aiguës périphériques, les douleurs chroniques d'origine inflammatoire, les douleurs induites par un traitement anticancéreux.

Les composés de formule (I) selon l'invention peuvent être utilisés comme médicaments dans la médecine humaine ou vétérinaire dans le traitement et la prévention des troubles de l'appétit, de l'appétence (pour les sucres, carbohydrates, drogues, alcools ou toute substance appétissante) et/ou des conduites alimentaires, notamment pour le traitement de l'obésité ou de la boulimie ainsi que pour le traitement du diabète de type II ou diabète non insulinodépendant et pour le traitement des dyslipidémies, du syndrome métabolique. Ainsi les composés de formule (I) selon l'invention sont utiles dans le traitement et la prévention de l'obésité et des risques associés à l'obésité, notamment les risques cardio-vasculaires.

De plus, les composés de formule (I) selon l'invention peuvent être utilisés en tant que médicaments dans le traitement et la prévention des troubles gastro-intestinaux, des troubles diarrhéiques, des ulcères, des vomissements, des troubles vésicaux et urinaires, des maladies du foie telles que la cirrhose chronique, la fibrose, la stéatose hépatique, la stéatohépatite ; ainsi que des troubles d'origine endocrinienne, des troubles cardio-vasculaires, de l'hypotension, de l'atherosclérose, du choc hémorragique, du choc septique, de l'asthme, de la bronchite chronique, des maladies pulmonaires chroniques obstructives, du syndrome de Raynaud, du glaucome, des troubles de la fertilité, de l'interruption de grossesse, de l'accouchement prématuré, des phénomènes inflammatoires, des maladies du système immunitaire, en particulier autoimmunes et neuroinflammatoires tel que l'arthrite rhumatoïde, l'arthrite réactionnelle, les maladies entraînant une démyélinisation, la sclérose en plaque, des maladies infectieuses et virales telles que les encéphalites, des accidents vasculaires cérébraux ainsi qu'en tant que médicaments pour la chimiothérapie anticancéreuse, pour le traitement du syndrome de Guillain-Barré et pour le traitement des maladies des os et de l'ostéoporose.

De plus, les composés de formule (I) selon l'invention peuvent être utilisés pour leurs effets protecteurs contre la cardiotoxicité induite par les médicaments.

Selon la présente invention, les composés de formule (I) sont tout particulièrement utiles pour le traitement des désordres psychiatriques, en particulier la schizophrénie, les troubles de l'attention et de la vigilance, les troubles de l'attention et de l'hyperactivité (TDAH) chez les enfants hyperkinétiques ; pour le traitement des troubles de l'appétit et de l'obésité ; pour le traitement des déficits mnésiques et des troubles cognitifs ; pour le traitement de la dépendance et du sevrage à une substance, en particulier la dépendance alcoolique, la dépendance nicotinique, le sevrage alcoolique et le sevrage tabagique ; des maladies neurodégénératives aiguës ou chroniques.

Plus particulièrement, les composés de formule (1) selon la présente invention sont utiles dans la préparation de médicaments utiles dans le traitement et la prévention des troubles de l'appétit, les troubles de l'appétence, des troubles du métabolisme, l'obésité, le diabète de type II, le syndrome métabolique, la dyslipidémie, des troubles gastro-intestinaux, des phénomènes inflammatoires, des maladies du système immunitaire, des troubles psychotiques, de la dépendance alcoolique, de la dépendance nicotinique.

Selon un de ses aspects, la présente invention est relative à un composé de formule (I), de ses sels pharmaceutiquement acceptables et de ses solvats ou hydrates pour le traitement des troubles et maladies indiqués ci-dessus.

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention, ou un sel pharmaceutiquement acceptable, un solvat ou hydrate dudit composé, ainsi qu'au moins un excipient pharmaceutiquement acceptable.

Lesdits excipients sont choisis selon la forme pharmaceutique et le mode d'administration souhaité, parmi les excipients habituels qui sont connus de l'Homme du métier.

Les compositions pharmaceutiques selon la présente invention peuvent contenir à côté d'un composé de formule (I) un (ou plusieurs) autre principe actif utile dans le traitement des troubles et maladies indiquées ci-dessus.

Ainsi, la présente invention a également pour objet des compositions pharmaceutiques contenant un composé de formule (I) selon la présente invention associé à un (ou plusieurs) principe actif choisi parmi l'une des classes thérapeutiques suivantes :
- un autre antagoniste ou modulateurs allosteriques des récepteurs CB₁ aux cannabinoïdes ;
- un modulateur des récepteurs CB₂ aux cannabinoïdes ;
- un antagoniste des récepteurs AT₁ de l'angiotensine II ;
- un inhibiteur de l'enzyme de conversion ;
- un antagoniste calcique ;
- un diurétique ;
- un béta-bloquant ;
- un antihyperlipémiant ou un antihypercholestérolémiant ;
- un antidiabétique ;
- un autre agent anti-obésité ou agissant sur les troubles du métabolisme ;
- un agoniste nicotinique, un agoniste nicotinique partiel ;
- un antidépresseur, un antispychotique, un anxiolytique ;
- un anticancéreux ou un agent antiprolifératif ;
- un antagoniste des opioïdes ;
ainsi que :
- un agent améliorant la mémoire ;
- un agent utile dans le traitement de l'alcoolisme ou des symptômes de sevrage ;
- un agent utile pour traiter l'ostéoporose ;
- un anti-inflammatoire non stéroïdien ou stéroïdien ;
- un anti-infectieux ;
- un analgésique ;
- un antiasthmatique.

Par antagoniste des récepteurs AT₁ de l'angiotensine II, on entend un composé tel que candésartan cilexitil, éprosartan, irbésartan, losartan potassium, olmésartan médoxomil, telmisartan, valsartan, chacun de ces composés pouvant être lui-même associé à un diurétique tel que l'hydrochlorothiazide.

Par inhibiteur de l'enzyme de conversion, on entend un composé tel que alacépril, bénazépril, captopril, cilazapril, énalapril, énalaprilat, fosinopril, imidapril; lisinopril, moexipril, périndopril, quinapril, ramipril, spirapril, temocapril, trandolapril, zofénopril, chacun de ces composés pouvant lui-même être associé à un diurétique tel que l'hydrochlorothiazide ou l'indapamide ou à un antagoniste calcique tel que l'amlodipine, le diltiazem, le félodipine ou le vérapamil.

Par antagoniste calcique, on entend un composé tel que amlodipine, aranidipine, bénidipine, bépridil, cilnidipine, diltiazem, éfonidipine hydrochloride éthanol, fasudil, félodipine, isradipine, lacidipine, lercanidipine hydrochloride, manidipine, mibéfradil hydrochloride, nicardipine, nifédipine, nilvadipine, nimodipine, nisoldipine, nitrendipine, térodiline, vérapamil.

Par béta-bloquant, on entend un composé tel que acébutolol, alprénolol, amosulalol, arotinolol, aténolol, béfunolol, bétaxolol, bévantolol, bisoprolol, bopindolol, bucumolol, bufétolol, bunitrolol, butofilolol, carazolol, cartéolol, carvédilol, cloranolol, épanolol, esmolol, indénolol, labétalol, landiolol, lévobunolol, lévomoprolol, mépindolol, métipranolol, métoprolol, nadolol, nébivolol, nifénalol, nipradilol, oxprénolol, penbutolol, pindolol, propranolol, salmétérol, sotalol, talinolol, tertatolol, tilisolol, timolol, xamotérol, xibénolol.

Par antihyperlipémiant ou antihypercholestérolémiant, on entend un composé choisi parmi les fibrates tels que alufibrate, béclobrate, bézafibrate, ciprofibrate, clinofibrate, clofibrate, étofibrate, fénofibrate ; les statines (inhibiteurs de HMG-CoA reductase), telles que atorvastatine, fluvastatine sodium, lovastatine, pravastatine, rosuvastatine, simvastatine, ou un composé tel que acipimox, aluminum nicotinate, azacostérol, cholestyramine, dextrothyroxine, méglutol, nicéritrol, nicoclonate, acide nicotinique, béta-sitosterol, tiadénol.

Par antidiabétique, on entend un composé appartenant à l'une des classes suivantes : les sulfonylurées, les biguanidines, les inhibiteurs d'alpha glucosidase, les thiazolidinedione, les métiglinides, tel que acarbose, acétohexamide, carbutamide, chlorpropamide, glibenclamide, glibomuride, gliclazide, glimépiride, glipizide, gliquidone, glisoxepide, glybuzole, glymidine, métahexamide, métformin, miglitol, natéglinide, pioglitazone, répaglinide, rosiglitazone, tolazamide, tolbutamide, troglitazone, voglibose, ainsi que l'insuline et les analogues de l'insuline.

Par autre agent anti-obésité ou agissant sur les troubles du métabolisme, on entend un composé tel que amfépramone, benfluorex, benzphétamine, indanorex, mazindole, méfénorex, méthamphétamine, D-norpseudoéphédrine, sibutramine, un topiramate, un inhibiteur de lipase (orlistat cetilistat), un agoniste PPAR (de l'anglais Peroxisome Proliferator Activated Receptor Agonist), un agoniste de la dopamine, un agoniste des récepteurs de leptine, un inhibiteur du reuptake de la sérotonine, un béta-3 agoniste, un CCK-A agoniste, un inhibiteur de NPY,un agoniste des récepteurs MC4, un antagoniste des récepteurs MCH (de l'anglais Melanin Concentrating Hormone), un antagoniste de l'orexine, un inhibiteur de phosphodiesterase, un inhibiteur de 11βHSD (11-β-hydroxy steroid deshydrogenase), un inhibiteur de DPP-IV (dipeptidyl peptidase IV), un antagoniste (ou agoniste inverse de l'histamine H3, un dérivé CNTF (de l'anglais Ciliary Neurotrophic Factor), un agoniste des récepteurs GHS (de l'anglais Growth Hormone Secretagogue, un modulateur de la ghréline, un inhibiteur de diacyglycerol acyltransferase (DGAT), un inhibiteur de phosphodiesterase (PDE), un agoniste d'hormone thyroïdienne, un antagoniste des récepteurs glucocorticoïdes, un inhibiteur de stearoyl-CoA- desaturase (SCD), un modulateur des transporteurs de phosphate, de glucose, d'acide gras, de dicarboxylate, un antagoniste 5HT₂, un antagoniste 5HT₆, un agoniste de la bombesine.

Par antagoniste des opioïdes on entend un composé tel que naltrexone, naloxone ou nalméfène.

Par agent utile dans le traitement de l'alcoolisme ainsi que des symptômes de sevrage on entend l'acamprosate, les benzodiazepines, les béta-bloquants, la clonidine, la carbamazépine.

Par agent utile, pour traiter l'ostéoporose, on entend par exemple, les biphosphonates tels que étidronate, clodronate, tiludronate, risédronate.

Selon !a présente invention, on peut également associer d'autres composés ayant des propriétés antihyperlipemiantes, antihypercholesterolemiantes, antidiabétiques ou anti-obésité. Plus particulièrement on peut associer des composés appartenant à l'une des classes suivantes :
inhibiteurs de PTP 1 B (de l'anglais Protein Tyrosine Phosphase -1B), les agonistes des récepteurs VPAC 2, les modulateurs de GLK, les modulateurs retinoides, les inhibiteurs de glycogen phosporylase (HGLPa), les antagonistes du glucagon, les inhibiteurs de glucose-6 phosphate, les activateurs de pyruvate dehydrogenase kinase (PKD), les modulateurs de RXR, FXR, LXR, les inhibiteurs de SGLT (de l'anglais Sodium Dependant Glucose Transporter), les inhibiteurs de CETP (de l'anglais Cholesterylester Transfer Protein), les inhibiteurs de squalene synthetase, les inhibiteurs de squalene epoxidase, les inhibiteurs de synthèse des triglycérides, les inducteurs de récepteurs LDL (de l'anglais Low Density Lipoprotein), les inhibiteurs de IBAT, les inhibiteurs de FBPase (fructose-1,6-biphosphatase), les modulateurs de CART (de l'anglais Cocaïne-Amphétamine-Regulated Transcript),les modulateurs MC 4 (mélanocortin 4), les antagonistes des récepteurs de l'oréxine.

Selon un autre aspect de l'invention, le composé de formule (I), ou un de ses solvats ou hydrates et l'autre principe actif associé peuvent être administrés de manière simultanée, séparée ou étalée dans le temps.

On entend par « utilisation simultanée » l'administration des composés de la composition selon l'invention compris dans une seule et même forme pharmaceutique.

On entend par « utilisation séparée » l'administration, en même temps, des deux composés de la composition selon l'invention chacun compris dans une forme pharmaceutique distincte.

On entend par « utilisation étalée dans le temps » l'administration successive, du premier composé de la composition de l'invention, compris dans une forme pharmaceutique, puis du deuxième composé de la composition selon l'invention, compris dans une forme pharmaceutique distincte. Dans ce cas, le laps de temps écoulé entre l'administration du premier composé de la composition selon l'invention et l'administration du deuxième composé de la même composition selon l'invention n'excède généralement pas 24 heures.

Dans les compositions pharmaceutiques de la présente- invention pour l'administration orale, sublinguale, sous-cutanée, intramusculaire, intra-veineuse, topique, locale, intratrachéale, intranasale, transdermique ou rectale, le principe actif de formule (I) ci-dessus, ou son solvat ou hydrate éventuel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | | |
|---|---|---|
| Composé selon l'invention | : | 50,0 mg |
| Mannitol | : | 223,75 mg |
| Croscarmellose sodique | : | 6,0 mg |
| Amidon de maïs | : | 15,0 mg |
| Hydroxypropyl-méthylcellulose | : | 2,25 mg |
| Stéarate de magnésium | : | 3,0 mg |

Par voie orale, la dose de principe actif administrée par jour peut atteindre 0,01 à 100 mg/kg, en une ou plusieurs prises, préférentiellement 0,02 à 50 mg/kg.

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

La présente invention, selon un autre de ses aspects, concerne également un composé de formule (I) dans une méthode de traitement des pathologies ci-dessus indiquées qui comprend l'administration, à un patient, d'une dose efficace d'un composé selon l'invention, ou hydrates ou solvats.

## Revendications

1. Composé répondant à la formule (I) : 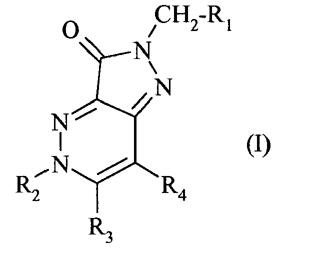 dans laquelle :
- R₁ représente :
. un (C₁-C₁₂)alkyle non substitué ou substitué une ou plusieurs fois par un atome de fluor ;
. un radical carbocyclique non aromatique en (C₃-C₁₂) non substitué ou substitué une ou plusieurs fois par des substituants choisis indépendamment parmi un (C₁-C₄)alkyle, un (C₁-C₄)alcoxy, un atome de fluor, un hydroxyle, un radical trifluorométhyle, un radical trifluorométhoxy, un (C₁-C₄)alkylthio ;
. un phényle non substitué ou substitué une ou plusieurs fois par des substituants choisis indépendamment parmi un atome d'halogène, un hydroxyle, un groupe Alk, un groupe OAlk, un méthylènedioxy, un groupe CH₂-NHAlk, un groupe -CH₂N(Alk)₂, un cyano, un nitro, un groupe S(O)ₙAlk, un groupe OS(O)ₙAlk, un groupe (C₁-C₄)alkylcarbonyle, un groupe (C₁-C₄)alcoxycarbonyle ; ou parmi un radical phényle, phénoxy, pyrrolyle, imidazolyle, pyridyle, pyrazolyle, oxazolyle, thiazolyle, triazolyle ou thiadiazolyle, ledit radical étant non substitué ou substitué une ou plusieurs fois par un (C₁-C₄)alkyle ;
. un benzyle non substitué ou substitué une ou plusieurs fois sur le phényle par des substituants choisis indépendamment parmi un atome d'halogène, un groupe Alk, un hydroxyle, un groupe OAlk, un méthylènedioxy, un groupe S(O)ₙAlk, un groupe OS(O)ₙAlk ;
. un phénéthyle non substitué ou substitué une ou plusieurs fois sur le phényle par des substituants choisis indépendamment parmi un atome d'halogène, un (C₁-C₄)alkyle, un (C₁-C₄)alcoxy, un radical trifluorométhyle, un radical trifluorométhoxy ;
. un benzhydryle ; un benzhydrylméthyle ;
. un radical hétérocyclique aromatique choisi parmi un pyrrolyle, un imidazolyle, un furyle, un thiényle, un pyrazolyle, un oxazolyle, un pyridyle, un indolyle, un benzothiényle, un thiéno[3,2-b]thiényle, ledit radical étant non substitué ou substitué une ou plusieurs fois par des substituants choisis indépendamment parmi un atome d'halogène, un groupe Alk, un groupe OAlk, un cyano, un nitro, un groupe S(O)ₙAlk ;
- R₂ représente un phényle non substitué ou substitué une ou plusieurs fois par des substituants choisis indépendamment parmi un atome d'halogène, un hydroxy, un groupe Alk, un groupe OAlk, un groupe S(O)ₙAlk, un groupe OS(O)ₙAlk, un groupe -O(CH₂)ₘR₅ ou un groupe -S(CH₂)ₘR₆ ;
- R₃ représente un phényle non substitué ou substitué une ou plusieurs fois par des substituants choisis indépendamment parmi un atome d'halogène, un hydroxy, un groupe Alk, un groupe OAlk, un groupe S(O)ₙAlk, un groupe OS(O)ₙAlk, un groupe -O(CH₂)ₘR₅ ou un groupe -S(CH₂)ₘR₆ ;
- R₄ représente un atome d'hydrogène, un (C₁-C₄)alkyle, un (C₁-C₄)alcoxy, un hydroxy ;
- R₅ représente un groupe -NR₇R₈ ou un groupe -SAlk ;
- R₆ représente un hydroxy, un groupe -NR₇R₈, un groupe NR₇COR₈ ou un groupe -NR₇SO₂R₉;
- R₇ représente un atome d'hydrogène ou un (C₁-C₄)alkyle ;
- R₈ représente un atome d'hydrogène, un groupe Alk, un (C₃-C₇)cycloalkyle ;
- R₉ représente un (C₁-C₄)alkyle ;
- m représente 2 ou 3 ;
- n représente 0, 1 ou 2 ;
- Alk représente un (C₁-C₄)alkyle non substitué ou substitué une ou plusieurs fois par un atome de fluor ;
ainsi que ses sels d'addition, ses hydrates ou ses solvats.

2. Composé de formule (IA) selon la revendication 1 dans laquelle R₁ représente :
. un (C₁-C₁₂)alkyle non substitué ou substitué une ou plusieurs fois par un atome de fluor ;
les substituants R₂, R₃ et R₄ sont tels que définis pour les composés de formule (I) à la revendication 1 ;
ainsi que ses sels d'addition, ses hydrates ou ses solvats.

3. Composé de formule (IB) selon la revendication 1 dans laquelle R₁ représente :
. un radical carbocyclique non aromatique en (C₃-C₁₂) non substitué ou substitué une ou plusieurs fois par des substituants choisis indépendamment parmi un (C₁-C₄)alkyle, un (C₁-C₄)alcoxy, un atome de fluor, un hydroxyle, un radical trifluorométhyle, un radical trifluorométhoxy, un (C₁-C₄)alkylthio ;
les substituants R₂, R₃ et R₄ sont tels que définis pour les composés de formule (I) à la revendication 1;
ainsi que ses sels d'addition, ses hydrates ou ses solvats.

4. Composés de formule (IC) selon la revendication 1 dans laquelle R₁ représente :
. un phényle non substitué ou substitué une ou plusieurs fois par des substituants choisis indépendamment parmi un atome d'halogène, un hydroxyle, un groupe Alk, un groupe OAlk, un méthylènedioxy, un groupe CH₂-NHAlk, un groupe -CH₂N(Alk)₂, un cyano, un nitro, un groupe S(O)ₙAlk, un groupe OS(O)ₙAlk, un groupe (C₁-C₄)alkylcarbonyle, un groupe (C₁-C₄)alcoxycarbonyle ; ou parmi un radical phényle, phénoxy, pyrrolyle, imidazolyle, pyridyle, pyrazolyle, oxazolyle, thiazolyle, triazolyle ou thiadiazolyle, ledit radical étant non substitué ou substitué une ou plusieurs fois par un (C₁-C₄)alkyle ;
les substituants R₂, R₃ et R₄ sont tels que définis pour les composés de formule (I) à la revendication 1;
ainsi que ses sels d'addition, ses hydrates ou ses solvats.

5. Composés de formule (ID) selon la revendication 1 dans laquelle R₁ représente :
. un benzyle non substitué ou substitué une ou plusieurs fois sur le phényle par des substituants choisis indépendamment parmi un atome d'halogène, un groupe Alk, un hydroxyle, un groupe OAlk, un méthylènedioxy, un groupe S(O)ₙAlk, un groupe OS(O)ₙAlk ;
les substituants R₂, R₃ et R₄ sont tels que définis pour les composés de formule (I) à la revendication 1;
ainsi que ses sels d'addition, ses hydrates ou ses solvats.

6. Composés de formule (IE) dans laquelle R₁ représente :
. un radical hétérocyclique aromatique choisi parmi un pyrrolyle, un imidazolyle, un furyle, un thiényle, un pyrazolyle, un oxazolyle, un pyridyle, un indolyle, un benzothiényle, un thiéno[3,2-b]thiényle, ledit radical étant non substitué ou substitué une ou plusieurs fois par des substituants choisis indépendamment parmi un atome d'halogène, un groupe Alk, un groupe OAlk, un cyano, un nitro, un groupe S(O)ₙAlk ;
les substituants R₂, R₃ et R₄ sont tels que définis pour les composés de formule (I) à la revendication 1;
ainsi que ses sels d'addition, ses hydrates ou ses solvats.

7. Composé de formule (I), selon la revendication 1 dans laquelle :
- R₁ représente :
. un (C₁-C₁₂)alkyle ;
. un (C₃-C₇)cycloalkyle non substitué ou substitué par un (C₁-C₄)alkyle, un radical trifluorométhyle ; un adamantyle ;
. un phényle non substitué ou substitué une ou plusieurs fois par des substituants choisis indépendamment parmi un atome d'halogène, un groupe Alk, un groupe OAlk, un groupe CH₂N(Alk)₂, un groupe -S(O)ₙAlk, un groupe (C₁-C₄)alcoxycarbonyle ; ou parmi un radical phényle, triazolyle, thiadiazolyle ;
. un benzyle non substitué ou substitué une ou plusieurs fois sur le phényle par des substituants choisi indépendamment parmi un atome d'halogène, un groupe Alk ;
. un radical hétérocyclique aromatique choisi parmi un pyridyle, un thiéno[3,2-b]thiényle, un benzothiényle, ledit radical étant non substitué ou substitué par un atome d'halogène, un radical trifluorométhyle ;
- R₂ représente un phényle mono- ou disubstitué par un atome d'halogène, un hydroxy, un groupe OAlk, un groupe S(O)ₙAlk, un groupe OS(O)ₙAlk, un groupe -O(CH₂)ₘR₅, un groupe -S(CH₂)ₘR₆ ;
- R₃ représente un phényle mono- ou disubstitué par un atome d'halogène, un groupe OAlk, un groupe S(O)ₙ Alk, un groupe -O(CH₂)ₘR₅, un groupe -S(CH₂)ₘR₆ ;
- R₄ représente un atome d'hydrogène, un (C₁-C₄)alkyle, un (C₁-C₄)alcoxy, un hydroxy ;
- n représente 0, 1 ou 2 ;
- Alk représente un (C₁-C₄)alkyle non substitué ou substitué une ou plusieurs fois par un atome de fluor ;
- les substituants m, R₅ et R₆ sont tels que définis pour les composés de formule (I) à la revendication 1 ;
ainsi que ses sels d'addition, ses hydrates ou ses solvats.

8. Composé de formule (I), selon la revendication 1 dans laquelle :
- R₁ représente :
. un 1-propylbutyle ;
. un cyclohexyle, un 4-*tert*-butylcyclohexyle, un 4-(trifluorométhyl)cyclohexyle ; un adamantan-1-yle ;
. un phényle, 4-fluorophényle, un 2-méthylphényle, un 4-méthylphényle, un 4-isopropylphényle, un 4-butylphényle, un 4-*tert*-butylphényle, un 4-(trifluorométhyl)phényle, un 4-méthoxyphényle, un 4-butoxyphényle, un 4-*tert-*butoxyphényle, un 3-(trifluorométhoxy)phényle, un 4-(trifluorométhoxy)phényle, un 4-(difluorométhoxy)phényle, un 4-(1,1,2,2-tétrafluoroéthoxy)phényle, un 4-(éthylthio)phényle, un 3-[(trifluorométhyl)thio]phényle, un 4-[(trifluorométhyl)thio]phényle, un 4-[(2,2,2-trifluoroéthyl)thio]phényle, un 4-(méthylsulfonyl)phényl, un 4-[[éthyl(propyl)amino]méthyl]phényle, un 4-[[méthyl(2,2,2-trifluoroéthyl)amino]méthyl]phényle, un 3-chloro-4-(trifluorométhyl)phényle, un 2-fluoro-4-(trifluorométhyl)phényle, un 3-fluoro-4-(trifluorométhyl)phényle, un 3-fluoro-4-propoxyphényle, un 3-chloro-4-(trifluorométhoxy)phényle, un 3,5-bis(trifluorométhyl)phényle, un 4-(méthoxycarbonyl)phényle, un biphényl-4-yle, un 4-(1*H*-1,2,4-triazol-1-yl)phényle, un 4-(1,2,3-thiadiazol-4-yl)phényle ;
. un benzyle , un [3,5-difluoro-4-(trifluorométhyl)phényl]méthyle ;
. un pyridin-4-yle, un 6-(trifluorométhyl)pyridin-3-yle, un thiéno[3,2-b]thién-2-yle, un 5-chloro-1-benzothién-2-yle ;
- R₂ représente un 4-bromophényle, un 4-chlorophényle, un 4-fluorophényle, un 4-méthoxyphényle, un 4-(méthylthio)phényle, un 4-hydroxyphényle, un 4-[[(3,3,3-trifluoropropyl)sulfonyl]oxy]phényle, un 4-[(propylsulfonyl)oxy]phényle, un 2,4-dichlorophényle, un 4-(trifluorométhoxy)phényle, un 4-[(trifluorométhyl)thio]phényle, un 4-[2-(diméthylamino)éthoxy]phényle, un 4-[(3-hydroxypropyl)thio]phényle, un 4-[(2-acétamidoéthyl)thio]phényle, un 4-[[3-[(méthylsulfonyl)amino]propyl]thio]phényle ;
- R₃ représente un 2-chlorophényle, un 4-chlorophényle, un 2,4-dichlorophényle, un 4-bromo-2-chlorophényle, un 2-chloro-4-fluorophényle, un 2-chloro-4-méthoxyphényle, un 2-chloro-4-(méthylthio)phényle, un 2-chloro-4-(éthylthio)phényle, un 2-chloro-4-[(3,3,3-trifluoropropyl)thio]phényle, un 2-chloro-4-(2,2,2-trifluoroéthoxy)phényle, un 2-chloro-4-[2-(diméthylamino)éthoxy]phényle, un 2-chloro-4-[2-(méthylthio)éthoxy]phényle, un 2-chloro-4-[(3-hydroxypropyl)thio]phényle, un 2-chloro-4-[(2-acétamidoéthyl)thio]phényle, un 2-chloro-4-[[3-[(méthylsulfonyl)amino]propyl]thio]phényle, un 4-[(2-aminoéthyl)thio]-2-chlorophényle, un 2-chloro-4-[[2-(diméthylamino)éthyl]thio]phényle, un 2-chloro-4-[[2-(diéthylamino)éthyl]thio]phényle, un 2-chloro-4-[[2-(isopropylamino)éthyl]thio]phényle, un 2-chloro-4-[(2-formamidoéthyl)thio]phényle, un 2-chloro-4-[[2-[(méthylsulfonyl)amino]éthyl]thio]phényle, un 2-chloro-4-[[2-[(trifluoroacétyl)amino]éthyl]thio]phényl, un 2-chloro-4-[[2-[(cyclopropylcarbonyl)amino]éthyl]thio]phényle ;
- R₄ représente un atome d'hydrogène, un méthyle, un méthoxy, un hydroxy ;
ainsi que ses sels d'addition, ses hydrates ou ses solvats.

9. Composé de formule (I), selon la revendication 1 dans laquelle :
- R₁ représente :
. un 4-isopropylphényle, un 4-*tert*-butylphényle, un 4-(trifluorométhyl)phényle, un 4-(trifluorométhoxy)phényle, un 4-[(trifluorométhyl)thio]phényle, un 2-fluoro-4-(trifluorométhyl)phényle, un 3-fluoro-4-(trifluorométhyl)phényle ;
- R₂ représente un 4-bromophényle, un 4-chlorophényle, un 4-fluorophényle, un 4-méthoxyphényle, un 4-(méthylthio)phényle, un 4-[[(3,3,3-trifluoropropyl)sulfonyl]oxy]phényle, un 4-[(propylsulfonyl)oxy]phényle, un 4-[[3-[(méthylsulfonyl)amino]propyl]thio]phényle ;
- R₃ représente un 2-chlorophényle, un 2,4-dichlorophényle, un 4-bromo-2-chlorophényle, un 2-chloro-4-méthoxyphényle, un 2-chloro-4-(méthylthio)phényle, un 2-chloro-4-[2-(diméthylamino)éthoxy]phényle, un 2-chloro-4-[2-(méthylthio)éthoxy]phényle, un 2-chloro-4-[(3-hydroxypropyl)thio]phényle, un 2-chloro-4-[(2-acétamidoéthyl)thio]phényle, un 4-[(2-aminoéthyl)thio]-2-chlorophényle, un 2-chloro-4-[[2-(diméthylamino)éthyl]thio]phényle, un 2-chloro-4-[[2-(diéthylamino)éthyl]thio]phényle, un 2-chloro-4-[[2-(isopropylamino)éthyl]thio]phényle, un 2-chloro-4-[[2-[(méthylsulfonyl)amino]éthyl]thio]phényle, un 2-chloro-4-[(2-formamidoéthyl)thio]phényle, un 2-chloro-4-[[2-[(trifluoroacétyl)amino]éthyl]thio]phényl, un 2-chloro-4-[[2-[(cyclopropylcarbonyl)amino]éthyl]thio]phényle ;
- R₄ représente un atome d'hydrogène ;
ainsi que ses sels d'addition, ses hydrates ou ses solvats.

10. Composé de formule (I), selon la revendication 1, choisi parmi :
- 5-(4-chlorophényl)-6-(2,4-dichlorophényl)-2-(4-isopropylbenzyl)-2,5-dihydro-3*H*-pyrazolo[4,3-c]pyridazin-3-one ;
- 2-(4-*tert*-butylbenzyl)-5-(4-chlorophényl)-6-(2,4-dichlorophényl)-2,5-dihydro-3*H*-pyrazolo[4,3-c]pyridazin-3-one ;
- 5-(4-chlorophényl)-6-(2,4-dichlorophényl)-2-[4-(trifluorométhyl)benzyl]-2,5-dihydro-3*H*-pyrazolo[4,3-c]pyridazin-3-one ;
- 5-(4-chlorophényl)-6-(2,4-dichlorophényl)-2-[2-fluoro-4-(trifluorométhyl)benzyl]-2,5-dihydro-3*H*-pyrazolo[4,3-c]pyridazin-3-one ;
- 5-(4-chlorophényl)-6-(2,4-dichlorophényl)-2-[4-[(trifluorométhyl)thio]benzyl]-2,5-dihydro-3*H*-pyrazolo[4,3-c]pyridazin-3-one ;
- 5-(4-bromophényl)-6-(2,4-dichlorophényl)-2-(4-isopropylbenzyl)-2,5-dihydro-3*H*-pyrazolo[4,3-c]pyridazin-3-one ;
- 5-(4-bromophényl)-2-(4-butylbenzyl)-6-(2,4-dichlorophényl)-2,5-dihydro-3*H-*pyrazolo[4,3-c]pyridazin-3-one;
- 5-(4-bromophényl)-6-(2,4-dichlorophényl)-2-[3-fluoro-4-(trifluorométhyl)benzyl]-2,5-dihydro-3*H*-pyrazolo[4,3-c]pyridazin-3-one ;
- 5-(4-bromophényl)-6-(2,4-dichlorophényl)-2-[4-(trifluorométhoxy)benzyl]-2,5-dihydro-3*H*-pyrazolo[4,3-c]pyridazin-3-one ;
- 5-(4-bromophényl)-6-(2,4-dichlorophényl)-2-[4-[(trifluorométhyl)thio]benzyl]-2,5-dihydro-3*H*-pyrazolo[4,3-c]pyridazin-3-one ;
- 6-(2,4-dichlorophényl)-5-[4-(méthylthio)phényl]-2-[4-(trifluorométhyl)benzyl]-2,5-dihydro-3*H*-pyrazolo[4,3-c]pyridazin-3-one ;
- 6-(2,4-dichlorophényl)-2-[2-fluoro-4-(trifluorométhyl)benzyl]-5-[4-(méthylthio)phényl]-2,5-dihydro-3*H*-pyrazolo[4,3-c]pyridazin-3-one ;
- 6-(2,4-dichlorophényl)-5-[4-(méthylthio)phényl]-2-[4-(trifluorométhoxy)benzyl]-2,5-dihydro-3*H*-pyrazolo[4,3-c]pyridazin-3-one ;
- 6-(2-chlorophényl)-5-(4-chlorophényl)-2-[4-[(trifluorométhyl)thio]benzyl]-2,5-dihydro-3*H*-pyrazolo[4,3-c]pyridazin-3-one ;
- 6-(2,4-dichlorophényl)-5-(4-méthoxyphényl)-2-[4-[(trifluorométhoxy)benzyl]-2,5-dihydro-3*H*-pyrazolo[4,3-c]pyridazin-3-one ;
- 6-(2,4-dichlorophényl)-5-(4-méthoxyphényl)-2-[4-[(trifluorométhyl)thio]benzyl]-2,5-dihydro-3*H*-pyrazolo[4,3-c]pyridazin-3-one ;
- 6-(2-chlorophényl)-5-(4-fluorophényl)-2-[4-[(trifluorométhyl)thio]benzyl]-2,5-dihydro-3*H*-pyrazolo[4,3-c]pyridazin-3-one ;
- 4-[6-(2,4-dichlorophényl)-3-oxo-2-[4-[(trifluorométhyl)thio] benzyl]-2,3-dihydro-5*H*-pyrazolo[4,3-c]pyridazin-5-yl]phényl-3,3,3-trifluoropropane-1-sulfonate ;
- 4-[6-(2,4-dichlorophényl)-3-oxo-2-[4-[(trifluorométhyl)thio] benzyl]-2,3-dihydro-5*H*-pyrazolo[4,3-c]pyridazin-5-yl]phénylpropane-1-sulfonate ;
- 6-(4-bromo-2-chlorophényl)-5-[4-(méthylthio)phényl]-2-[4-(trifluorométhyl)benzyl]-2,5-dihydro-3*H*-pyrazolo[4,3-*c*]pyridazin-3-one ;
- 6-{2-chloro-4-[(3-hydroxypropyl)thio]phényl}-5-[4-(méthylthio)phényl]-2-[4-(trifluorométhyl)benzyl]-2,5-dihydro-3*H*-pyrazolo[4,3-*c*]pyridazin-3-one ;
- 6-(4-bromo-2-chlorophényl)-5-[4-(méthylthio)phényl]-2-{4-[(trifluorométhyl)thio]benzyl}-2,5-dihydro-3*H*-pyrazolo[4,3-*c*]pyridazin-3-one ;
- *N*-(2-{[3-chloro-4-(5-[4-(méthylthio)phényl]-3-oxo-2-{4-[(trifluorométhyl)thio]benzyl}-3,5-dihydro-2*H*-pyrazolo[4,3-*c*]pyridazin-6-yl)phényl]thio}éthyl)acetamide ;
- 6-{2-chloro-4-[(3-hydroxypropyl)thio]phényl}-5-[4-(méthylthio)phényl]-2-{4-[(trifluorométhyl)thio]benzyl}-2,5-dihydro-3*H*-pyrazolo[4,3-*c*]pyridazin-3-one ;
- 6-(2-chloro-4-méthoxyphényl)-5-[4-(méthylthio)phényl]-2-[4-(trifluorométhyl)benzyl]-2,5-dihydro-3*H*-pyrazolo[4,3-*c*]pyridazin-3-one ;
- 6-(2-chloro-4-méthoxyphényl)-5-[4-(méthylthio)phényl]-2-[4-(trifluorométhoxy)benzyl]-2,5-dihydro-3*H*-pyrazolo[4,3-*c*]pyridazin-3-one ;
- 6-(2-chloro-4-méthoxyphényl)-5-[4-(méthylthio)phényl]-2- {4-[(trifluorométhyl)thio]benzyl}-2,5-dihydro-3*H*-pyrazolo[4,3-*c*]pyridazin-3-one ;
- 6-{2-chloro-4-[2-(diméthylamino)éthoxy]phényl}-5-[4-(méthylthio)phényl]-2-[4-(trifluorométhyl)benzyl]-2,5-dihydro-3*H*-pyrazolo[4,3-*c*]pyridazin-3-one ;
- 6-{2-chloro-4-[2-(méthylthio)éthoxy]phényl}-5-[4-(méthylthio)phényl]-2-[4-(trifluorométhyl)benzyl]-2,5-dihydro-3*H*-pyrazolo[4,3-*c*]pyridazin-3-one ;
- 6-[2-chloro-4-(méthylthio)phényl]-5-[4-(méthylthio)phényl]-2-[4-(trifluorométhyl)benzyl]-2,5-dihydro-3*H*-pyrazolo[4,3-*c*]pyridazin-3-one ;
- 6-[2-chloro-4-(méthylthio)phényl]-5-[4-(méthylthio)phényl]-2-{4-[(trifluorométhyl)thio]benzyl}-2,5-dihydro-3*H*-pyrazolo[4,3-*c*]pyridazin-3-one ;
- *N*-[3-({4-[6-(2,4-dichlorophényl)-3-oxo-2-{4-[(trifluorométhyl)thio]benzyl}-2,3-dihydro-5*H*-pyrazolo[4,3-*c*]pyridazin-5-yl]phényl}thio)propyl]méthanesulfonamide ;
- *N*-{3-[(4-{6-(2,4-dichlorophényl)-3-oxo-2-[4-(trifluorométhyl)benzyl]-2,3-dihydro-5*H*-pyrazolo[4,3-*c*]pyridazin-5-yl}phényl)thio]propyl}méthanesulfonamide ;
- 6-{4-[(2-aminoéthyl)thio]-2-chlorophényl}-5-[4-(méthylthio)phényl]-2-[4-(trifluorométhyl)benzyl]-2,5-dihydro-3*H*-pyrazolo[4,3-*c*]pyridazin-3-one ;
- 6-(2-chloro-4-{[2-(diméthylamino)éthyl]thio}phényl)-5-[4-(méthylthio)phényl]-2-[4-(trifluorométhyl)benzyl]-2,5-dihydro-3*H*-pyrazolo[4,3-*c*]pyridazin-3-one ;
- 6-(2-chloro-4-{[2-(diéthylamino)éthyl]thio}phényl)-5-[4-(méthylthio)phényl]-2-[4-(trifluorométhyl)benzyl]-2,5-dihydro-3*H*-pyrazolo[4,3-*c*]pyridazin-3-one ;
- 6-(2-chloro-4-{[2-(isopropylamino)éthyl]thio}phényl)-5-[4-(méthylthio)phényl]-2-[4-(trifluorométhyl)benzyl]-2,5-dihydro-3*H*-pyrazolo[4,3-*c*]pyridazin-3-one ;
- *N*-{2-[(3-chloro-4-{5-[4-(méthylthio)phényl]-3-oxo-2-[4-(trifluorométhyl)benzyl]-3,5-dihydro-2*H*-pyrazolo[4,3-*c*]pyridazin-6-yl}phényl)thio]éthyl}méthanesulfonamide ;
- *N*-{2-[(3-chloro-4-{5-[4-(méthylthio)phényl]-3-oxo-2-[4-(trifluorométhyl)benzyl]-3,5-dihydro-2*H*-pyrazolo[4,3-*c*]pyridazin-6-yl}phényl)thio]éthyl}formamide ;
- *N*-{2-[(3-chloro-4-{5-[4-(methylthio)phenyl]-3-oxo-2-[4-(trifluoromethyl)benzyl]-3,5-dihydro-2*H*-pyrazolo[4,3-*c*]pyridazin-6-yl}phenyl)thio]ethyl}-2,2,2-trifluoroacetamide ;
- *N*-{2-[(3-chloro-4-{5-[4-(methylthio)phenyl]-3-oxo-2-[4-(trifluoromethyl)benzyl]-3,5-dihydro-2*H*-pyrazolo[4,3-*c*]pyridazin-6-yl}phenyl)thio]ethyl}cyclopropanecarboxamide ;
ainsi que ses sels d'addition, ses hydrates ou ses solvats.

11. Procédé de préparation des composés de formule (I) selon la revendication 1, **caractérisé en ce que** :
on fait réagir, en présence d'une base, un composé de formule : 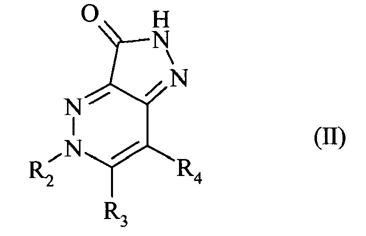 dans laquelle R₂, R₃ et R₄ sont tels que définis pour un composé de formule (I) à la revendication 1 avec un composé de formule :
Y-CH₂-R₁ (III)
dans laquelle R₁ est tel que défini pour un composé de formule (I) à la revendication 1 et Y représente un groupe partant choisi parmi un atome d'halogène ou un groupe hydroxy activé choisi parmi un groupe méthanesulfonate, benzènesulfonate, p-toluènesulfonate ou triflate.

12. Composés de formule : 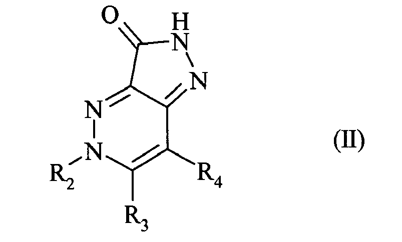 dans laquelle :
- R₂ représente un phényle non substitué ou substitué une ou plusieurs fois par des substituants choisis indépendamment parmi un atome d'halogène, un hydroxy, un groupe Alk, un groupe OAlk, un groupe S(O)ₙAlk ou un groupe OS(O)ₙAlk ;
- R₃ représente un phényle non substitué ou substitué une ou plusieurs fois par des substituants choisis indépendamment parmi un atome d'halogène, un hydroxy, un groupe Alk, un groupe OAlk, un groupe S(O)ₙAlk ou un groupe OS(O)ₙAlk ;
- R₄ représente un atome d'hydrogène ou un (C₁-C₄)alkyle, un (C₁-C₄)alcoxy, un hydroxy ;
- n représente 0, 1 ou 2 ;
- Alk représente un (C₁-C₄)alkyle non substitué ou substitué une ou plusieurs fois par un atome de fluor.

13. Médicament, **caractérisé en ce qu'**il comprend un composé de formule (I) selon l'une quelconque des revendications 1 à 10, ou un sel d'addition de ce composé à un acide pharmaceutiquement acceptable, ou encore un hydrate ou un solvat du composé de formule (I).

14. Composition pharmaceutique, **caractérisée en ce qu'**elle comprend un composé de formule (I) selon l'une quelconque des revendications 1 à 10, ou un sel pharmaceutiquement acceptable, un hydrate ou un solvat de ce composé, ainsi qu'au moins un excipient pharmaceutiquement acceptable.

15. Utilisation d'un composé de formule (I) selon l'une quelconque des revendications 1 à 10 pour la préparation d'un médicament pour le traitement ou la prévention des maladies dans lesquelles les récepteurs CB₁ sont impliqués.

16. Utilisation selon la revendication 15 **caractérisée en ce que** les maladies sont les désordres psychiatriques, la dépendance et le sevrage à une substance, les troubles cognitifs, les troubles de l'attention et de la vigilance, et les maladies neurodégénératives aiguës et chroniques.

17. Utilisation selon la revendication 15 **caractérisée en ce que** les maladies sont les troubles du métabolisme, les troubles de l'appétance, les troubles de l'appétit, l'obésité, le diabète de type II, le syndrome métabolique, la dyslipidémie.

18. Utilisation selon la revendication 15 **caractérisée en ce que** les maladies sont la douleur, la douleur neuropathique, la douleur induite par le traitement anticancéreux.

19. Utilisation selon la revendication 15 **caractérisée en ce que** les maladies sont les troubles gastro-intestinaux, les vomissements, les troubles diarrhéiques, l'ulcère, les maladies du foie.

20. Utilisation selon la revendication 15 **caractérisée en ce que** les maladies sont les maladies du système immunitaire, arthrite rhymatoïde, la démyélinisation, la sclérose en plaque, les maladies inflammatoires.

21. Utilisation selon la revendication 15 **caractérisée en ce que** les maladies sont les maladies d'Alzheimer, de Parkinson, la schizophrénie, les troubles cognitifs, le diabète, l'obésité, le syndrome métabolique et le sevrage tabagique.

## Claims

1. Compound corresponding to the formula (I) : 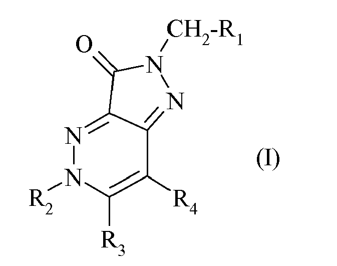 in which:
- R₁ is:
. a (C₁-C₁₂)alkyl which is unsubstituted or substituted one or more times with a fluorine atom;
. a non-aromatic (C₃-C₁₂) carbocyclic radical which is unsubstituted or substituted one or more times with substituents selected independently from a (C₁-C₄) alkyl, a (C₁-C₄)alkoxy, a fluorine atom, a hydroxyl, trifluoromethyl radical, a trifluoromethoxy radical and a (C₁-C₄)alkylthio;
. a phenyl which is unsubstituted or substituted one or more times with substituents selected independently from a halogen atom, a hydroxyl, an Alk group, an OAlk group, a methylenedioxy, a CH₂-NHAlk group, a -CH₂N(Alk)₂ group, a cyano, a nitro, an S(O)ₙAlk group, an OS(O)ₙAlk group, a (C₁-C₄)alkylcarbonyl group and a (C₁-C₄)alkoxycarbonyl group; or from a phenyl, phenoxy, pyrrolyl, imidazolyl, pyridyl, pyrazolyl, oxazolyl, thiazolyl, triazolyl or thiadiazolyl radical, said radical being unsubstituted or substituted one or more times with a (C₁-C₄)alkyl;
. a benzyl which is unsubstituted or substituted one or more times on the phenyl with substituents selected independently from a halogen atom, an Alk group, a hydroxyl, an OAlk group, a methylenedioxy, an S(O)ₙAlk group and an OS(O)ₙAlk group;
. a phenethyl which is unsubstituted or substituted one or more times on the phenyl with substituents selected independently from a halogen atom, a (C₁-C₄)alkyl, a (C₁-C₄)alkoxy, a trifluoromethyl radical and a trifluoromethoxy radical;
. a benzhydryl; a benzhydrylmethyl;
. an aromatic heterocyclic radical selected from a pyrrolyl, an imidazolyl, a furyl, a thienyl, a pyrazolyl, an oxazolyl, a pyridyl, an indolyl, a benzothienyl and a thieno[3,2-b]thienyl, said radical being unsubstituted or substituted one or more times with substituents selected independently from a halogen atom, an Alk group, an OAlk group, a cyano, a nitro and an S(O)ₙAlk group;
- R₂ is a phenyl which is unsubstituted or substituted one or more times with substituents selected independently from a halogen atom, a hydroxyl, an Alk group, an OAlk group, an S(O)ₙAlk group, an OS(O)ₙAlk group, an -O(CH₂)ₘR₅ group or an -S(CH₂)ₘR₆ group;
- R₃ is a phenyl which is unsubstituted or substituted one or more times with substituents selected independently from a halogen atom, a hydroxyl, an Alk group, an OAlk group, an S(O)ₙAlk group, an OS(O)ₙAlk group, an -O(CH₂)ₘR₅ group or an -S(CH₂)ₘR₆ group;
- R₄ is a hydrogen atom, a (C₁-C₄)alkyl, a (C₁-C₄)alkoxy or a hydroxyl;
- R₅ is an -NR₇R₈ group or an -SAlk group;
- R₆ is a hydroxyl, an -NR₇R₈ group, an NR₇COR₈ group or an -NR₇SO₂R₉ group;
- R₇ is a hydrogen atom or a (C₁-C₄)alkyl;
- R₈ is a hydrogen atom, an Alk group or a (C₃-C₇)cycloalkyl;
- R₉ is a (C₁-C₄)alkyl;
- m is 2 or 3;
- n is 0, 1 or 2;
- Alk is a (C₁-C₄)alkyl which is unsubstituted or substituted one or more times with a fluorine atom;
and also addition salts thereof, hydrates thereof or solvates thereof.

2. Compound of formula (IA) according to Claim 1, in which R₁ is:
. a (C₁-C₁₂)alkyl which is unsubstituted or substituted one or more times with a fluorine atom;
the substituents R₂, R₃ and R₄ are as defined for the compounds of formula (I) in Claim 1;
and also addition salts thereof, hydrates thereof or solvates thereof.

3. Compound of formula (IB) according to Claim 1, in which R₁ is:
. a non-aromatic (C₃-C₁₂) carbocyclic radical which is unsubstituted or substituted one or more times with substituents selected independently from a (C₁-C₄)alkyl, a (C₁-C₄)alkoxy, a fluorine atom, a hydroxyl, a trifluoromethyl radical, a trifluoromethoxy radical and a (C₁-C₄)alkylthio; the substituents R₂, R₃ and R₄ are as defined for the compounds of formula (I) in Claim 1;
and also addition salts thereof, hydrates thereof or solvates thereof.

4. Compounds of formula (IC) according to Claim 1, in which R₁ is:
. a phenyl which is unsubstituted or substituted one or more times with substituents selected independently from a halogen atom, a hydroxyl, an Alk group, an OAlk group, a methylenedioxy, a CH₂-NHAlk group, a -CH₂N(Alk)₂ group, a cyano, a nitro, an S(O)ₙAlk group, an OS(O)ₙAlk group, a (C₁-C₄)alkylcarbonyl group and a (C₁-C₄)alkoxycarbonyl group; or from a phenyl, phenoxy, pyrrolyl, imidazolyl, pyridyl, pyrazolyl, oxazolyl, thiazolyl, triazolyl or thiadiazolyl radical, said radical being unsubstituted or substituted one or more times with a (C₁-C₄)alkyl;
the substituents R₂, R₃ and R₄ are as defined for the compounds of formula (I) in Claim 1;
and also addition salts thereof, hydrates thereof or solvates thereof.

5. Compounds of formula (ID) according to Claim 1, in which R₁ is:
. a benzyl which is unsubstituted or substituted one or more times on the phenyl with substituents selected independently from a halogen atom, an Alk group, a hydroxyl, an OAlk group, a methylenedioxy, an S(O)ₙAlk group and an OS(O)ₙAlk group;
the substituents R₂, R₃ and R₄ are as defined for the compounds of formula (I) in Claim 1;
and also addition salts thereof, hydrates thereof or solvates thereof.

6. Compounds of formula (IE), in which R₁ is:
. an aromatic heterocyclic radical selected from a pyrrolyl, an imidazolyl, a furyl, a thienyl, a pyrazolyl, an oxazolyl, a pyridyl, an indolyl, a benzothienyl and a thieno[3,2-b]thienyl, said radical being unsubstituted or substituted one or more times with substituents selected independently from a halogen atom, an Alk group, an OAlk group, a cyano, a nitro and an S(O)ₙAlk group;
the substituents R₂, R₃ and R₄ are as defined for the compounds of formula (I) in Claim 1;
and also addition salts thereof, hydrates thereof or solvates thereof.

7. Compound of formula (I) according to Claim 1, in which:
- R₁ is:
. a (C₁-C₁₂)alkyl;
. a (C₃-C₇)cycloalkyl which is unsubstituted or substituted with (C₁-C₄)alkyl, a trifluoromethyl radical; an adamantyl;
. a phenyl which is unsubstituted or substituted one or more times with substituents selected independently from a halogen atom, an Alk group, an OAlk group, a CH₂N(Alk)₂ group, an -S(O)ₙAlk group, a (C₁-C₄)alkoxycarbonyl group; or from a phenyl, triazolyl or thiadiazolyl radical;
. a benzyl which is unsubstituted or substituted one or more times on the phenyl with substituents selected independently from a halogen atom and an Alk group;
. an aromatic heterocyclic radical selected from a pyridyl, a thieno[3,2-b]thienyl and a benzothienyl, said radical being unsubstituted or substituted with a halogen atom or a trifluoromethyl radical;
- R₂ is a phenyl mono- or disubstituted with a halogen atom, a hydroxyl, an OAlk group, an S(O)ₙAlk group, an OS(O)ₙAlk group, an -O(CH₂)ₘR₅ group or an -S (CH₂)ₘR₆ group;
- R₃ is a phenyl mono- or disubstituted with a halogen atom, an OAlk group, an S(O)ₙAlk group, an - O(CH₂)ₘR₅ group or an -S(CH₂)ₘR₆ group;
- R₄ is a hydrogen atom, a (C₁-C₄)alkyl, a (C₁-C₄)alkoxy or a hydroxyl;
- n is 0, 1 or 2;
- Alk is a (C₁-C₄)alkyl which is unsubstituted or substituted one or more times with a fluorine atom;
- the substituents m, R₅ and R₆ are as defined for the compounds of formula (I) in Claim 1;
and also the addition salts thereof, hydrates thereof or solvates thereof.

8. Compound of formula (I) according to Claim 1, in which:
- R₁ is:
. a 1-propylbutyl;
. a cyclohexyl, a 4-*tert*-butylcyclohexyl, a 4-(trifluoromethyl)cyclohexyl; an adamantan-1-yl;
. a phenyl, 4-fluorophenyl, a 2-methylphenyl, a 4-methylphenyl, a 4-isopropylphenyl, a 4-butylphenyl, a 4-*tert*-butylphenyl, a 4-(trifluoromethyl)phenyl, a 4-methoxyphenyl, a 4-butoxyphenyl, a 4-tert-butoxyphenyl, a 3-(trifluoromethoxy)phenyl, a 4-(trifluoromethoxy)phenyl, a 4-(difluoromethoxy)phenyl, a 4-(1,1,2,2-tetrafluoroethoxy)phenyl, a 4-(ethylthio)phenyl, a 3-[(trifluoromethyl)thio]phenyl, a 4-[(trifluoromethyl)thio]phenyl, a 4-[(2,2,2-trifluoroethyl)thio]phenyl, a 4-(methylsulphonyl)phenyl, a 4-[[ethyl(propyl)amino]methyl]phenyl, a 4-[[methyl(2,2,2-trifluoroethyl)amino]methyl]phenyl, a 3-chloro-4-(trifluoromethyl)phenyl, a 2-fluoro-4-(trifluoromethyl)phenyl, a 3-fluoro-4-(trifluoromethyl)phenyl, a 3-fluoro-4-propoxyphenyl, a 3-chloro-4-(trifluoromethoxy)phenyl, a 3,5-bis(trifluoromethyl)phenyl, a 4-(methoxycarbonyl)phenyl, a biphenyl-4-yl, a 4-(1*H*-1,2,4-triazol-1-yl)phenyl, a 4-(1,2,3-thiadiazol-4-yl)phenyl;
. a benzyl, a [3,5-difluoro-4-(trifluoromethyl)phenyl]methyl;
. a pyridin-4-yl, a 6-(trifluoromethyl)pyridin-3-yl, a thieno[3,2-b]thien-2-yl, a 5-chloro-1-benzothien-2-yl;
- R₂ is a 4-bromophenyl, a 4-chlorophenyl, a 4-fluorophenyl, a 4-methoxyphenyl, a 4-(methylthio)phenyl, a 4-hydroxyphenyl, a 4-[[(3,3,3-trifluoropropyl)sulphonyl]oxy]phenyl, a 4-[(propylsulphonyl)oxy]phenyl, a 2,4-dichlorophenyl, a 4-(trifluoromethoxy)phenyl, a 4-[(trifluoromethyl)thio]phenyl, a 4-[2-(dimethylamino)ethoxy]phenyl, a 4-[(3-hydroxypropyl)thio]phenyl, a 4-[(2-acetamidoethyl)thio]phenyl or a 4-[[3-[(methylsulphonyl)amino]propyl]thio]phenyl;
- R₃ is a 2-chlorophenyl, a 4-chlorophenyl, a 2,4-dichlorophenyl, a 4-bromo-2-chlorophenyl, a 2-chloro-4-fluorophenyl, a 2-chloro-4-methoxyphenyl, a 2-chloro-4-(methylthio)phenyl, a 2-chloro-4-(ethylthio)phenyl, a 2-chloro-4-[(3,3,3-trifluoropropyl)thio]phenyl, a 2-chloro-4-(2,2,2-trifluoroethoxy)phenyl, a 2-chloro-4-[2-(dimethylamino)ethoxy]phenyl, a 2-chloro-4-[2-(methylthio)ethoxy]phenyl, a 2-chloro-4-[(3-hydroxypropyl)thio]phenyl, a 2-chloro-4-[(2-acetamidoethyl)thio]phenyl, a 2-chloro-4-[[3-[(methylsulphonyl)amino]propyl]thio]phenyl, a 4-[(2-aminoethyl)thio]-2-chlorophenyl, a 2-chloro-4-[[2-(dimethylamino)ethyl]thio]phenyl, a 2-chloro-4-[[2-(diethylamino)ethyl]thio]phenyl, a 2-chloro-4-[[2-(isopropylamino)ethyl]thio]phenyl, a 2-chloro-4-[(2-formamidoethyl)thio]phenyl, a 2-chloro-4-[[2-[(methylsulphonyl)amino]ethyl]thio]phenyl, a 2-chloro-4-[[2-[(trifluoroacetyl)amino]ethyl]thio]phenyl or a 2-chloro-4-[[2-[(cyclopropylcarbonyl)amino]ethyl]thio]phenyl;
- R₄ is a hydrogen atom, a methyl, a methoxy or a hydroxyl;
and also the addition salts thereof, hydrates thereof or solvates thereof.

9. Compound of formula (I) according to Claim 1, in which:
- R₁ is:
. a 4-isopropylphenyl, a 4-*tert*-butylphenyl, a 4-(trifluoromethyl)phenyl, a 4-(trifluoromethoxy)phenyl, a 4-[(trifluoromethyl)thio]phenyl, a 2-fluoro-4-(trifluoromethyl)phenyl or a 3-fluoro-4-(trifluoromethyl)phenyl;
- R₂ is a 4-bromophenyl, a 4-chlorophenyl, a 4-fluorophenyl, a 4-methoxyphenyl, a 4-(methylthio)phenyl, a 4-[[(3,3,3-trifluoropropyl)sulphonyl]oxy]phenyl, a 4-[(propylsulphonyl)oxy]phenyl or a 4-[[3-[(methylsulphonyl)amino]propyl]thio]phenyl;
- R₃ is a 2-chlorophenyl, a 2,4-dichlorophenyl, a 4-bromo-2-chlorophenyl, a 2-chloro-4-methoxyphenyl, a 2-chloro-4-(methylthio)phenyl, a 2-chloro-4-[2-(dimethylamino)ethoxy]phenyl, a 2-chloro-4-[2-(methylthio)ethoxy]phenyl, a 2-chloro-4-[(3-hydroxypropyl)thio]phenyl, a 2-chloro-4-[(2-acetamidoethyl)thio]phenyl, a 4-[(2-aminoethyl)thio]-2-chlorophenyl, a 2-chloro-4-[[2-(dimethylamino)ethyl]thio]phenyl, a 2-chloro-4-[[2-(diethylamino)ethyl]thio]phenyl, a 2-chloro-4-[[2-(isopropylamino)ethyl]thio]phenyl, a 2-chloro-4-[[2-[(methylsulphonyl)amino]ethyl]thio]phenyl, a 2-chloro-4-[(2-formamidoethyl)thio]phenyl, a 2-chloro-4-[[2-[(trifluoroacetyl)amino]ethyl]thio]phenyl or a 2-chloro-4-[[2-[(cyclopropylcarbonyl)amino]-ethyl]thio]phenyl;
- R₄ is a hydrogen atom;
and also the addition salts thereof, hydrates thereof or solvates thereof.

10. Compound of formula (I) according to Claim 1, selected from:
- 5-(4-chlorophenyl)-6-(2,4-dichlorophenyl)-2-(4-isopropylbenzyl)-2,5-dihydro-3*H*-pyrazolo[4,3-c]pyridazin-3-one;
- 2-(4-tert-butylbenzyl)-5-(4-chlorophenyl)-6-(2,4-dichlorophenyl)-2,5-dihydro-3*H*-pyrazolo[4,3-c]pyridazin-3-one;
- 5-(4-chlorophenyl)-6-(2,4-dichlorophenyl)-2-[4-(trifluoromethyl)benzyl]-2,5-dihydro-3*H-*pyrazolo[4,3-c]pyridazin-3-one;
- 5-(4-chlorophenyl)-6-(2,4-dichlorophenyl)-2-[2-fluoro-4-(trifluoromethyl)benzyl]-2,5-dihydro-3*H-*pyrazolo[4,3-c]pyridazin-3-one;
- 5-(4-chlorophenyl)-6-(2,4-dichlorophenyl)-2-[4-[(trifluoromethyl)thio]benzyl]-2,5-dihydro-3*H-*pyrazolo[4,3-c]pyridazin-3-one;
- 5-(4-bromophenyl)-6-(2,4-dichlorophenyl)-2-(4-isopropylbenzyl)-2,5-dihydro-3*H*-pyrazolo[4,3-c]pyridazin-3-one;
- 5-(4-bromophenyl)-2-(4-butylbenzyl)-6-(2,4-dichlorophenyl)-2,5-dihydro-3*H*-pyrazolo[4,3-c]pyridazin-3-one;
- 5-(4-bromophenyl)-6-(2,4-dichlorophenyl)-2-[3-fluoro-4-(trifluoromethyl)benzyl]-2,5-dihydro-3*H-*pyrazolo[4,3-c]pyridazin-3-one;
- 5-(4-bromophenyl)-6-(2,4-dichlorophenyl)-2-[4-(trifluoromethoxy)benzyl]-2,5-dihydro-3*H-*pyrazolo[4,3-c]pyridazin-3-one;
- 5-(4-bromophenyl)-6-(2,4-dichlorophenyl)-2-[4-[(trifluoromethyl)thio]benzyl]-2,5-dihydro-3*H-*pyrazolo[4,3-c]pyridazin-3-one;
- 6-(2,4-dichlorophenyl)-5-[4-(methylthio)phenyl]-2-[4-(trifluoromethyl)benzyl]-2,5-dihydro-3*H-*pyrazolo[4,3-c]pyridazin-3-one;
- 6-(2,4-dichlorophenyl)-2-[2-fluoro-4-(trifluoromethyl)benzyl]-5-[4-(methylthio)phenyl]-2,5-dihydro-3*H*-pyrazolo[4,3-c]pyridazin-3-one;
- 6-(2,4-dichlorophenyl)-5-[4-(methylthio)phenyl]-2-[4-(trifluoromethoxy)benzyl]-2,5-dihydro-3*H-*pyrazolo[4,3-c]pyridazin-3-one;
- 6-(2-chlorophenyl)-5-(4-chlorophenyl)-2-[4-[(trifluoromethyl)thio]benzyl]-2,5-dihydro-3*H-*pyrazolo[4,3-c]pyridazin-3-one;
- 6-(2,4-dichlorophenyl)-5-(4-methoxyphenyl)-2-[4-[(trifluoromethoxy)benzyl]-2,5-dihydro-3*H-*pyrazolo[4,3-c]pyridazin-3-one;
- 6-(2,4-dichlorophenyl)-5-(4-methoxyphenyl)-2-[4-[(trifluoromethyl)thio]benzyl]-2,5-dihydro-3*H-*pyrazolo[4,3-c]pyridazin-3-one;
- 6-(2-chlorophenyl)-5-(4-fluorophenyl)-2-[4-[(trifluoromethyl)thio]benzyl]-2,5-dihydro-3*H-*pyrazolo[4,3-c]pyridazin-3-one;
- 4-[6-(2,4-dichlorophenyl)-3-oxo-2-[4-[(trifluoromethyl)thio]benzyl]-2,3-dihydro-5*H-*pyrazolo[4,3-c]pyridazin-5-yl]phenyl-3,3,3-trifluoropropane-1-sulphonate;
- 4-[6-(2,4-dichlorophenyl)-3-oxo-2-[4-[(trifluoromethyl)thio]benzyl]-2,3-dihydro-5*H-*pyrazolo[4,3-c]pyridazin-5-yl]phenylpropane-1-sulphonate;
- 6-(4-bromo-2-chlorophenyl)-5-[4-(methylthio)phenyl]-2-[4-(trifluoromethyl)benzyl]-2,5-dihydro-3*H*-pyrazolo[4,3-*c*]pyridazin-3-one;
- 6-{2-chloro-4-[(3-hydroxypropyl)thio]phenyl}-5-[4-(methylthio)phenyl]-2-[4-(trifluoromethyl)benzyl]-2,5-dihydro-3*H*-pyrazolo[4,3-*c*]pyridazin-3-one;
- 6-(4-bromo-2-chlorophenyl)-5-[4-(methylthio)phenyl]-2-{4-[(trifluoromethyl)thio]benzyl}-2,5-dihydro-3*H-*pyrazolo[4,3-*c*]pyridazin-3-one;
- *N*-(2-{[3-chloro-4-(5-[4-(methylthio)phenyl]-3-oxo-2-{4-[(trifluoromethyl)thio]benzyl}-3,5-dihydro-2*H-*pyrazolo[4,3-*c*]pyridazin-6-yl)phenyl]thio}ethyl)acetamide;
- 6-{2-chloro-4-[(3-hydroxypropyl)thio]phenyl}-5-[4-(methylthio)phenyl]-2-{4-[(trifluoromethyl)thio]benzyl}-2,5-dihydro-3*H-*pyrazolo[4,3-*c*]pyridazin-3-one;
- 6-(2-chloro-4-methoxyphenyl)-5-[4-(methylthio)phenyl]-2-[4-(trifluoromethyl)benzyl]-2,5-dihydro-3*H*-pyrazolo[4,3-*c*]pyridazin-3-one;
- 6-(2-chloro-4-methoxyphenyl)-5-[4-(methylthio)phenyl]-2-[4-(trifluoromethoxy)benzyl]-2,5-dihydro-3*H*-pyrazolo[4,3-*c*]pyridazin-3-one;
- 6-(2-chloro-4-methoxyphenyl)-5-[4-(methylthio)phenyl]-2-{4-[(trifluoromethyl)thio]benzyl}-2,5-dihydro-3*H-*pyrazolo[4,3-*c*]pyridazin-3-one;
- 6-{2-chloro-4-[2-(dimethylamino)ethoxy]phenyl}-5-[4-(methylthio)phenyl]-2-[4-(trifluoromethyl)benzyl]-2,5-dihydro-3*H-*pyrazolo[4,3-*c*]pyridazin-3-one;
- 6-{2-chloro-4-[2-(methylthio)ethoxy]phenyl}-5-[4-(methylthio)phenyl]-2-[4-(trifluoromethyl)benzyl]-2,5-dihydro-3*H*-pyrazolo[4,3-*c*]pyridazin-3-one;
- 6-[2-chloro-4-(methylthio)phenyl]-5-[4-(methylthio)phenyl]-2-[4-(trifluoromethyl)benzyl]-2,5-dihydro-3*H*-pyrazolo[4,3-*c*]pyridazin-3-one;
- 6-[2-chloro-4-(methylthio)phenyl]-5-[4-(methylthio)phenyl]-2-{4-[(trifluoromethyl)thio]benzyl}-2,5-dihydro-3*H-*pyrazolo[4,3-*c*]pyridazin-3-one;
- *N*-[3-({4-[6-(2,4-dichlorophenyl)-3-oxo-2-{4-[(trifluoromethyl)thio]benzyl}-2,3-dihydro-5*H-*pyrazolo[4,3-*c*]pyridazin-5-yl]phenyl}thio)propyl]methanesulphonamide;
- *N*-{3-[(4-{6-(2,4-dichlorophenyl)-3-oxo-2-[4-(trifluoromethyl)benzyl]-2,3-dihydro-5*H-*pyrazolo[4,3-*c*]pyridazin-5-yl}phenyl)thio]propyl}methanesulphonamide;
- 6-{4-[(2-aminoethyl)thio]-2-chlorophenyl}-5-[4-(methylthio)phenyl]-2-[4-(trifluoromethyl)benzyl]-2,5-dihydro-3*H*-pyrazolo[4,3-*c*]pyridazin-3-one;
- 6-(2-chloro-4-{[2-(dimethylamino)ethyl]thio}phenyl)-5-[4-(methylthio)phenyl]-2-[4-(trifluoromethyl)benzyl]-2,5-dihydro-3*H*-pyrazolo[4,3-*c*]pyridazin-3-one;
- 6-(2-chloro-4-{[2-(diethylamino)ethyl]thio}phenyl-5-[4-(methylthio)phenyl]-2-[4-(trifluoromethyl)benzyl]-2,5-dihydro-3*H-*pyrazolo[4,3-*c*]pyridazin-3-one;
- 6-(2-chloro-4-{[2-(isopropylamino)ethyl]thio}phenyl)-5-[4-(methylthio)phenyl]-2-[4-(trifluoromethyl)benzyl]-2,5-dihydro-3*H*-pyrazolo[4,3-*c*]pyridazin-3-one;
- *N*-{2-[(3-chloro-4-{5-[4-(methylthio)phenyl]-3-oxo-2-[4-(trifluoromethyl)benzyl]-3,5-dihydro-2*H-*pyrazolo[4,3-*c*]pyridazin-6-yl}phenyl)thio]ethyl}methanesulphonamide;
- *N*-{2-[(3-chloro-4-{5-[4-(methylthio)phenyl]-3-oxo-2-[4-(trifluoromethyl)benzyl]-3,5-dihydro-2*H-*pyrazolo[4,3-*c*]pyridazin-6-yl}phenyl)thio]ethyl}formamide;
- *N*-{2-[(3-chloro-4-{5-[4-(methylthio)phenyl]-3-oxo-2-[4-(trifluoromethyl)benzyl]-3,5-dihydro-2*H-*pyrazolo[4,3-*c*]pyridazin-6-yl}phenyl)thio]ethyl}-2,2,2-trifluoroacetamide;
- *N*-{2-[(3-chloro-4-{5-[4-(methylthio)phenyl]-3-oxo-2-[4-(trifluoromethyl)benzyl]-3,5-dihydro-2*H-*pyrazolo[4,3-*c*]pyridazin-6-yl}phenyl)thio]ethyl}cyclopropanecarboxamide;
and also the addition salts thereof, hydrates thereof or solvates thereof.

11. Process for preparing the compounds of formula (I) according to Claim 1, **characterized in that**:
a compound of formula: 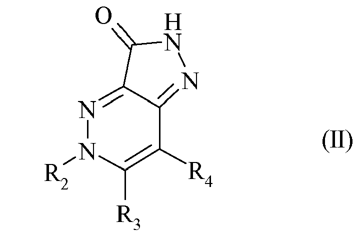 in which R₂, R₃ and R₄ are as defined for a compound of formula (I) in Claim 1, is reacted, in the presence of a base, with a compound of formula:
Y-CH₂-R₁ (III)
in which R₁ is as defined for a compound of formula (I) in Claim 1 and Y is a leaving group selected from a halogen atom or an activated hydroxyl group selected from a methanesulphonate, benzenesulphonate, p-toluenesulphonate or triflate group.

12. Compounds of formula: 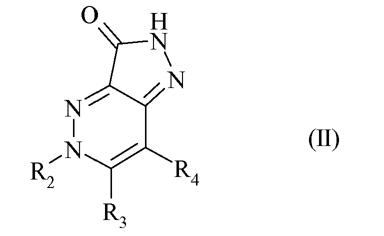 in which:
- R₂ is a phenyl which is unsubstituted or substituted one or more times with substituents selected independently from a halogen atom, a hydroxyl, an Alk group, an OAlk group, an S(O)ₙAlk group or an OS(O)ₙAlk group;
- R₃ is a phenyl which is unsubstituted or substituted one or more times with substituents selected independently from a halogen atom, a hydroxyl, an Alk group, an OAlk group, an S(O)ₙAlk group or an OS(O)ₙAlk group;
- R₄ is a hydrogen atom, a (C₁-C₄)alkyl, a (C₁-C₄)alkoxy or a hydroxyl;
- n is 0, 1 or 2;
- Alk is a (C₁-C₄)alkyl which is unsubstituted or substituted one or more times with a fluorine atom.

13. Medicament, **characterized in that** it comprises a compound of formula (I) according to any one of Claims 1 to 10, or an addition salt of this compound with a pharmaceutically acceptable acid, or else a hydrate or a solvate of the compound of formula (I).

14. Pharmaceutical composition, **characterized in that** it comprises a compound of formula (I) according to any one of Claims 1 to 10, or a pharmaceutically acceptable salt, a hydrate or a solvate of this compound, and also at least one pharmaceutically acceptable excipient.

15. Use of a compound of formula (I) according to any one of Claims 1 to 10, for the preparation of a medicament for the treatment or prevention of diseases in which CB₁ receptors are involved.

16. Use according to Claim 15, **characterized in that** the diseases are psychiatric disorders, dependency on and withdrawal from a substance, cognitive disorders, attention and consciousness disorders, and acute and chronic neurodegenerative diseases.

17. Use according to Claim 15, **characterized in that** the diseases are metabolic disorders, appetence disorders, appetite disorders, obesity, type II diabetes, metabolic syndrome and dyslipidemia.

18. Use according to Claim 15, **characterized in that** the diseases are pain, neuropathic pain, and pain caused by anticancer treatment.

19. Use according to Claim 15, **characterized in that** the diseases are gastrointestinal disorders, vomiting, diarrhoea disorders, ulcers, and liver diseases.

20. Use according to Claim 15, **characterized in that** the diseases are immune system diseases, rheumatoid arthritis, demyelinization, multiple sclerosis, and inflammatory diseases.

21. Use according to Claim 15, **characterized in that** the diseases are Alzheimer's disease, Parkinson's disease, schizophrenia, cognitive disorders, diabetes, obesity, metabolic syndrome and tobacco withdrawal.

## Patentansprüche

1. Verbindung der Formel (I): 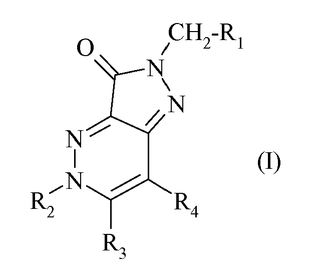 worin:
- R₁ für:
. (C₁-C₁₂)-Alkyl, das gegebenenfalls ein- oder mehrfach durch ein Fluoratom substituiert ist;
. einen nichtaromatischen carbocyclischen (C₃-C₁₂)-Rest, der gegebenenfalls ein- oder mehrfach durch Substituenten substituiert ist, die unabhängig voneinander unter (C₁-C₄)-Alkyl, (C₁-C₄)-Alkoxy, einem Fluoratom, Hydroxyl, einem Trifluormethylrest, einem Trifluormethoxyrest und (C₁-C₄)-Alkylthio ausgewählt sind;
. Phenyl, das gegebenenfalls ein- oder mehrfach durch Substituenten substituiert ist, die unabhängig voneinander unter einem Halogenatom, Hydroxyl, einer Alk-Gruppe, einer OAlk-Gruppe, Methylendioxy, einer CH₂-NHAlk-Gruppe, einer -CH₂N(Alk)₂-Gruppe, Cyano, Nitro, einer S(O)ₙAlk-Gruppe, einer OS(O)ₙAlk-Gruppe, einer (C₁-C₄)-Alkylcarbonylgruppe, einer (C₁-C₄)-Alkoxycarbonylgruppe oder einem Phenyl-, Phenoxy-, Pyrrolyl-, Imidazolyl-, Pyridyl-, Pyrazolyl-, Oxazolyl-, Thiazolyl-, Triazolyl- oder Thiadiazolylrest, wobei diese Reste gegebenenfalls ein- oder mehrfach durch (C₁-C₄)-Alkyl substituiert sind, ausgewählt sind;
. Benzyl, das gegebenenfalls am Phenyl ein- oder mehrfach durch Substituenten substituiert ist, die unabhängig voneinander unter einem Halogenatom, einer Alk-Gruppe, Hydroxyl, einer OAlk-Gruppe, Methylendioxy, einer S(O)ₙAlk-Gruppe und einer OS(O)ₙAlk-Gruppe ausgewählt sind;
. Phenethyl, das gegebenenfalls am Phenyl ein- oder mehrfach durch Substituenten substituiert ist, die unabhängig voneinander unter einem Halogenatom, (C₁-C₄)-Alkyl, (C₁-C₄)-Alkoxy, einem Trifluormethylrest und einem Trifluormethoxyrest ausgewählt sind;
. Benzhydryl; Benzhydrylmethyl;
. einen aromatischen heterocyclischen Rest, der unter Pyrrolyl, Imidazolyl, Furyl, Thienyl, Pyrazolyl, Oxazolyl, Pyridyl, Indolyl, Benzothienyl und Thieno[3,2-b]-thienyl ausgewählt ist, wobei der Rest gegebenenfalls ein- oder mehrfach durch Substituenten substituiert ist, die unabhängig voneinander unter einem Halogenatom, einer Alk-Gruppe, einer OAlk-Gruppe, Cyano, Nitro und einer S(O)ₙAlk-Gruppe ausgewählt sind;
steht;
- R₂ für Phenyl, das gegebenenfalls ein- oder mehrfach durch Substituenten substituiert ist, die unabhängig voneinander unter einem Halogenatom, Hydroxy, einer Alk-Gruppe, einer OAlk-Gruppe, einer S(O)ₙAlk-Gruppe, einer OS(O)ₙAlk-Gruppe, einer -O(CH₂)ₘR₅-Gruppe und einer -S(CH₂)ₘR₆-Gruppe ausgewählt sind, steht;
- R₃ für Phenyl, das gegebenenfalls ein- oder mehrfach durch Substituenten substituiert ist, die unabhängig voneinander unter einem Halogenatom, Hydroxy, einer Alk-Gruppe, einer OAlk-Gruppe, einer S(O)ₙAlk-Gruppe, einer OS(O)ₙAlk-Gruppe, einer -O(CH₂)ₘR₅-Gruppe und einer -S(CH₂)ₘR₆-Gruppe ausgewählt sind, steht;
- R₄ für ein Wasserstoffatom, (C₁-C₄)-Alkyl, (C₁-C₄)-Alkoxy oder Hydroxy steht;
- R₅ für eine -NR₇R₈-Gruppe oder eine -SAlk-Gruppe steht;
- R₆ für Hydroxy, eine -NR₇R₈-Gruppe, eine NR₇COR₈-Gruppe oder eine -NR₇SO₂R₉-Gruppe steht;
- R₇ für ein Wasserstoffatom oder (C₁-C₄)-Alkyl steht;
- R₈ für ein Wasserstoffatom, eine Alk-Gruppe oder (C₃-C₇)-Cycloalkyl steht;
- R₉ für (C₁-C₄)-Alkyl steht;
- m für 2 oder 3 steht;
- n für 0, 1 oder 2 steht;
- Alk für (C₁-C₄)-Alkyl, das gegebenenfalls ein- oder mehrfach durch ein Fluoratom substituiert ist, steht;
sowie Additionssalze davon, Hydrate davon oder Solvate davon.

2. Verbindung der Formel (IA) nach Anspruch 1, worin R₁ für:
. (C₁-C₁₂)-Alkyl, das gegebenenfalls ein- oder mehrfach durch ein Fluoratom substituiert ist; steht;
wobei die Substitutenten R₂, R₃ und R₄ die für die Verbindungen der Formel (I) in Anspruch 1 angegebene Bedeutung besitzen;
sowie Additionssalze davon, Hydrate davon oder Solvate davon.

3. Verbindung der Formel (IB) nach Anspruch 1, worin R₁ für:
. einen nichtaromatischen carbocyclischen (C₃-C₁₂)-Rest, der gegebenenfalls ein- oder mehrfach durch Substituenten substituiert ist, die unabhängig voneinander unter (C₁-C₄)-Alkyl, (C₁-C₄)-Alkoxy, einem Fluoratom, Hydroxyl, einem Trifluormethylrest, einem Trifluormethoxyrest und (C₁-C₄)-Alkylthio ausgewählt sind;
steht;
wobei die Substitutenten R₂, R₃ und R₄ die für die Verbindungen der Formel (I) in Anspruch 1 angegebene Bedeutung besitzen;
sowie Additionssalze davon, Hydrate davon oder Solvate davon.

4. Verbindungen der Formel (IC) nach Anspruch 1, worin R₁ für:
. Phenyl, das gegebenenfalls ein- oder mehrfach durch Substituenten substituiert ist, die unabhängig voneinander unter einem Halogenatom, Hydroxyl, einer Alk-Gruppe, einer OAlk-Gruppe, Methylendioxy, einer CH₂-NHAlk-Gruppe, einer -CH₂N(Alk)₂-Gruppe, Cyano, Nitro, einer S(O)ₙAlk-Gruppe, einer OS(O)ₙAlk-Gruppe, einer (C₁-C₄)-Alkylcarbonylgruppe, einer (C₁-C₄)-Alkoxycarbonylgruppe oder einem Phenyl-, Phenoxy-, Pyrrolyl-, Imidazolyl-, Pyridyl-, Pyrazolyl-, Oxazolyl-, Thiazolyl-, Triazolyl- oder Thiadiazolylrest, wobei diese Reste gegebenenfalls ein- oder mehrfach durch (C₁-C₄)-Alkyl substituiert sind, ausgewählt sind;
steht;
wobei die Substitutenten R₂, R₃ und R₄ die für die Verbindungen der Formel (I) in Anspruch 1 angegebene Bedeutung besitzen;
sowie Additionssalze davon, Hydrate davon oder Solvate davon.

5. Verbindungen der Formel (ID) nach Anspruch 1, worin R₁ für:
. Benzyl, das gegebenenfalls am Phenyl ein- oder mehrfach durch Substituenten substituiert ist, die unabhängig voneinander unter einem Halogenatom, einer Alk-Gruppe, Hydroxyl, einer OAlk-Gruppe, Methylendioxy, einer S(O)ₙAlk-Gruppe und einer OS(O)ₙAlk-Gruppe ausgewählt sind;
steht;
wobei die Substitutenten R₂, R₃ und R₄ die für die Verbindungen der Formel (I) in Anspruch 1 angegebene Bedeutung besitzen;
sowie Additionssalze davon, Hydrate davon oder Solvate davon.

6. Verbindungen der Formel (IE) nach Anspruch 1, worin R₁ für:
. einen aromatischen heterocyclischen Rest, der unter Pyrrolyl, Imidazolyl, Furyl, Thienyl, Pyrazolyl, Oxazolyl, Pyridyl, Indolyl, Benzothienyl und Thieno[3,2-b]thienyl ausgewählt ist, wobei der Rest gegebenenfalls ein- oder mehrfach durch Substituenten substituiert ist, die unabhängig voneinander unter einem Halogenatom, einer Alk-Gruppe, einer OAlk-Gruppe, Cyano, Nitro und einer S(O)ₙAlk-Gruppe ausgewählt sind;
steht;
wobei die Substitutenten R₂, R₃ und R₄ die für die Verbindungen der Formel (I) in Anspruch 1 angegebene Bedeutung besitzen;
sowie Additionssalze davon, Hydrate davon oder Solvate davon.

7. Verbindung der Formel (I) nach Anspruch 1, worin:
- R₁ für:
. (C₁-C₁₂)-Alkyl;
. (C₃-C₇)-Cycloalkyl, das gegebenenfalls durch (C₁-C₄)-Alkyl, einen Trifluormethylrest oder Adamantyl substituiert ist;
. Phenyl, das gegebenenfalls ein- oder mehrfach durch Substituenten substituiert ist, die unabhängig voneinander unter einem Halogenatom, einer Alk-Gruppe, einer OAlk-Gruppe, einer -CH₂N(Alk)₂-Gruppe, einer S(O)ₙAlk-Gruppe, einer (C₁-C₄)-Alkoxycarbonylgruppe oder einem Phenyl-, Triazolyl- oder Thiadiazolylrest ausgewählt sind;
. Benzyl, das gegebenenfalls am Phenyl ein- oder mehrfach durch Substituenten substituiert ist, die unabhängig voneinander unter einem Halogenatom und einer Alk-Gruppe ausgewählt sind;
. einen aromatischen heterocyclischen Rest, der unter Pyridyl, Thieno[3,2-b]thienyl und Benzothienyl ausgewählt ist, wobei der Rest gegebenenfalls durch ein Halogenatom oder einen Trifluormethylrest substitiuiert ist;
steht;
- R₂ für Phenyl, das ein- oder zweifach durch ein Halogenatom, Hydroxy, eine OAlk-Gruppe, eine S(O)ₙAlk-Gruppe, eine OS(O)ₙAlk-Gruppe, eine -O(CH₂)ₘR₅-Gruppe oder eine -S(CH₂)ₘR₆-Gruppe substituiert ist, steht;
- R₃ für Phenyl, das ein- oder zweifach durch ein Halogenatom, eine OAlk-Gruppe, eine S(O)ₙAlk-Gruppe, eine -O(CH₂)ₘR₅-Gruppe oder eine -S(CH₂)ₘR₆-Gruppe substituiert ist, steht;
- R₄ für ein Wasserstoffatom, (C₁-C₄)-Alkyl, (C₁-C₄)-Alkoxy oder Hydroxy steht;
- n für 0, 1 oder 2 steht;
- Alk für (C₁-C₄)-Alkyl, das gegebenenfalls ein- oder mehrfach durch ein Fluoratom substituiert ist, steht;
- die Substituenten m, R₅ und R₆ die für die Verbindungen der Formel (I) in Anspruch 1 angegebene Bedeutung besitzen;
sowie Additionssalze davon, Hydrate davon oder Solvate davon.

8. Verbindung der Formel (I) nach Anspruch 1, worin:
- R₁ für:
. 1-Propylbutyl;
. Cyclohexyl, 4-*tert*.-Butylcyclohexyl, 4-(Trifluormethyl)cyclohexyl; Adamantan-1-yl;
. Phenyl, 4-Fluorphenyl, 2-Methylphenyl, 4-Methylphenyl, 4-Isopropylphenyl, 4-Butylphenyl, 4-*tert*.-Butylphenyl, 4-(Trifluormethyl)phenyl, 4-Methoxyphenyl, 4-Butoxyphenyl, 4-tert.-Butoxyphenyl, 3-(Trifluormethoxy)phenyl, 4-(Trifluormethoxy)phenyl, 4-(Difluormethoxy)-phenyl, 4-(1,1,2,2-Tetrafluorethoxy)phenyl, 4-(Ethylthio)phenyl, 3-[(Trifluormethyl)thio]-phenyl, 4-[(Trifluormethyl)thio]phenyl, 4-[(2,2,2-Trifluorethyl)thio]phenyl, 4-(Methylsulfonyl)phenyl, 4-[[Ethyl(propyl)amino]-methyl]phenyl, 4-[[Methyl(2,2,2-trifluorethyl)-amino]methyl]phenyl, 3-Chlor-4-(trifluormethyl)phenyl, 2-Fluor-4-(trifluormethyl)-phenyl, 3-Fluor-4-(trifluormethyl)phenyl, 3-Fluor-4-propoxyphenyl, 3-Chlor-4-(trifluormethoxy)phenyl, 3,5-Bis(trifluormethyl)phenyl, 4-(Methoxycarbonyl)phenyl, Biphenyl-4-yl, 4-(1*H*-1,2,4-Triazol-1-yl)phenyl, 4-(1,2,3-Thiadiazol-4-yl)phenyl;
. Benzyl, [3,5-Difluor-4-(trifluormethyl)-phenyl]methyl;
. Pyridin-4-yl, 6-(Trifluormethyl)pyridin-3-yl, Thieno[3,2-b]thien-2-yl, 5-Chlor-1-benzothien-2-yl;
steht;
- R² für 4-Bromphenyl, 4-Chlorphenyl, 4-Fluorphenyl, 4-Methoxyphenyl, 4-(Methylthio)phenyl, 4-Hydroxyphenyl, 4-[[(3,3,3-Trifluorpropyl)-sulfonyl]oxy]phenyl, 4-[(Propylsulfonyl)oxy]-phenyl, 2,4-Dichlorphenyl, 4-(Trifluormethoxy)-phenyl, 4-[(Trifluormethyl)thio]phenyl, 4-[2-(Dimethylamino)ethoxy]phenyl, 4-[(3-Hydroxypropyl)thio]phenyl, 4-[(2-Acetamidoethyl)thio]-phenyl oder 4-[[3-[(Methylsulfonyl)amino]-propyl]thio]phenyl steht;
- R₃ für 2-Chlorphenyl, 4-Chlorphenyl, 2,4-Dichlorphenyl, 4-Brom-2-chlorphenyl, 2-Chlor-4-fluorphenyl, 2-Chlor-4-methoxyphenyl, 2-Chlor-4-(methylthio)phenyl, 2-Chlor-4-(ethylthio)-phenyl, 2-Chlor-4-[(3,3,3-trifluorpropyl)thio]-phenyl, 2-Chlor-4-(2,2,2-trifluorethoxy)phenyl, 2-Chlor-4-[2-(dimethylamino)ethoxy]phenyl, 2-Chlor-4-[2-(methylthio)ethoxy]phenyl, 2-Chlor-4-[(3-hydroxypropyl)thio]phenyl, 2-Chlor-4-[(2-acetamidoethyl)thio]phenyl, 2-Chlor-4-[[3-[(methylsulfonyl)amino]propyl]thio]phenyl, 4-[(2-Aminoethyl)thio]-2-chlorphenyl, 2-Chlor-4-[[2-(dimethylamino)ethyl]thio]phenyl, 2-Chlor-4-[[2-(diethylamino)ethyl]thio]phenyl, 2-Chlor-4-[[2-(isopropylamino)ethyl]thio]phenyl, 2-Chlor-4-[(2-formamidoethyl)thio]phenyl, 2-Chlor-4-[[2-[(methylsulfonyl)amino]ethyl]thio]-phenyl, 2-Chlor-4-[[2-[(trifluoracetyl)amino]-ethyl]thio]phenyl oder 2-Chlor-4-[[2-[(cyclopropylcarbonyl)amino]ethyl]thio]phenyl steht;
- R₄ für ein Wasserstoffatom, Methyl, Methoxy oder Hydroxy steht;
sowie Additionssalze davon, Hydrate davon oder Solvate davon.

9. Verbindung der Formel (I) nach Anspruch 1, worin:
- R₁ für:
. 4-Isopropylphenyl, 4-*tert*.-Butylphenyl, 4-(Trifluormethyl)phenyl, 4-(Trifluormethoxy)-phenyl, 4-[(Trifluormethyl)thio]phenyl, 2-Fluor-4-(trifluormethyl)phenyl oder 3-Fluor-4-(trifluormethyl)phenyl
steht;
- R² für 4-Bromphenyl, 4-Chlorphenyl, 4-Fluorphenyl, 4-Methoxyphenyl, 4-(Methylthio)phenyl, 4-[[(3,3,3-Trifluorpropyl)sulfonyl]oxy]phenyl, 4-[(Propylsulfonyl)oxy]phenyl oder 4-[[3-[(Methylsulfonyl)amino]propyl]thio]phenyl steht;
- R₃ für 2-Chlorphenyl, 2,4-Dichlorphenyl, 4-Brom-2-chlorphenyl, 2-Chlor-4-methoxyphenyl, 2-Chlor-4-(methylthio)phenyl, 2-Chlor-4-[2-(dimethylamino)ethoxy]phenyl, 2-Chlor-4-[2-(methylthio)ethoxy]phenyl, 2-Chlor-4-[(3-hydroxypropyl)thio]phenyl, 2-Chlor-4-[(2-acetamidoethyl)thio]phenyl, 4-[(2-Aminoethyl)thio]-2-chlorphenyl, 2-Chlor-4-[[2-(dimethylamino)-ethyl]thio]phenyl, 2-Chlor-4-[[2-(diethylamino)ethyl]thio]phenyl, 2-Chlor-4-[[2-(isopropylamino)ethyl]thio]phenyl, 2-Chlor-4-[[2-[(methylsulfonyl)amino]ethyl]thio]phenyl, 2-Chlor-4-[(2-formamidoethyl)thio]phenyl, 2-Chlor-4-[[2-[(trifluoracetyl)amino]ethyl]thio]-phenyl oder 2-Chlor-4-[[2-[(cyclopropylcarbonyl)amino]ethyl]thio]phenyl steht;
- R₄ für ein Wasserstoffatom steht;
sowie Additionssalze davon, Hydrate davon oder Solvate davon.

10. Verbindung der Formel (I) nach Anspruch 1, ausgewählt unter:
- 5-(4-Chlorphenyl)-6-(2,4-dichlorphenyl)-2-(4-isopropylbenzyl)-2,5-dihydro-3H-pyrazolo[4,3-c]-pyridazin-3-on;
- 2-(4-*tert*.-Butylbenzyl)-5-(4-chlorphenyl)-6-(2,4-dichlorphenyl)-2,5-dihydro-3H-pyrazolo[4,3-c]pyridazin-3-on;
- 5-(4-Chlorphenyl)-6-(2,4-dichlorphenyl)-2-[4-(trifluormethyl)benzyl]-2,5-dihydro-3*H*-pyrazolo-[4,3-c]pyridazin-3-on;
- 5-(4-Chlorphenyl)-6-(2,4-dichlorphenyl)-2-[2-fluor-4-(trifluormethyl)benzyl]-2,5-dihydro-3*H-*pyrazolo[4,3-c]pyridazin-3-on;
- 5-(4-Chlorphenyl)-6-(2,4-dichlorphenyl)-2-[4-[(trifluormethyl)thio]benzyl]-2,5-dihydro-3*H-*pyrazolo[4,3-c]pyridazin-3-on;
- 5-(4-Bromphenyl)-6-(2,4-dichlorphenyl)-2-(4-isopropylbenzyl)-2,5-dihydro-3*H*-pyrazolo[4,3-c]-pyridazin-3-on;
- 5-(4-Bromphenyl)-2-(4-butylbenzyl)-6-(2,4-dichlorphenyl)-2,5-dihydro-3*H*-pyrazolo[4,3-c]-pyridazin-3-on;
- 5-(4-Bromphenyl)-6-(2,4-dichlorphenyl)-2-[3-fluor-4-(trifluormethyl)benzyl]-2,5-dihydro-3*H-*pyrazolo[4,3-c]pyridazin-3-on;
- 5-(4-Bromphenyl)-6-(2,4-dichlorphenyl)-2-[4-(trifluormethoxy)benzyl]-2,5-dihydro-3*H*-pyrazolo-[4,3-c]pyridazin-3-on;
- 5-(4-Bromphenyl)-6-(2,4-dichlorphenyl)-2-[4-[(trifluormethyl)thio]benzyl]-2,5-dihydro-3*H-*pyrazolo[4,3-c]pyridazin-3-on;
- 6-(2,4-dichlorphenyl)-5-[4-(methylthio)phenyl]-2-[4-(Trifluormethyl)benzyl]-2,5-dihydro-3*H-*pyrazolo[4,3-c]pyridazin-3-on;
- 6-(2,4-Dichlorphenyl)-2-[2-fluor-4-(trifluormethyl)benzyl]-5-[4-(methylthio)phenyl]-2,5-dihydro-3*H*-pyrazolo[4,3-c]pyridazin-3-on;
- 6-(2,4-Dichlorphenyl)-5-[4-(methylthio)phenyl]-2-[4-(trifluormethoxy)benzyl]-2,5-dihydro-3*H-*pyrazolo[4,3-c]pyridazin-3-on;
- 6-(2-Chlorphenyl)-5-(4-chlorphenyl)-2-[4-[(trifluormethyl)thio]benzyl]-2,5-dihydro-3*H*-pyrazolo-[4,3-c]pyridazin-3-on;
- 6-(2,4-Dichlorphenyl)-5-(4-methoxyphenyl)-2-[4-[(trifluormethoxy)benzyl]-2,5-dihydro-3*H*-pyrazolo-[4,3-c]pyridazin-3-on;
- 6-(2,4-Dichlorphenyl)-5-(4-methoxyphenyl)-2-[4-[(trifluormethyl)thio]benzyl]-2,5-dihydro-3*H-*pyrazolo[4,3-c]pyridazin-3-on;
- 6-(2-Chlorphenyl)-5-(4-fluorphenyl)-2-[4-[(trifluormethyl)thio]benzyl]-2,5-dihydro-3*H-*pyrazolo[4,3-c]pyridazin-3-on;
- 4-[6-(2,4-Dichlorphenyl)-3-oxo-2-[4-[(trifluormethyl)thio]benzyl]-2,3-dihydro-5*H*-pyrazolo[4,3-c]pyridazin-5-yl]phenyl-3,3,3-trifluorpropan-1-sulfonat;
- 4-[6-(2,4-Dichlorphenyl)-3-oxo-2-[4-[(trifluormethyl)thio]benzyl]-2,3-dihydro-5*H*-pyrazolo[4,3-c]pyridazin-5-yl]phenylpropan-1-sulfonat;
- 6-(4-Brom-2-chlorphenyl)-5-[4-(methylthio)phenyl]-2-[4-(trifluormethyl)benzyl]-2,5-dihydro-3*H*-pyrazolo[4,3-*c*]pyridazin-3-on;
- 6-{2-Chlor-4-[(3-hydroxypropyl)thio]phenyl}-5-[4-(methylthio)phenyl]-2-[4-(trifluormethyl)benzyl]-2,5-dihydro-3*H*-pyrazolo[4,3-*c*]pyridazin-3-on;
- 6-(4-Brom-2-chlorphenyl)-5-[4-(methylthio)phenyl]-2-{4-[(trifluormethyl)thio]benzyl}-2,5-dihydro-3*H*-pyrazolo[4,3-*c*]pyridazin-3-on;
- *N*-(2-{[3-Chlor-4-(5-[4-(methylthio)phenyl]-3-oxo-2-{4-[(trifluormethyl)thio]benzyl}-3,5-dihydro-2*H*-pyrazolo[4,3-*c*]pyridazin-6-yl)phenyl]-thio}ethyl)acetamid;
- 6-{2-Chlor-4-[(3-hydroxypropyl)thio]phenyl}-5-[4-(methylthio)phenyl]-2-{4-[(trifluormethyl)thio]benzyl}-2,5-dihydro-3*H*-pyrazolo-[4,3-*c*]pyridazin-3-on;
- 6-(2-Chlor-4-methoxyphenyl)-5-[4-(methylthio)phenyl]-2-[4-(trifluormethyl)benzyl]-2,5-dihydro-3*H*-pyrazolo[4,3-*c*]pyridazin-3-on;
- 6-(2-Chlor-4-methoxyphenyl)-5-[4-(methylthio)phenyl]-2-[4-(trifluormethoxy)benzyl]-2,5-dihydro-3*H*-pyrazolo[4,3-*c*]pyridazin-3-on;
- 6-(2-Chlor-4-methoxyphenyl)-5-[4-(methylthio)phenyl]-2-{4-[(trifluormethyl)thio]-benzyl}-2,5-dihydro-3*H*-pyrazolo[4,3-*c*]pyridazin-3-on;
- 6-{2-Chlor-4-[2-(dimethylamino)ethoxy]phenyl}-5-[4-(methylthio)phenyl]-2-[4-(trifluormethyl)benzyl]-2,5-dihydro-3H-pyrazolo-[4,3-*c*]pyridazin-3-on;
- 6-{2-Chlor-4-[2-(methylthio)ethoxy]phenyl}-5-[4-(methylthio)phenyl]-2-[4-(trifluormethyl)benzyl]-2,5-dihydro-3*H*-pyrazolo[4,3-*c*]pyridazin-3-on;
- 6-[2-Chlor-4-(methylthio)phenyl]-5-[4-(methylthio)phenyl]-2-[4-(trifluormethyl)benzyl]-2,5-dihydro-3*H*-pyrazolo[4,3-*c*]pyridazin-3-on;
- 6-[2-Chlor-4-(methylthio)phenyl]-5-[4-(methylthio)phenyl]-2-{4-[(trifluormethyl)thio]-benzyl}-2,5-dihydro-3*H*-pyrazolo[4,3-*c*]pyridazin-3-on;
- *N*-[3-({4-[6-(2,4-Dichlorphenyl)-3-oxo-2-{4-[(trifluormethyl)thio]benzyl}-2,3-dihydro-5*H-*pyrazolo[4,3-*c*]pyridazin-5-yl]phenyl}thio)propyl]methansulfonamid;
- *N*-{3-[(4-{6-(2,4-Dichlorphenyl)-3-oxo-2-[4-(trifluormethyl)benzyl]-2,3-dihydro-5*H-*pyrazolo[4,3-*c*]pyridazin-5-yl}phenyl)thio]propyl}methansulfonamid;
- 6-{4-[(2-Aminoethyl)thio]-2-chlorphenyl}-5-[4-(methylthio)phenyl]-2-[4-(trifluormethyl)benzyl]-2,5-dihydro-3*H*-pyrazolo[4,3-*c*]pyridazin-3-on;
- 6-(2-Chlor-4-{[2-(dimethylamino)-ethyl]thio}phenyl)-5-[4-(methylthio)phenyl]-2-[4-(trifluormethyl)benzyl]-2,5-dihydro-3*H*-pyrazolo-[4,3-*c*]pyridazin-3-on;
- 6-(2-Chlor-4-{[2-(diethylamino)ethyl]-thio}phenyl-5-[4-(methylthio)phenyl]-2-[4-(trifluormethyl)benzyl]-2,5-dihydro-3H-pyrazolo-[4,3-*c*]pyridazin-3-on;
- 6-(2-Chlor-4-{[2-(isopropylamino)ethyl]-thio}phenyl)-5-[4-(methylthio)phenyl]-2-[4-(trifluormethyl)benzyl]-2,5-dihydro-3H-pyrazolo-[4,3-*c*]pyridazin-3-on;
- *N*-{2-[(3-Chlor-4-{5-[4-(methylthio)phenyl]-3-oxo-2-[4-(trifluormethyl)benzyl]-3,5-dihydro-2*H-*pyrazolo[4,3-*c*]pyridazin-6-yl}phenyl)thio]-ethyl}methansulfonamid;
- *N*-{2-[(3-Chlor-4-{5-[4-(methylthio)phenyl]-3-oxo-2-[4-(trifluormethyl)benzyl]-3,5-dihydro-2*H-*pyrazolo[4,3-*c*]pyridazin-6-yl}phenyl)thio]-ethyl}formamid;
- *N*-{2-[(3-Chlor-4-{5-[4-(methylthio)phenyl]-3-oxo-2-[4-(trifluormethyl)benzyl]-3,5-dihydro-2*H-*pyrazolo[4,3-*c*]pyridazin-6-yl}phenyl)thio]ethyl}-2,2,2-trifluoracetamid;
- *N*-{2-[(3-Chlor-4-{5-[4-(methylthio)phenyl]-3-oxo-2-[4-(trifluormethyl)benzyl]-3,5-dihydro-2*H-*pyrazolo[4,3-*c*]pyridazin-6-yl}phenyl)-thio]ethyl}cyclopropancarboxamid;
sowie Additionssalze davon, Hydrate davon oder Solvate davon.

11. Verfahren zur Herstellung von Verbindungen der Formel (I) nach Anspruch 1, **dadurch gekennzeichnet, daß** man:
eine Verbindung der Formel: 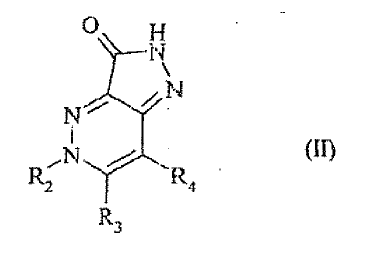 worin R₂, R₃ und R₄ die für eine Verbindung der Formel (I) in Anspruch 1 angegebene Bedeutung besitzen, in Gegenwart einer Base mit einer Verbindung der Formel:
Y-CH₂-R₁ (III)
worin R₁ die für eine Verbindung der Formel (I) in Anspruch 1 angegebene Bedeutung besitzt und Y für eine Abgangsgruppe steht, die unter einem Halogenatom oder einer aktivierten Hydroxylgruppe, die unter einer Methansulfonat-, Benzolsulfonylat-, p-Toluolsulfonat- oder Triflatgruppe ausgewählt ist, ausgewählt ist, umsetzt.

12. Verbindungen der Formel: 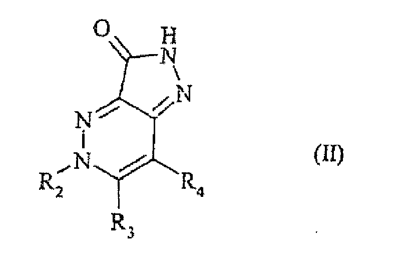 worin:
- R₂ für Phenyl, das gegebenenfalls ein- oder mehrfach durch ein Halogenatom, Hydroxy, eine Alk-Gruppe, eine OAlk-Gruppe, eine S(O)ₙAlk-Gruppe oder eine OS(O)ₙAlk-Gruppe substituiert ist, steht;
- R₃ für Phenyl, das gegebenenfalls ein- oder mehrfach durch ein Halogenatom, Hydroxy, eine Alk-Gruppe, eine OAlk-Gruppe, eine S(O)ₙAlk-Gruppe oder eine OS(O)ₙAlk-Gruppe substituiert ist, steht;
- R₄ für ein Wasserstoffatom, (C₁-C₄)-Alkyl, (C₁-C₄)-Alkoxy oder Hydroxy steht;
- n für 0, 1 oder 2 steht;
- Alk für (C₁-C₄)-Alkyl, das gegebenenfalls ein- oder mehrfach durch ein Fluoratom substituiert ist, steht.

13. Arzneimittel, **dadurch gekennzeichnet, daß** es eine Verbindung der Formel (I) nach einem der Ansprüche 1 bis 10 oder ein pharmazeutisch unbedenkliches Additionssalz, ein Hydrat oder ein Solvat der Verbindung der Formel (I) enthält.

14. Pharmazeutische Zusammensetzung, **dadurch gekennzeichnet, daß** sie eine Verbindung der Formel (I) nach einem der Ansprüche 1 bis 10 oder ein pharmazeutisch unbedenkliches Additionssalz, ein Hydrat oder ein Solvat dieser Verbindung sowie mindestens einen pharmazeutisch unbedenklichen Hilfsstoff enthält.

15. Verwendung einer Verbindung der Formel (I) gemäß einem der Ansprüche 1 bis 10 zur Herstellung eines Arzneimittels zur Behandlung oder Prävention von Erkrankungen, an denen die CB₁-Rezeptoren beteiligt sind.

16. Verwendung nach Anspruch 15, **dadurch gekennzeichnet, daß** es sich bei den Erkrankungen um psychiatrische Störungen, Substanzabhängigkeit und -entwöhnung, kognitive Störungen, Aufmerksamkeits- und Wachsamkeitsstörungen und akute und chronische neurodegenerative Erkrankungen handelt.

17. Verbindung nach Anspruch 15, **dadurch gekennzeichnet, daß** es sich bei den Erkrankungen um Stoffwechselstörungen, Appetenzstörungen, Appetitstörungen, Obesitas, Typ-II-Diabetes, metabolisches Syndrom und Dyslipidämie handelt.

18. Verwendung nach Anspruch 15, **dadurch gekennzeichnet, daß** es sich bei den Erkrankungen um Schmerzen, neuropathische Schmerzen und durch Antikrebsbehandlung induzierte Schmerzen handelt.

19. Verwendung nach Anspruch 15, **dadurch gekennzeichnet, daß** es sich bei den Erkrankungen um Magen-Darm-Störungen, Erbrechen, Durchfallstörungen, Geschwüre und Lebererkrankungen handelt.

20. Verwendung nach Anspruch 15, **dadurch gekennzeichnet, daß** es sich bei den Erkrankungen um Erkrankungen des Immunsystems, rheumatoide Arthritis, Demyelinisierung, multiple Sklerose und entzündliche Erkrankungen handelt.

21. Verwendung nach Anspruch 15, **dadurch gekennzeichnet, daß** es sich bei den Erkrankungen um Alzheimer-Krankheit, Parkinson-Krankheit, Schizophrenie, kognitive Störungen, Diabetes, Obesitas, metabolisches Syndrom und Tabakentwöhnung handelt.
